(19) **Europäisches Patentamt / European Patent Office / Office européen des brevets**

(11) **EP 4 058 037 B1**

(12) **EUROPEAN PATENT SPECIFICATION**

(45) Date of publication and mention of the grant of the patent:
**28.01.2026  Bulletin 2026/05**

(21) Application number: **20886462.9**

(22) Date of filing: **16.11.2020**

(51) International Patent Classification (IPC):
**A61K 35/50** (2015.01)  **A61K 35/33** (2015.01)
**A61K 35/36** (2015.01)  **A61P 9/10** (2006.01)

(52) Cooperative Patent Classification (CPC):
(C-Sets available)
**A61K 38/2026; A61K 31/155; A61K 31/4045;
A61K 31/65; A61K 35/28; A61K 35/33;
A61K 38/13; A61K 38/1808; A61K 38/1825;
A61K 38/1841; A61K 38/1866; A61K 38/193;
A61K 38/2066; A61K 38/2086; A61K 45/06;**
(Cont.)

(86) International application number:
**PCT/US2020/060724**

(87) International publication number:
**WO 2021/097423 (20.05.2021 Gazette 2021/20)**

(54) **FIBROBLAST-BASED THERAPY FOR TREATMENT AND PREVENTION OF STROKE**

FIBROBLASTENBASIERTE THERAPIE ZUR BEHANDLUNG UND VORBEUGUNG VON SCHLAGANFALL

THÉRAPIE À BASE DE FIBROBLASTES POUR LE TRAITEMENT ET LA PRÉVENTION D'UN ACCIDENT VASCULAIRE CÉRÉBRAL

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **17.11.2019  US 201962936548 P
02.07.2020  US 202063047813 P**

(43) Date of publication of application:
**21.09.2022  Bulletin 2022/38**

(73) Proprietor: **SpinalCyte LLC
Houston, TX 77289 (US)**

(72) Inventors:
• **O'HEERON, Pete
Houston, Texas 77059 (US)**
• **ICHIM, Thomas
Houston, Texas 77058 (US)**

(74) Representative: **J A Kemp LLP
80 Turnmill Street
London EC1M 5QU (GB)**

(56) References cited:
**WO-A1-2020/093051      WO-A2-2007/035843**

• **TASSEW NARDOS G. ET AL: "Exosomes Mediate Mobilization of Autocrine Wnt10b to Promote Axonal Regeneration in the Injured CNS", CELL REPORTS, vol. 20, no. 1, 1 July 2017 (2017-07-01), US, pages 99 - 111, XP093084807, ISSN: 2211-1247, DOI: 10.1016/ j.celrep.2017.06.009**
• **ARBELÁEZ-QUINTERO ISAAC ET AL: "To Use or Not to Use Metformin in Cerebral Ischemia: A Review of the Application of Metformin in Stroke Rodents", STROKE RESEARCH AND TREATMENT, vol. 2017, 28 May 2017 (2017-05-28), pages 1 - 13, XP093084951, ISSN: 2090-8105, Retrieved from the Internet <URL:https://www.ncbi.nlm.nih.gov/pmc/ articles/PMC5467394/pdf/SRT2017-9756429.pdf> DOI: 10.1155/2017/9756429**

**(Cont. next page)**

- **SHENG ZHAOFU ET AL: "Efficacy of Minocycline in Acute Ischemic Stroke: A Systematic Review and Meta-Analysis of Rodent and Clinical Studies", FRONTIERS IN NEUROLOGY, vol. 9, 20 December 2018 (2018-12-20), XP093084955, DOI: 10.3389/fneur.2018.01103**
- **CHOU ET AL.: "Intracerebral transplantation of erythropoietin-producing fibroblasts facilitates neurogenesis and functional recovery in an ischemic stroke model", BRAIN AND BEHAVIOR, vol. 9, no. 5, May 2019 (2019-05-01), pages 1 - 11, XP055705295**
- **CHEN ET AL.: "Brain-Derived Neurotrophic Factor-Transfected and Nontransfected 3T3 Fibroblasts Enhance Migratory Neuroblasts and Functional Restoration in Mice With Intracerebral Hemorrhage", JOURNAL OF NEUROPATHOLOGY AND EXPERIMENTAL NEUROLOGY, vol. 71, no. 12, December 2012 (2012-12-01), pages 1123 - 1136, XP055823408**

(52) Cooperative Patent Classification (CPC): (Cont.)
**A61P 9/10**

C-Sets
**A61K 31/155, A61K 2300/00;
A61K 31/4045, A61K 2300/00;
A61K 31/65, A61K 2300/00;
A61K 35/28, A61K 2300/00;
A61K 35/33, A61K 2300/00;
A61K 38/13, A61K 2300/00;
A61K 38/1808, A61K 2300/00;
A61K 38/1825, A61K 2300/00;
A61K 38/1841, A61K 2300/00;
A61K 38/1866, A61K 2300/00;
A61K 38/193, A61K 2300/00;
A61K 38/2026, A61K 2300/00;
A61K 38/2086, A61K 2300/00**

## Description

TECHNICAL FIELD

Described herein are exosomes derived from fibroblasts for use in a method of treating or preventing a stroke in a subject, the method comprising providing to the subject an effective amount of said exosome derived from fibroblasts.

BACKGROUND

Stroke is the third leading cause of death and disability in adults in the United States. Thrombolytic therapy only benefits about 2% of ischemic stroke patients. The dismal record of neurorestorative regimens for stroke both in the laboratory and the clinic solicits an urgent need to develop novel therapies. Because the secondary cellular death that ensues after the initial stroke episode occurs over an extended time, treatment strategies directed at rescuing these ischemic neurons have the potential to retard the disease progression and even afford restoration of function. The recognition of this delay in secondary stroke-induced pathophysiologic alterations has prompted investigations on neurorestorative treatments, including cell therapy, to salvage the ischemic penumbra and promote functional recovery from stroke. Cell therapy thus offers a new avenue for the treatment and management of stroke.

Intracerebral hemorrhage (ICH) is a devastating kind of stroke with high mortality and morbidity rate, effects 40 000 people in the United States annually[1]. Despite of significant progress in diagnostics of this disease and on-going research on new therapy strategies there is no cure significantly decreasing mortality and improving life quality of survivals. Only fifty percent of patients will survive ICH, and these individuals will be afflicted with significant brain atrophy and lifelong neurological deficits[2].

Primary bleeding initiates several pathophysiological pathways leading to the secondary brain injury. This pathways include activation of microglia[3], production of pro-inflammatory cytokines[4] and reactive oxygen species[5] and infiltration by systemic immunocells [6]. Inflammation and ROS production are known events leading to disruption of blood-brain barrier, development of brain edema, the most live-threatened event after ICH. Long-term effect of factors mentioned above is a brain atrophy and long-lasting neurological deficit, events well investigated in the model of experimental ICH in our laboratory[7] and by others[8].

Recent publications demonstrated that the brain after ICH is capable to self-repair[9]. It was also demonstrated that some endogenous and exogenous factors can stimulate post-injury neurogenesis and angiogenesis and promote brain recovery after ICH resulting in significant improvement of neurological outcome [10, 11].

The surgical treatment of spontaneous intracerebral haemorrhage (SIH) is still a matter of controversy, although most Neurosurgeons agree that surgery is indicated in selected cases. The introduction of computer tomography (CT) permits a more accurate determination of the localization, size and expansion of an intracerebral haemorrhage. One study aimed to evaluate the results of surgical and conservative therapy in selected cases. The decision for surgical treatment was made on the basis of the patient's conditions and the findings in the CT scan. Thirty-nine patients with mainly medium-sized haemorrhages underwent surgery and 35 were conservatively treated. The mortality after three months was 5/39 (13%) in the surgical and 7/35 (20%) in the conservative group. The volume of haemorrhage was significantly larger in the patients who died and 9/10 patients with a haematoma volume above 80 mL died. Five of these 10 were operated and the other 5 not and surgery seemed to be of little benefit to this group. Dilatation of the contralateral ventricle is another indicator of a bad prognosis. Long-term follow-up investigation was carried out 4-38 months after the initial treatment. Total mortality was 19 out of 68 patients that could be reached for late follow-up. Eleven patients (29%) were fully recovered and 16 had minor neurological deficits. There was no difference in late results between the surgical and the conservative groups, but the patients in the surgical group were generally in a worse condition and had larger haemorrhages that the others. The fact that the total mortality in this material is lower than in other conservatively treated series favours surgery in selected cases of SIH. The use of CT gives valuable information as to the prognosis and especially the volume of haemorrhage seems to be a good prognostic factor [12].

Spontaneous intracerebral hematomas acutely increase intracranial volume and reduce intracranial compliance, thus potentially increasing intracranial pressure and reducing cerebral perfusion. Therefore, any physiologic perturbation that could increase cerebral blood volume (hypercarbia, hypoxia, vasodilation) or reduce cerebral perfusion (hypotension) should be avoided. Acute hypertension may be a response to increased intracranial pressure and should be treated conservatively. Treatment of increased intracranial pressure is accomplished initially with hyperventilation, but this approach should be replaced rapidly with cerebrospinal fluid drainage alone or in combination with osmotic therapy employing diuretics, osmotic agents, or hypertonic saline to produce a state of hyperosmolality and euvolemia. Although the role of surgery remains controversial, it may be helpful in selected patients. Careful attention to pulmonary and metabolic status and to the consequences of therapy is required. Outcome is primarily determined by hemorrhage size, location, and initial level of consciousness. Stereotaxic aspiration of hematomas is a promising new therapy [13].

New discoveries suggested that regenerative cells based therapy may be a promising route in promoting endogenous process leading to brain repair after ICH [14] and in producing and replacing of new neurons as well. Several publications demonstrated that cell transplantation leads to significant decrease in inflammatory reactions and ameliorates production of reactive oxygen species (ROS), thereby supports ICH-suppressed endogenous neurogenesis. Additionally, cell therapy can directly increase endogenous neurogenesis[15].

Tassew et al (Cell Reports, vol. 20, no. 1, 1 July 2017, pages 99-111) relates to exosomes mediating mobilization of autocrine Wnt10b to promote axonal regeneration in the injured CNS. Arbeláez-Quintero et al (Stroke Research and Treatment, vol. 2017, 28 May 2017, pages 1-13) relates to the use of metformin in cerebral ischemia and a review of the application of metformin in stroke rodents. Sheng et al (Frontiers in Neurology, vol. 9, 20 December 2018) relates to the efficacy of minocycline in acute ischemic stroke and a systematic review and meta-analysis of rodent and clinical studies. WO 2020/093051 A1 relates to treatment of cerebral hypoxia including stroke, chronic traumatic encephalopathy, and traumatic brain injury. Chou et al (Brain and Behavior, vol. 9, no. 5, pages 1-11) relates to intracerebral transplantation of erythropoietin-producing fibroblasts facilitates neurogenesis and functional recovery in an ischemic stroke model. Chen et al (Journal of Neuropathology and Experimental Neurology, vol. 71, no. 12, pages 1123-1136) relates to brain-derived neurotrophic factor-transfected and nontransfected 3T3 fibroblasts enhancing migratory neuroblasts and functional restoration in mice with intracerebral hemorrhage.

The present disclosure satisfies a long felt need in the art for treatment and prevention of stroke, including ischemic stroke and hemorrhagic stroke.

SUMMARY OF THE INVENTION

The invention provides exosomes derived from fibroblasts for use in a method of treating or preventing a stroke in a subject, the method comprising providing to the subject an effective amount of said exosome derived from fibroblasts.

The invention is defined in the independent claim. Further aspects and preferred embodiments are defined in the dependent claims. Any aspects, embodiments and examples of the present disclosure which do not fall under the scope of the appended claims do not form part of the invention and are merely provided for illustrative purposes.

References to the methods of treatment by therapy or surgery or *in vivo* diagnosis methods in the description are to be interpreted as references to compounds, pharmaceutical compositions and medicaments of the present invention for use in those methods.

SUMMARY OF THE TECHNICAL TEACHINGS

Aspects of the disclosure are based, at least in part, on the discovery that fibroblasts of various tissues, either alone and/or after culture with dedifferentiation or cellular activating factors, can support viability of neurons and other parenchymal cells in the brain subsequent to a stroke. Also disclosed is the discovery that fibroblasts, alone or under induced conditions, are capable of secreting multiple factors beneficial to neighboring and/or distal cells that stimulate regeneration, immune modulation, and prevention of neuronal cell death subsequent to a stroke. As disclosed herein, fibroblasts, when introduced to a subject, activate endogenous cells to proliferate and differentiate. In addition, fibroblasts, when introduced to a site at or near a brain injury or spinal cord injury, are capable of producing and secreting an array of factors including exosomes, microvesicles, trophic factors, cytokines, and growth factors. These factors may activate cells including, but not limited to, endogenous stem cells and/or ependymal cells to proliferate and differentiate into parenchymal cells such as neurons. Thus, in some aspects, disclosed herein are methods for promoting repair and plasticity of a central nervous system (CNS) tissue using fibroblasts and/or factors (e.g., exosomes) derived therefrom.

Disclosed herein, in some aspects, are methods of treating or preventing a stroke in a subject comprising providing to the subject an effective amount of fibroblasts or exosomes derived from fibroblasts. In some aspects, the method comprises providing the subject with an effective amount of fibroblasts. In some aspects, the stroke is an ischemic stroke. In some aspects, the stroke is a hemorrhagic stroke. In some aspects, the fibroblasts are plastic-adherent fibroblasts. In some aspects, the fibroblasts are in a proliferative state. In some aspects, the fibroblasts are allogenic fibroblasts. In some aspects, the fibroblasts are xenogenic fibroblasts. In some aspects, the fibroblasts are autologous fibroblasts. In some aspects, the fibroblasts are fibroblasts isolated from placenta, cord blood, peripheral blood, omentum, hair follicle, skin, bone marrow, adipose tissue, or Wharton's Jelly. In some aspects, the fibroblasts induce neuroregeneration in the subject.

In some aspects, the fibroblasts induce immune modulation in the subject. In some aspects, the immune modulation comprises enhancing production of a cytokine associated with neuroprotection in the subject. In some aspects, the cytokine is interleukin-10. In some aspects, the cytokine is interleukin-4. In some aspects, the cytokine is interleukin-13. In some aspects, the cytokine is interleukin-35. In some aspects, the cytokine is TGF-$\beta$.

In some aspects, the fibroblasts are provided to the subject prior to the onset of the stroke, thereby preventing the stroke. In some aspects, the fibroblasts are provided to the subject subsequent to the onset of the stroke, thereby treating the stroke. In some aspects, the fibroblasts reduce production of IL-17 from microglial cells in the subject. In some aspects, the fibroblasts reduce production of TNF-$\alpha$ from microglial cells in the subject. In some aspects, the fibroblasts reduce neuroinflammation in the subject. In some aspects, the neuroinflammation is reduced in an ischemic penumbra of the stroke.

In some aspects, the method further comprises providing a TNF-α inhibitor to the subject. In some aspects, the TNF-α inhibitor is melatonin. In some aspects, the TNF-α inhibitor is cycloheximide, auranofin, sodium aurothiomalate, Leukotriene B4, interleukin-4, interleukin-13, polymyxin B, bile acids, interleukin-6, lactulose, oxpentifylline, mometasone, glucocorticoids, colchicine, chloroquine, FK-506, berberine, resveratrol, pterostilbene, vitamin A, vitamin C, cyclosporine, phosphodiesterase inhibitors such as vinpocetine, milrinone, CI-930, rolipram, nitroquazone, zaprinast, synthetic lipid A, amrinone, N-acetylcysteine, dithiocarbamates and metal chelators, exosurf synthetic surfactant, dehydroepiandrosterone, delta-tetrahydrocannabinol, phosphatidylserine, TCV-309, a PAF antagonist, thalidomide, a cytochrome p450 inhibitor, cytochalasin D, ketamine, TGF-beta, interleukin-10, pentoxifylline, BRL 61,063, a calcium antagonist, curcumin, kappa-selective opioid agoinst U50,488H (trans-3,4-dichloro-N-methyl-N-[7-(1-pyrrolidinyl)cyclohexyl]benzene-acetamide methanesulfonate), alendronate, tetrandrine, sulfasalazine, epinephrine, BMS-182123, adenosine, E3330, nicotine, IVIG, cardiotrophin-1, KB-R7785, CGRP, ligustrazine, dexanabinol, iloprost, activated protein C, a growth hormone, spermine, FR-167653, gm-6001, estradiol, aspirin, or amiodarone.

In some aspects, the method further comprises providing to the subject an agent capable of inhibiting responsiveness to TNF-α. In some aspects, the agent capable of inhibiting responsiveness to TNF-α is ibuprofen, indomethacin, Nedocromil sodium, cromolyn (sodium cromoglycate), a spleen derived factor, pentoxifylline, NG-methyl-L-arginine, dexamethasone, chlorpromazine, activated alpha 2 macroglobulin, serum amyloid A protein, a neutrophil derived proteolytic enzyme, phentolamine, propranolol, a leukotriene inhibitor, nordihydroguaiaretic acid, genistein, butylated hydroxyanisole, CNI-1493, quercetin, gabexate mesylate, SM-12502, monoclonal nonspecific suppressor factor (MNSF), pyrrolidine dithiocarbamate (PDTC), or aprotinin.

In some aspects, the fibroblasts stimulate proliferation of neural progenitor cells in the subject. In some aspects, the neural progenitor cells are in the dentate gyrus of the subject. In some aspects, the neural progenitor cells are in the subventricular zone of the subject.

In some aspects, the method further comprises comprising providing an anti-apoptotic agent to the subject. In some aspects, the anti-apoptotic agent is a caspase inhibitor. In some aspects, the caspase inhibitor is a caspase-3 inhibitor or a caspase-9 inhibitor. In some aspects, the anti-apoptotic agent is EGF, FGF, carbon monoxide, FEDVI peptide, or TGF-β.

In some aspects, the method further comprises providing an additional agent to the subject, wherein the additional agent is n-acetylcysteine, ascorbic acid, alpha lipoic acid, human chorionic gonadotropin, VEGF, TNF-α, retinoic acid, alpha tocopherol, interleukin-3, G-CSF, GM-CSF, leukemia inhibitory factor, placental growth factor, angiopoietin, hydrogenated water, or NGF. In some aspects, the method further comprises providing endothelial progenitor cells to the subject. In some aspects, the endothelial progenitor cells are derived from the subject. In some aspects, the endothelial progenitor cells are allogenic. In some aspects, the endothelial progenitor cells are derived from peripheral blood, mobilized peripheral blood, bone marrow, adipose derived stromal vascular fraction, cord blood, Wharton's jelly, or placenta.

In some aspects, the method further comprises mobilizing endothelial progenitor cells in the subject. In some aspects, mobilizing the endothelial progenitor cells comprises administration of G-CSF to the subject. In some aspects, mobilizing the endothelial progenitor cells comprises administration of GM-CSF to the subject. In some aspects, mobilizing the endothelial progenitor cells comprises administration of IL-3 to the subject. In some aspects, mobilizing the endothelial progenitor cells comprises administration of TPO to the subject. In some aspects, mobilizing the endothelial progenitor cells comprises administration of FLT3 ligand (FL) to the subject. In some aspects, mobilizing the endothelial progenitor cells comprises administration of G-CSF to the subject.

In some aspects, the method further comprises providing a regenerative cell to the subject. In some aspects, the regenerative cell is a stem cell. In some aspects, the stem cell is a hematopoietic stem cell. In some aspects, the hematopoietic stem cell expresses CD34, CD133, or c-kit. In some aspects, the hematopoietic stem cell does not express one or more of CD38 and thrombopoietin receptor. In some aspects, the hematopoietic stem cell is an autologous hematopoietic stem cell. In some aspects, the hematopoietic stem cell is an allogenic hematopoietic stem cell. In some aspects, the hematopoietic stem cell is a xenogenic hematopoietic stem cell. In some aspects, the hematopoietic stem cell is derived from adipose, bone marrow, peripheral blood, mobilized peripheral blood, or cord blood. In some aspects, the method further comprises comprising providing to the subject a mesenchymal stem cell or populations thereof. In some aspects, the mesenchymal stem cell expresses CD90, CD105, or CD73. In some aspects, the mesenchymal stem cell does not express one or more of HLA, CD34, or CD14. In some aspects, the mesenchymal stem cell is plastic adherent. In some aspects, the mesenchymal stem cell is allogenic to the subject. In some aspects, the mesenchymal stem cell is autologous to the subject. In some aspects, the mesenchymal stem cell is derived from adipose, bone marrow, peripheral blood, mobilized peripheral blood, menstrual blood, fallopian tube, or cord blood.

In some aspects, the method further comprises providing to the subject an effective amount of exosomes derived from one or more stem cells, wherein the one or more stem cells comprise hematopoietic stem cells, mesenchymal stem cells, or a combination thereof. In some aspects, the exosomes are derived from the one or more stem cells via ultracentrifugation. In some aspects, the exosomes are derived from the one or more stem cells via chromatography. In some aspects, the exosomes are derived from the one or more stem cells via affinity purification. In some aspects, an outer surface of the exosomes comprises phosphatidylserine, CD9, CD19, or a tetraspanin protein. In some aspects, the method further comprises stimulating the one or more stem cells to secrete the exosomes. In some aspects, the stimulating comprises culturing the one or more stem cells in hypoxic conditions. In some aspects, the hypoxic conditions comprise between 0.01% and 10% oxygen. In some aspects, the hypoxic conditions comprise 3% oxygen. In some aspects, the one or more stem cells are cultured in the hypoxic conditions for less than 14 days. In some aspects, the one or more stem cells are cultured in the hypoxic conditions for about 4 days.

In some aspects, the method further comprises culturing the fibroblasts with metformin prior to providing the fibroblasts to the subject. In some aspects, the fibroblasts stimulate production of an angiogenic cytokine in the subject. In some aspects, the angiogenic cytokine is VEGF, FGF-1, FGF-2, or IGF-1. In some aspects, the fibroblasts stimulate production of a neurogenic cytokine in the subject. In some aspects, the neurogenic cytokine is BDNF, NGF, or CNTF.

In some aspects, the method further comprises transfecting the fibroblasts with one or more angiogenic genes prior to providing the fibroblasts to the subject. In some aspects, the one or more angiogenic genes comprise activin A, adrenomedullin, aFGF, ALK1, ALK5, ANF, angiogenin, angiopoietin-1, angiopoietin-2, angiopoietin-3, angiopoietin-4, bFGF, B61, bFGF inducing activity, cadherins, CAM-RF, cGMP analogs, ChDI, CLAF, claudins, collagen, connexins, Cox-2, ECDGF (endothelial cell-derived growth factor), ECG, ECI, EDM, EGF, EMAP, endoglin, endothelins, endostatin, endothelial cell growth inhibitor, endothelial cell-viability maintaining factor, endothelial differentiation shpingolipid G-protein coupled receptor-1 (EDG1), ephrins, Epo, HGF, TGF-beta, PD-ECGF, PDGF, IGF, IL8, growth hormone, fibrin fragment E, FGF-5, fibronectin, fibronectin receptor, Factor X, HB-EGF, HBNF, HGF, HUAF, heart derived inhibitor of vascular cell proliferation, IL1, IGF-2 IFN-gamma, $\alpha 1\beta 1$ integrin, $\alpha 2\beta 1$ integrin, K-FGF, LIF, leiomyoma-derived growth factor, MCP-1, macrophage-derived growth factor, monocyte-derived growth factor, MD-ECI, MECIF, MMP2, MMP3, MMP9, urokiase plasminogen activator, neuropilin, neurothelin, nitric oxide donors, nitric oxide synthases (NOSs), notch, occludins, zona occludins, oncostatin M, PDGF, PDGF-B, PDGF receptors, PDGFR-$\beta$, PD-ECGF, PAI-2, PD-ECGF, PF4, P1GF, PKR1, PKR2, PPAR-gamma, PPAR-gamma ligands, phosphodiesterase, prolactin, prostacyclin, protein S, smooth muscle cell-derived growth factor, smooth muscle cell-derived migration factor, sphingosine-1-phosphate-1 (SIP1), Syk, SLP76, tachykinins, TGF-beta, Tie 1, Tie2, TGF-$\beta$, TGF-$\beta$ receptors, TIMPs, TNF-$\alpha$, transferrin, thrombospondin, urokinase, VEGF-A, VEGF-B, VEGF-C, VEGF-D, VEGF-E, VEGF, VEGF(164), VEGI, EG-VEGF, or a combination thereof.

In some aspects, the method comprises providing to the subject exosomes derived from fibroblasts. In some aspects, the exosomes are derived after culturing the fibroblasts in serum-free media.

Described herein, fibroblasts and/or fibroblast derived products may be administered at a concentration and frequency sufficient to reduce pathological properties of said cerebral hemorrhage to an individual having, or suspected of having, a cerebral hemorrhage. The fibroblasts and/or fibroblast-derived products may be any fibroblasts and/or fibroblast-derived products described herein. Also described herein, the fibroblasts may be allogeneic, autologous, or xenogeneic with respect to the individual being administered the fibroblasts and/or fibroblast-derived products. Also described herein, the fibroblasts may express, or be modified (such as by transfection) to express, an anti-apoptotic gene, including Bcl-2, Bcl-xL, survivin, or a combination thereof. Also described herein, the fibroblasts may be from tissue selected from the group consisting of blood, bone marrow, adipose, skin, nail, hair follicle, placenta, umbilical cord, Wharton's Jelly, mobilized peripheral blood, and a combination thereof. Also described herein, administration of the fibroblasts and/or fibroblast-derived products may reduce the growth of a hematoma intracranially, induce regression of the growth of a hematoma intracranially, induce anti-apoptotic properties in neurons in the individual, suppress microglial activation in the individual, and/or induce neuroregeneration in the individual.

BRIEF DESCRIPTION OF THE DRAWINGS

FIG. 1 shows a graph demonstrating the protection of neuron cell line PC-12 from cell death in glucose/oxygen deprivation by fibroblast conditioned media. In the groupings of three bars, from left to right the individual bars represent 0%, 5%, or 10% volume per volume.

FIG. 2 shows a graph demonstrating the protection of primary neurons from cell death in glucose/oxygen deprivation by fibroblast conditioned media. In the groupings of three bars, from left to right the individual bars represent 0%, 5%, or 10% volume per volume.

FIG. 3 shows a graph demonstrating the protection of neuron cell line PC-12 from cell death in glucose/oxygen deprivation by fibroblast exosomes. In the groupings of three bars, from left to right the individual bars represent concentration of 0, 50 ng/mL, and 100 ng/mL.

FIG. 4 shows a graph demonstrating the protection of primary neurons from cell death in glucose/oxygen deprivation by fibroblast exosomes. In the groupings of three bars, from left to right the individual bars represent concentration of 0, 50 ng/mL, and 100 ng/mL.

FIG. 5 shows a graph demonstrating the synergy of fibroblast exosomes and metformin in neuroprotection of PC-12 cells. In the groupings of four bars, from left to right the individual bars represent control, metformin alone, exosomes alone, and combination of metformin and exosomes.

FIG. 6 shows a graph demonstrating the synergy of fibroblast exosomes and metformin in neuroprotection of primary neurons. In the groupings of four bars, from left to right the individual bars represent control, metformin alone, exosomes alone, and combination of metformin and exosomes.

FIG. 7 shows a graph demonstrating the synergy of fibroblast exosomes and minocycline in neuroprotection of PC-12 cells. In the groupings of four bars, from left to right the individual bars represent control, minocycline alone, exosomes alone, and combination of metformin and exosomes.

FIG. 8 shows a graph demonstrating the synergy of fibroblast exosomes and minocycline in neuroprotection of primary neurons. In the groupings of four bars, from left to right the individual bars represent control, minocycline alone, exosomes alone, and combination of metformin and exosomes.

FIG. 9 shows the results of four treatments (control, amniotic fluid unseparated fibroblasts, CD73 selected fibroblasts, and aCD56 selected amniotic stem cells) on mean infarcted volume in a stroke rat model. desxc

DETAILED DESCRIPTION

## I. Examples of Definitions

In keeping with long-standing patent law convention, the words "a" and "an" when used in the present specification in concert with the word comprising, including the claims, denote "one or more." Some aspects of the disclosure may consist of or consist essentially of one or more elements, method steps, and/or methods of the disclosure. It is contemplated that any method or composition described herein can be implemented with respect to any other method or composition described herein and that different aspects may be combined.

As used herein, the terms "or" and "and/or" are utilized to describe multiple components in combination or exclusive of one another. For example, "x, y, and/or z" can refer to "x" alone, "y" alone, "z" alone, "x, y, and z," "(x and y) or z," "x or (y and z)," or "x or y or z." It is specifically contemplated that x, y, or z may be specifically excluded from an aspect.

Throughout this application, the term "about" is used according to its plain and ordinary meaning in the area of cell and molecular biology to indicate that a value includes the standard deviation of error for the device or method being employed to determine the value.

As used herein, "allogeneic" refers to tissues or cells or other material from another body that in a natural setting are immunologically incompatible or capable of being immunologically incompatible, although from one or more individuals of the same species.

"Autologous," as used herein, refers to cells derived from the same subject. The term "engraft" as used herein refers to the process of stem cell incorporation into a tissue of interest *in vivo* through contact with existing cells of the tissue.

As used herein, the term "approximately" or "about," as applied to one or more values of interest, refers to a value that is similar to a stated reference value. In certain aspects, the term "approximately" or "about" refers to a range of values that fall within 25%, 20%, 19%, 18%, 17%, 16%, 15%, 14%, 13%, 12%, 11%, 10%, 9%, 8%, 7%, 6%, 5%, 4%, 3%, 2%, 1%, or less in either direction (greater than or less than) of the stated reference value unless otherwise stated or otherwise evident from the context (except where such number would exceed 100% of a possible value).

As used herein, "cell line" refers to a population of cells formed by one or more subcultivations of a primary cell culture. Each round of subculturing is referred to as a passage. When cells are subcultured, they are referred to as having been passaged. A specific population of cells, or a cell line, is sometimes referred to or characterized by the number of times it has been passaged. For example, a cultured cell population that has been passaged ten times may be referred to as a P10 culture. The primary culture, i.e., the first culture following the isolation of cells from tissue, is designated P0. Following the first subculture, the cells are described as a secondary culture (P1 or passage 1). After the second subculture, the cells become a tertiary culture (P2 or passage 2), and so on. It will be understood by those of skill in the art that there may be many population doublings during the period of passaging; therefore the number of population doublings of a culture is greater than the passage number. The expansion of cells (i.e., the number of population doublings) during the period between passaging depends on many factors, including but not limited to seeding density, substrate, medium, growth conditions, and time between passaging.

As used herein, "conditioned medium" describes medium in which a specific cell or population of cells has been cultured for a period of time, and then removed, thus separating the medium from the cell or cells. When cells are cultured in a medium, they may secrete cellular factors that can provide trophic support to other cells. Such trophic factors include, but are not limited to exosomes, hormones, cytokines, extracellular matrix (ECM), proteins, vesicles, antibodies, and granules. In this example, the medium containing the cellular factors is conditioned medium.

As used herein, a "trophic factor" describes a substance that promotes and/or supports survival, growth, proliferation and/or maturation of a cell. Alternatively or in addition, a trophic factor stimulates increased activity of a cell.

The term "comprising," which is synonymous with "including," "containing," or "characterized by," is inclusive or open-ended and does not exclude additional, unrecited elements or method steps. The phrase "consisting of" excludes any element, step, or ingredient not specified. The phrase "consisting essentially of" limits the scope of described subject matter to the specified materials or steps and those that do not materially affect its basic and novel characteristics. It is contemplated that aspects described in the context of the term "comprising" may also be implemented in the context of the term "consisting of" or "consisting essentially of."

The terms "reduce," "inhibit," "diminish," "suppress," "decrease," "prevent" and grammatical equivalents (including "lower," "smaller," *etc.*) when in reference to the expression of any symptom in an untreated subject relative to a treated subject, mean that the quantity and/or magnitude of the symptoms in the treated subject is lower than in the untreated subject by any amount that is recognized as clinically relevant by any medically trained personnel. In one aspect, the quantity and/or magnitude of the symptoms in the treated subject is at least 10% lower than, at least 25% lower than, at least 50% lower than, at least 75% lower than, and/or at least 90% lower than the quantity and/or magnitude of the symptoms in the untreated subject.

As used herein, the term "therapeutically effective amount" is synonymous with "effective amount", "therapeutically effective dose", and/or "effective dose" and refers to the amount of compound that will elicit the biological, cosmetic or clinical response being sought by the practitioner in an individual in need thereof. As one example, an effective amount is the amount sufficient to reduce immunogenicity of a group of cells. The appropriate effective amount to be administered for a particular application of the disclosed methods can be determined by those skilled in the art, using the guidance provided herein. For example, an effective amount can be extrapolated from *in vitro* and *in vivo* assays as described in the present specification. One skilled in the art will recognize that the condition of the individual can be monitored throughout the course of therapy and that the effective amount of a compound or composition disclosed herein that is administered can be adjusted accordingly.

As used herein, the terms "treatment," "treat," or "treating" refers to intervention in an attempt to alter the natural course of the individual or cell being treated, and may be performed either for prophylaxis or during the course of pathology of a disease or condition. Treatment may serve to accomplish one or more of various desired outcomes, including, for example, preventing occurrence or recurrence of disease, alleviation of symptoms, and diminishment of any direct or indirect pathological consequences of the disease, preventing metastasis, lowering the rate of disease progression, amelioration or palliation of the disease state, and remission or improved prognosis.

Reference throughout this specification to "one aspect," "an aspect," "a particular aspect," "a related aspect," "a certain aspect," "an additional aspect," or "a further embodiment" or combinations thereof means that a particular feature, structure or characteristic described in connection with the aspect is included in at least one aspect of the present disclosure. Thus, the appearances of the foregoing phrases in various places throughout this specification are not necessarily all referring to the same aspect. Furthermore, the particular features, structures, or characteristics may be combined in any suitable manner in one or more aspects.

A variety of aspects of this disclosure can be presented in a range format. It should be understood that the description in range format is merely for convenience and brevity and should not be construed as an inflexible limitation on the scope of the present disclosure. Accordingly, the description of a range should be considered to have specifically disclosed all the possible subranges as well as individual numerical values within that range as if explicitly written out. For example, description of a range such as from 1 to 6 should be considered to have specifically disclosed subranges such as from 1 to 3, from 1 to 4, from 1 to 5, from 2 to 4, from 2 to 6, from 3 to 6 etc., as well as individual numbers within that range, for example, 1, 2, 3, 4, 5, and 6. This applies regardless of the breadth of the range. When ranges are present, the ranges may include the range endpoints.

The term "subject," as used herein, may be used interchangeably with the term "individual" and generally refers to an individual in need of a therapy. The subject can be a mammal, such as a human, dog, cat, horse, pig or rodent. The subject can be a patient, e.g., have or be suspected of having or at risk for having a disease or medical condition related to bone. For subjects having or suspected of having a medical condition directly or indirectly associated with bone, the medical condition may be of one or more types. The subject may have a disease or be suspected of having the disease. The subject may be asymptomatic. The subject may be of any gender. The subject may be of a certain age, such as at least 30, 35, 40, 45, 50, 55, 60, 65, 70, 75, 80, 85, 90, 95, or 100 or more.

The term "fibroblast-derived product" (also "fibroblast-associated product" or "derivatives of fibroblasts"), as used herein, refers to a molecular or cellular agent derived or obtained from one or more fibroblasts. In some cases, a fibroblast-derived product is a molecular agent. Examples of molecular fibroblast-derived products include conditioned media from fibroblast culture, extracellular vesicles obtained from fibroblasts, exosomes obtained from fibroblasts, apoptotic vesicles (or apoptotic bodies) obtained from fibroblasts, nucleic acids (e.g., DNA, RNA, mRNA, miRNA, etc.) obtained from fibroblasts, proteins (e.g., growth factors, cytokines, etc.) obtained from fibroblasts, and lipids obtained from fibroblasts. In some cases, a fibroblast-derived product is a cellular agent. Examples of cellular fibroblast-derived products include cells (e.g., stem cells, hematopoietic cells, neural cells, etc.) produced by differentiation and/or de-differentiation of fibroblasts.

As used herein, "extracellular vesicle" refers to a cell-derived vesicle comprising a membrane that encloses an internal space. Extracellular vesicles comprise all membrane-bound vesicles that have a smaller diameter than the cell from which they are derived. Generally extracellular vesicles range in diameter from 20 nm to 1000 nm, and may comprise various macromolecular cargo either within the internal space, displayed on the external surface of the extracellular vesicle, and/or spanning the membrane. Said cargo may comprise nucleic acids, proteins, carbohydrates, lipids, small molecules, and/or combinations thereof. By way of example and without limitation, extracellular vesicles include apoptotic bodies, fragments of cells, vesicles derived from cells by direct or indirect manipulation (e.g., by serial extrusion or treatment with alkaline solutions), vesiculated organelles, and vesicles produced by living cells (e.g., by direct plasma membrane budding or fusion of the late endosome with the plasma membrane). Extracellular vesicles may be derived from a living or dead organism, explanted tissues or organs, and/or cultured cells.

As used herein, "nanovesicle" refers to a cell-derived small (between 20-250 nm in diameter, or 30-150 nm in diameter) vesicle comprising a membrane that encloses an internal space, and which is generated from said cell by direct or indirect manipulation such that said nanovesicle would not be produced by said producer cell without said manipulation. Appropriate manipulations of said producer cell include but are not limited to serial extrusion, treatment with alkaline solutions, sonication, or combinations thereof. The production of nanovesicles may, in some instances, result in the destruction of said producer cell. In some aspects, populations of nanovesicles are substantially free of vesicles that are derived from producer cells by way of direct budding from the plasma membrane or fusion of the late endosome with the plasma membrane. The nanovesicle comprises lipid or fatty acid and polypeptide, and optionally comprises a payload (e.g., a therapeutic agent), a receiver (e.g., a targeting moiety), a polynucleotide (e.g., a nucleic acid, RNA, or DNA), a sugar (e.g., a simple sugar, polysaccharide, or glycan) or other molecules. The nanovesicle, once it is derived from a producer cell according to said manipulation, may be isolated from the producer cell based on its size, density, biochemical parameters, or a combination thereof.

As used herein, "exosome" refers to a cell-derived small vesicle comprising a membrane that encloses an internal space, and which is generated from said cell by direct plasma membrane budding or by fusion of the late endosome with the plasma membrane. Generally, production of exosomes does not result in the destruction of the producer cell. The exosome comprises lipid or fatty acid and polypeptide, and optionally comprises a payload (e.g., a therapeutic agent), a receiver (e.g., a targeting moiety), a polynucleotide (e.g., a nucleic acid, RNA, or DNA), a sugar (e.g., a simple sugar, polysaccharide, or glycan) or other molecules. The exosome can be derived from a producer cell, and isolated from the producer cell based on its size, density, biochemical parameters, or a combination thereof. In specific aspects, nanovesicles refer to anything that is nano-sized and is a vesicle, and in some cases may by an artifact, whereas exosomes come from a specific producer cell and is not an artifact.

As used herein, "parent cell" or "producer cell" include any cell from which an extracellular vesicle may be isolated. The terms also encompasses a cell that shares a protein, lipid, sugar, or nucleic acid component of the extracellular vesicle. For example, a "parent cell" or "producer cell" may include a cell which serves as a source for the extracellular vesicle membrane.

As used herein, "passaging" refers to the process of transferring a portion of cells from one culture vessel into a new culture vessel.

As used herein, "purify," "purified," and "purifying" or "isolate," "isolated," or "isolating" or "enrich," "enriched" or "enriching" when referring to extracellular vesicles are used interchangeably and refer to the state of a population (e.g., a plurality of known or unknown amount and/or concentration) of desired extracellular vesicles, that have undergone one or more processes of purification, e.g., a selection or an enrichment of the desired extracellular vesicles composition, or alternatively a removal or reduction of residual biological products as described herein. In some aspects, a purified extracellular vesicles composition has no detectable undesired activity or, alternatively, the level or amount of the undesired activity is at or below an acceptable level or amount. In other aspects, a purified extracellular vesicle composition has an amount and/or concentration of desired extracellular vesicles at or above an acceptable amount and/or concentration. In other aspects, the purified extracellular vesicle composition is enriched as compared to the starting material (e.g., biological material collected from tissue, bodily fluid, or cell preparations) from which the composition is obtained. This enrichment may be by 10%, 20%, 30%, 40%, 50%, 60%, 70%, 80%, 90%, 95%, 96%, 97%, 98%, 99%, 99.9%, 99.99%, 99.999%, 99.9999%, or greater than 99.9999% as compared to the starting material.

As used herein, "payload" as used herein is a therapeutic agent that acts on a target (e.g. a target cell) that is contacted with the extracellular vesicles. Payloads that may be introduced into a extracellular vesicles and/or a producer cell include therapeutic agents such as, nucleotides (e.g. nucleotides comprising a detectable moiety or a toxin or that disrupt transcription), nucleic acids (e.g. DNA or mRNA molecules that encode a polypepetide such as an enzyme, or RNA molecules that have regulatory function such as miRNA, dsDNA, lncRNA, siRNA), amino acids (e.g. amino acids comprising a detectable moiety or a toxin or that disrupt translation), polypeptides (e.g. enzymes), lipids, carbohydrates, and small molecules (e.g. small molecule drugs and toxins). The payload may comprise nucleotides, e.g. nucleotides that are labeled with a detectable or cytotoxic moiety (e.g. a radiolabel).

As used herein, "transgene" or "exogenous nucleic acid" refers to a foreign or native nucleotide sequence that is introduced into an extracellular vesicle. Transgene and exogenous nucleic acid are used interchangeably herein and encompass recombinant nucleic acids.

As used herein, "hemostasis" refers to the arrest of bleeding, involving the physiological process of blood coagulation at ruptured or punctured blood vessels and possibly the contraction of damaged blood vessels.

As used herein, "inflammation" or "inflammatory" refers to the reaction of living tissues to injury, infection, or irritation. Anything that stimulates an inflammatory response is said to be inflammatory.

As used herein, "transplant" refers to the transplantation of an organ or body part from one organism to another.

## II. Methods for Disease Treatment or Prevention

Aspects of the present disclosure are directed to methods for treatment or prevention of disorders of the central nervous system (CNS). In some aspects, disclosed are methods for treatment or prevention of stroke. Also disclosed are compositions useful for such methods, including pharmaceutical compositions. In some aspects, disclosed are methods for treatment or prevention of stroke comprising providing to a subject an effective amount of fibroblasts, conditioned media (or components thereof) derived from fibroblasts, and/or exosomes derived from fibroblasts. In some aspects, compositions are provided to a subject subsequent to a stroke. In these examples, treating a subject for stroke comprises reducing the damage to the subject from the stroke, which can include reducing neuroinflammation, inducing neuroregeneration, or inducing immune modulation and subsequent neuroprotection. In some aspects, compositions are provided to a subject prior to a stroke, for example to a patient at risk for developing a stroke. In these examples, methods comprise preventing a stroke from occurring or delaying the onset of a stroke.

In some aspects, the cells of the present disclosure are administered to a subject suffering from a disease, disorder or condition involving the CNS. In some aspects, the condition is a stroke. Cells of the disclosure, including fibroblasts, may be administered to a subject suffering from a disease, disorder or condition involving the CNS. In some aspects, the fibroblasts and/or other cells of the disclosure are administered intravenously and/or to the brain or spinal cord of the subject. In some instances, the cells are administered to the adjacent site of injury in the subject's brain. The precise site of administration of the cells may depend on any number of factors, including but not limited to the site of the lesion to be treated, the type of disease being treated, the age of the subject, the severity of the disease, and the like. As disclosed herein, the cells can be administered to the subject via intracarotid or intravenous routes.

In some aspects, fibroblasts, conditioned media derived from fibroblasts, and/or exosomes derived from fibroblasts are provided to a subject together with one or more additional components. In some aspects, fibroblasts are provided to a subject prior to providing one or more additional components. In some aspects, fibroblasts are provided to a subject after providing one or more additional components. In some aspects, fibroblasts are provided to a subject substantially simultaneously with providing one or more additional components. The one or more additional components may include, for example, a TNF-α inhibitor, an agent capable of inhibiting responsiveness to TNF-α, an anti-apoptotic agent, endothelial progenitor cells, regenerative cells (e.g., stem cells), an anti-inflammatory agent, a neuroprotective agent, an agent capable of enhancing neurogenesis, or a combination thereof.

In some aspects, fibroblasts, conditioned media derived from fibroblasts, and/or exosomes derived from fibroblasts are provided in combination with a TNF-α inhibitor. A TNF-α inhibitor may be any agent capable of preventing TNF-α from binding to or activating a TNF-α receptor. In some aspects, the TNF-α inhibitor is melatonin. In some aspects, the TNF-α inhibitor is cycloheximide, auranofin, sodium aurothiomalate, Leukotriene B4, interleukin-4, interleukin-13, polymyxin B, bile acids, interleukin-6, lactulose, oxpentifylline, mometasone, glucocorticoids, colchicine, chloroquine, FK-506, berberine, resveratrol, pterostilbene, vitamin A, vitamin C, cyclosporine, phosphodiesterase inhibitors such as vinpocetine, milrinone, CI-930, rolipram, nitroquazone, zaprinast, synthetic lipid A, amrinone, N-acetylcysteine, dithiocarbamates and metal chelators, exosurf synthetic surfactant, dehydroepiandrosterone, delta-tetrahydrocannabinol, phosphatidylserine, TCV-309, a PAF antagonist, thalidomide, a cytochrome p450 inhibitor, cytochalasin D, ketamine, TGF-beta, interleukin-10, pentoxifylline, BRL 61,063, a calcium antagonist, curcumin, kappa-selective opioid agoinst U50,488H (trans-3,4-dichloro-N-methyl-N-[7-(1-pyrrolidinyl)cyclohexyl]benzene-acetamide methanesulfonate), alendronate, tetrandrine, sulfasalazine, epinephrine, BMS-182123, adenosine, E3330, nicotine, IVIG, cardiotrophin-1, KB-R7785, CGRP, ligustrazine, dexanabinol, iloprost, activated protein C, a growth hormone, spermine, FR-167653, gm-6001, estradiol, aspirin, or amiodarone.

In some aspects, fibroblasts, conditioned media derived from fibroblasts, and/or exosomes derived from fibroblasts are provided to a subject in combination with an agent capable of inhibiting responsiveness to TNF-α. An agent capable of inhibiting responsiveness to TNF-α may be any agent capable of reducing or preventing an immune response caused by TNF-α stimulation in a subject. In some aspects, the agent capable of inhibiting responsiveness to TNF-α is ibuprofen, indomethacin, Nedocromil sodium, cromolyn (sodium cromoglycate), a spleen derived factor, pentoxifylline, NG-methyl-L-arginine, dexamethasone, chlorpromazine, activated alpha 2 macroglobulin, serum amyloid A protein, a neutrophil derived proteolytic enzyme, phentolamine, propranolol, a leukotriene inhibitor, nordihydroguaiaretic acid, genistein, butylated hydroxyanisole, CNI-1493, quercetin, gabexate mesylate, SM-12502, monoclonal nonspecific suppressor factor (MNSF), pyrrolidine dithiocarbamate (PDTC), or aprotinin.

In some aspects, fibroblasts, conditioned media derived from fibroblasts, and/or exosomes derived from fibroblasts are provided to a subject in combination with an anti-apoptotic agent. An anti-apoptotic agent may be any agent capable of inducing apoptosis in a cell. In some aspects, an anti-apoptotic agent is a caspase inhibitor. In some aspects, a caspase inhibitor is a caspase-3 inhibitor or a caspase-9 inhibitor. In some aspects, the anti-apoptotic agent is EGF, FGF, carbon monoxide, FEDVI peptide, or TGF-β.

In some aspects, fibroblasts, conditioned media derived from fibroblasts, and/or exosomes derived from fibroblasts are provided to a subject in combination with an additional agent selected from n-acetylcysteine, ascorbic acid, alpha lipoic acid, human chorionic gonadotropin, VEGF, TNF-α, retinoic acid, alpha tocopherol, interleukin-3, G-CSF, GM-CSF, leukemia inhibitory factor, placental growth factor, angiopoietin, hydrogenated water, and NGF.

In some aspects, fibroblasts, conditioned media derived from fibroblasts, and/or exosomes derived from fibroblasts are provided to a subject in combination with endothelial progenitor cells (EPCs). The EPCs may be allogenic or autologous. EPCs may be derived from one or more sources, including peripheral blood, mobilized peripheral blood, bone marrow, adipose derived stromal vascular fraction, cord blood, Wharton's jelly, and placenta. In some aspects, EPCs are derived from peripheral blood.

In some aspects, fibroblasts, conditioned media derived from fibroblasts, and/or exosomes derived from fibroblasts are provided to a subject in combination with a one or more regenerative cells. A regenerative cell may be a stem cell. In some aspects, fibroblasts are provided to a subject in combination with one or more hematopoietic stem cells. The hematopoietic stem cells may be autologous, allogenic, or xenogenic. Hematopoietic stem cells may be derived from one or more sources including adipose, bone marrow, peripheral blood, mobilized peripheral blood, and cord blood. In some aspects, hematopoietic stem cells are derived from bone marrow. In some aspects, the hematopoietic stem cells express CD34, CD133, and/or c-kit. In some aspects, the hematopoietic stem cells do not express CD38 or thrombopoietin. In some aspects, fibroblasts are provided to a subject in combination with one or more mesenchymal stem cells. The mesenchymal stem cells may be autologous, allogenic, or xenogenic. Mesenchymal stem cells may be derived from one or more sources including adipose, bone marrow, peripheral blood, mobilized peripheral blood, menstrual blood, fallopian tube, or cord blood. In some aspects, the mesenchymal stem cells express CD90, CD105, and/or CD73. In some aspects, the mesenchymal stem cells do not express HLA, CD34, or CD14.

In some aspects, fibroblasts, conditioned media derived from fibroblasts, and/or exosomes derived from fibroblasts are provided to a subject in combination with a one or more anti-inflammatory agents. An anti-inflammatory agent may be any agent capable of reducing or preventing an immune response in a subject. In some aspects, the one or more anti-inflammatory agents comprise interleukin-10 (IL-10), pentoxyfilline, COX-2 inhibitors, 21-acetoxypregnenolone, alclometasone, algestone, amcinonide, beclomethasone, betamethasone, budesonide, chloroprednisone, clobetasol, clobetasone, clocortolone, cloprednol, corticosterone, cortisone, cortivazol, deflazacort, desonide, desoximetasone, dexamethasone, diflorasone, diflucortolone, difluprednate, enoxolone, fluazacort, flucloronide, flumethasone, flunisolide, fluocinolone acetonide, fluocinonide, fluocortin butyl, fluocortolone, fluorometholone, fluperolone acetate, fluprednidene acetate, fluprednisolone, flurandrenolide, fluticasone propionate, formocortal, halcinonide, halobetasol propionate, halometasone, halopredone acetate, hydrocortamate, hydrocortisone, loteprednol etabonate, mazipredone, medrysone, meprednisone, methylprednisolone, mometasone furoate, paramethasone, prednicarbate, prednisolone, prednisolone 25-diethylamino-acetate, prednisolone sodium phosphate, prednisone, prednival, prednylidene, rimexolone, tixocortol, triamcinolone, triamcinolone acetonide, triamcinolone benetonide, triamcinolone hexacetonide, aminoarylcarboxylic acid derivatives (e.g., enfenamic acid, etofenamate, flufenamic acid, isonixin, meclofenamic acid, mefenamic acid, niflumic acid, talniflumate, terofenamate, tolfenamic acid), arylacetic acid derivatives (e.g., aceclofenac, acemetacin, alclofenac, amfenac, amtolmetin guacil, bromfenac, bufexamac, cinmetacin, clopirac, diclofenac sodium, etodolac, felbinac, fenclozic acid, fentiazac, glucametacin, ibufenac, indomethacin, isofezolac, isoxepac, lonazolac, metiazinic acid, mofezolac, oxametacine, pirazolac, proglumetacin, sulindac, tiaramide, tolmetin, tropesin, zomepirac), arylbutyric acid derivatives (e.g., bumadizon, butibufen, fenbufen, xenbucin), arylcarboxylic acids (e.g., clidanac, ketorolac, tinoridine), arylpropionic acid derivatives (eg., alminoprofen, benoxaprofen, bermoprofen, bucloxic acid, carprofen, fenoprofen, flunoxaprofen, flurbiprofen, ibuprofen, ibuproxam, indoprofen, ketoprofen, loxoprofen, naproxen, oxaprozin, piketoprolen, pirprofen, pranoprofen, protizinic acid, suprofen, tiaprofenic acid, ximoprofen, zaltoprofen), pyrazoles (e.g., difenamizole, epirizole), pyrazolones (e.g., apazone, benzpiperylon, feprazone, mofebutazone, morazone, oxyphenbutazone, phenylbutazone, pipebuzone, propyphenazone, ramifenazone, suxibuzone, thiazolinobutazone), salicylic acid derivatives (e.g., acetaminosalol, aspirin, benorylate, bromosaligenin, calcium acetylsalicylate, diflunisal, etersalate, fendosal, gentisic acid, glycol salicylate, imidazole salicylate, lysine acetylsalicylate, mesalamine, morpholine salicylate, 1-naphthyl salicylate, olsalazine, parsalmide, phenyl acetylsalicylate, phenyl salicylate, salacetamide, salicylamide o-acetic acid, salicylsulfuric acid, salsalate, sulfasalazine), thiazinecarboxamides (e.g., ampiroxicam, droxicam, isoxicam, lornoxicam, piroxicam, tenoxicam), epsilon.-acetamidocaproic acid, s-adenosylmethionine, 3-amino-4-hydroxybutyric .acid, amixetrine, bendazac, benzydamine, .alpha.-bisabolol, bucolome, difenpiramide, ditazol, emorfazone, fepradinol, guaiazulene, nabumetone, nimesulide, oxaceprol, paranyline, perisoxal, proquazone, superoxide dismutase, tenidap, zileuton, candelilla wax, alpha bisabolol, aloe vera, Manjistha, Guggal, kola extract, chamomile, sea whip extract, glycyrrhetic acid, glycyrrhizic acid, oil soluble licorice extract, monoammonium glycyrrhizinate, monopotassium glycyrrhizinate, dipotassium glycyrrhizinate, 1-beta-glycyrrhetic acid, stearyl glycyrrhetinate, 3-stearyloxy-glycyrrhetinic acid, or a combination thereof.

In some aspects, fibroblasts, conditioned media derived from fibroblasts, and/or exosomes derived from fibroblasts are provided to a subject in combination with a one or more neuroprotective agents. A neuroprotective agent may be an agent capable of protecting a neuron from cell death. In some aspects, the one or more neuroprotective agents comprise erythropoietin, human chorionic gonadotropin, metformin, GLP-1, dimethylsulfoxide, felbamate, TGF-beta, aurintricarboxylic acid, amlodipine, MK-801, memantine, irbesartan, ebselen, green tea extract, BMP-6, minocycline, doxycycline, ceftriaxone, or a combination thereof.

In some aspects, fibroblasts, conditioned media derived from fibroblasts, and/or exosomes derived from fibroblasts are provided to a subject in combination with a one or more agents capable of enhancing neurogenesis. In some aspects, the one or more agents capable of enhancing neurogenesis comprise metformin.

The fibroblast cells of the present disclosure can be used to secrete an angiogenic hormone including but not limited to vascular growth factor, endothelial cell growth factor, and the like. Fibroblasts can be used to induce angiogenesis within a tissue in which various progenitor cells are present (e.g., neuronal tissue). Thus, in some aspects, a method of promoting neovascularization within a tissue using such cells is provided. In accordance with this method, cells may be introduced to the desired tissue under conditions sufficient for the cell to produce the angiogenic hormone. The presence of the hormone within the tissue may promote neovascularization within the tissue. In some aspects, promotion of neovascularization serves to treat or prevent a stroke in a subject.

In some aspects, fibroblasts, conditioned media derived from fibroblasts, and/or exosomes derived from fibroblasts are administered intravenously to a subject subsequent to a stroke, thereby decreasing an ischemic penumbra. An ischemic penumbra describes a region of reduced cerebral blood flow following a stroke. Administration of fibroblasts may serve to prevent cell death in an ischemic penumbra by, for example, promoting angiogenesis or stimulating cell growth. In some aspects, fibroblasts are administered to enhance activity of neuroprotective agents, which agents are provided together with the fibroblasts. In some aspects, the neuroprotective agents comprise calcium channel antagonists, N-methyl-D-aspartate (NMDA) receptor antagonists, free radical scavengers, anti-intercellular adhesion molecule 1 antibody, GM-1 ganglioside, gamma.-aminobutyric acid agonists, sodium channel antagonists, or a combination thereof.

The mode of administration of therapeutic compositions (e.g., fibroblasts, conditioned media derived from fibroblasts, and/or exosomes derived from fibroblasts) to the CNS of the subject may vary depending on several factors including the type of disease being treated, the age of the subject, whether the cells to be provided are differentiated, whether cells to be provided comprise heterologous DNA, and the like. Cells may be transplanted, grafted, infused, or otherwise introduced into a subject. In an example, cells are introduced into the brain of a subject by intracerebral or intravascular transplantation. A therapeutic composition of the disclosure may be introduced to the desired site by direct injection. A therapeutic composition of the disclosure may be administered into a host in a wide variety of ways. Example modes of administration are intravascular, intracerebral, parenteral, intraperitoneal, intravenous, epidural, intraspinal, intrastemal, intra-articular, intra-synovial, intrathecal, intra-arterial, intracardiac, and intramuscular. In some aspects, fibroblasts, conditioned media derived from fibroblasts, and/or exosomes derived from fibroblasts are administered to the brain by direct transplantation. In some aspects, fibroblasts, conditioned media derived from fibroblasts, and/or exosomes derived from fibroblasts are administered to the central nervous system (e.g., the spinal cord) by simple injection.

In some aspects, cells disclosed herein (e.g., fibroblasts) are from a donor that has been screened and that is genetically different than the subject for used (e.g., allogeneic transplantation). One example of administration of therapeutic cells for the disclosed methods is as follows. The blood type or haplotype compatibility is determined between the donor cells and the patient. Cells may be administered intravenously, or in some situations locally. Surgery may be performed using a Brown-Roberts-Wells computed tomographic (CT) stereotaxic guide. The patient is given local anesthesia in the scalp area and intravenously administered midazolam. The patient undergoes CT scanning to establish the coordinates of the region to receive the transplant. The injection cannula may consist of a 17-gauge stainless steel outer cannula with a 19-gauge inner stylet. This is inserted into the brain to the correct coordinates, then removed and replaced with a 19-gauge infusion cannula that has been preloaded with about 30 μl of tissue suspension. The cells are slowly infused at a rate of about 3 μL/min as the cannula is withdrawn. Multiple stereotactic needle passes are made throughout the area of interest, approximately 4 mm apart. The patient is examined by CT scan postoperatively for hemorrhage or edema. Neurological evaluations are performed at various post-operative intervals, as well as PET scans to determine metabolic activity of the implanted cells.

In some aspects, between about $10^5$ and about $10^{13}$ cells per 100 kg are administered to a human per infusion. In some aspects, between about $1.5 \times 10^6$ and about $1.5 \times 10^{12}$ cells are infused per 100 kg. In some aspects, between about $1 \times 10^9$ and about $5 \times 10^{11}$ cells are infused per 100 kg. In some aspects, between about $4 \times 10^9$ and about $2 \times 10^{11}$ cells are infused per 100 kg. In some aspects, between about $5 \times 10^8$ cells and about $1 \times 10^1$ cells are infused per 100 kg. In some aspects, a single administration of cells is provided. In some aspects, multiple administrations are provided. In some aspects, multiple administrations are provided over the course of 3-7 consecutive days. In some aspects, 3-7 administrations are provided over the course of 3-7 consecutive days. In some aspects, 5 administrations are provided over the course of 5 consecutive days. In some aspects, a single administration of between about $10^5$ and about $10^{13}$ cells per 100 kg is provided. In some aspects, a single administration of between about $1.5 \times 10^8$ and about $1.5 \times 10^{12}$ cells per 100 kg is provided. In some aspects, a single administration of between about $1 \times 10^9$ and about $5 \times 10^{11}$ cells per 100 kg is provided. In some aspects, a single administration of about $5 \times 10^{10}$ cells per 100 kg is provided. In some aspects, a single administration of $1 \times 10^{10}$ cells per 100 kg is provided. In some aspects, multiple administrations of between about $10^5$ and about $10^{13}$ cells per 100 kg are provided. In some aspects, multiple administrations of between about $1.5 \times 10^8$ and about $1.5 \times 10^{12}$ cells per 100 kg are provided. In some aspects, multiple administrations of between about $1 \times 10^9$ and about $5 \times 10^{11}$ cells per 100 kg are provided over the course of 3-7 consecutive days. In some aspects, multiple administrations of about $4 \times 10^9$ cells per 100 kg are provided over the course of 3-7 consecutive days. In some aspects, multiple administrations of about $2 \times 10^{11}$ cells per 100 kg are provided over the course of 3-7 consecutive days. In some aspects, 5 administrations of about $3.5 \times 10^9$ cells are provided over the course of 5 consecutive days. In some aspects, 5 administrations of about $4 \times 10^9$ cells are provided over the course of 5 consecutive days. In some aspects, 5 administrations of about $1.3 \times 10^{11}$ cells are provided over the course of 5 consecutive days. In some aspects, 5 administrations of about $2 \times 10^{11}$ cells are provided over the course of 5 consecutive days.

It is contemplated that methods and compositions comprising fibroblasts may comprise, in addition or in place of the fibroblasts, conditioned media from fibroblasts and/or exosomes derived from fibroblasts. As such, where methods discussed describe aspects comprising the use of fibroblasts, also contemplated are aspects where conditioned media and/or exosomes derived from fibroblasts are substituted for the fibroblasts. One or more of fibroblasts, conditioned media from fibroblasts, and exosomes derived from fibroblasts may be used alternatively or in combination in the disclosed methods and compositions.

In some aspects, fibroblasts are used as precursor cells and differentiated prior to or following administration to a subject. Fibroblasts may be differentiated *in vitro, ex vivo,* or *in vivo.* For example, fibroblasts can be used as precursor cells that differentiate (*e.g.,* into neural cells) *in vivo* following introduction into the CNS. In another example, cells are used which have been differentiated (*e.g.,* into neural cells) *ex vivo* or *in vitro* prior to introduction into the CNS. Fibroblasts can be differentiated to express at least one characteristic of a cell of the CNS including, but not limited to class III β-tubulin, the M subunit of neurofilaments, tyrosine hydroxylase, gluatmate receptor subunits of the GluR1-4 and GluR6 classes, glial fibrillary acidic protein, myelin basic protein, brain factor 1, NeuN, NF-M, NSE, nestin, and trkA. Further, the disclosure herein demonstrates that following introduction of fibroblasts into a mammal, the cells can secrete various factors. Such factors include, but are not limited to, growth factors, trophic factors and cytokines. In some instances, the secreted factors can have a therapeutic effect in the subject. The secreted factors can activate the originating cell. In addition, the secreted factors can activate neighboring and/or distal endogenous cells to stimulate proliferation and/or differentiation. In some aspects, a fibroblast secretes a cytokine or growth hormone such as human growth factor, fibroblast growth factor, nerve growth factor, insulin-like growth factors, hemopoietic stem cell growth factors, members of the fibroblast growth factor family, members of the platelet-derived growth factor family, vascular and endothelial cell growth factors, or members of the TGFβ family.

### III. Treatment of Cerebral Hemorrhage

Described herein are methods of ameliorating one or more effects of a cerebral hemorrhage in a subject comprising administering fibroblasts and/or modified fibroblasts and/or derivatives of fibroblasts. Also described herein, ameliorating one or more effects of a cerebral hemorrhage may comprise a reduction or prevention of any disease, symptom, and/or complication associated with cerebral hemorrhage. For example, administration of fibroblasts and/or modified fibroblasts and/or derivatives of fibroblasts may prevent or decrease the occurrence of neurological damage. Also described herein, the methods disclosed may be capable of suppressing complications of various types of hemorrhage which include sepsis and multiple organ failure. Also described herein, hemorrhagic shock, in addition to directly resulting in early fatality, may be a predictor of poor outcome in the injured patient. Also described herein, early hypotension (systolic blood pressure<90 mmHg) with hemorrhage in the field or at initial hospital evaluation may be associated with complications such as eventual organ failure and the development of infections, including sepsis. Further described herein, as a critically injured patient progresses through the phases of trauma care, death from causes unrelated to specific injuries may become more common. Also described herein, infections such as sepsis and pneumonia, systemic inflammatory response syndrome, and multiple organ failure may become the primary etiologies of traumatic death in the trauma patient. Also described herein, administration of fibroblasts and/or modified fibroblasts and/or derivatives of fibroblasts may reduce or prevent infection complications such as sepsis and/or pneumonia or other infections, inflammation, and/or organ failure.

In certain aspects, fibroblasts, modified fibroblasts, and/or derivatives of fibroblasts are administered to an individual to suppress the development of an immune deficiency that occurs after induction of trauma. It is known that severe infections, such as those accompanying traumatic injury, can be associated with sepsis. Sepsis, also known as systemic inflammatory response syndrome (SIRS), is a severe illness caused by overwhelming infection of the bloodstream by toxin-producing bacteria. Sepsis can be caused by bacterial infection that can originate anywhere in the body. Common sites include, but are not limited to, the kidneys (upper urinary tract infection), the liver or the gall bladder, the bowel (usually seen with peritonitis), the skin (cellulitis), and the lungs (bacterial pneumonia). In sepsis, blood pressure drops, resulting in shock. Major organs and systems, including the kidneys, liver, lungs, and central nervous system, stop functioning normally. Sepsis is often life-threatening, especially in people with a weakened immune system or other medical illnesses. Certain aspects concern the inhibition of developing sepsis subsequent to hemorrhage.

Described herein the process of cerebral hemorrhage evoking neuronal loss may be associated with multiple factors. One known factor may be activation of the clotting cascade, which is involved in actual expansion of the local volume surrounding the hemorrhage. In one study, local thrombin expression after hemorrhage induction in a mouse model was assessed. Hemorrhage in the intracerebral area was established, using stereotaxic orthotopic injection of autologous blood or silicone oil as inert volume substance into the striatum. Intracranial pressure (ICP), cerebral blood flow (CBF) and arterial blood pressure (MAP) were monitored during and 30 min after the procedure. No significant differences between ICP, CBF, and MAP were found between both groups. Prothrombin mRNA expression was upregulated early after ICH. Immunohistochemistry showed an increase of perilesional thrombin in both groups (blood 4.24-fold, silicone 3.10-fold), while prothrombin fragment (F1.2) was elevated only in the absence of whole blood. Thrombin expression is co-localized with neuronal antigen expression (NeuN). After 24 hrs, lesion size and neuronal loss were similar. Perihematomal thrombin correlated with increased neuronal loss and detrimental neurological outcome in-vivo [16].

Means of shrinking the area of hematoma formation subsequent to administration of fibroblasts, modified fibroblasts, and/or derivatives of fibroblasts are disclosed in aspects herein. In some aspects, fibroblasts, modified fibroblasts, and/or derivatives of fibroblasts are administered together with agents known to facilitate the shrinkage of hematoma formation. Such agents include statin drugs [17-20], urokinase [21-23], tissue plasminogen activator [24-28], endothelin inhibitors [29-31], muscimol [32], complement inhibition [33, 34], argatroban [35, 36], AMPA receptor antagonists [37], steroids [38], inhibitors of mast cell activation [39], memantine [40], proteasome inhibitors [41, 42], NMDA receptor antagonists [43, 44], ferritin reducing agents [45], edaravone [34, 46], protease inhibitors [47], chelating agents [48-50], ApoE mimetic peptides [51], retinoids [52], microglial inhibitors [53], xenon [54], TNF-alpha blockade [55, 56], and cox-2 inhibitors [57].

In some aspects, fibroblasts are utilized which possess ability to differentiate into other tissues. The disclosure provides the relationship between differentiation ability of fibroblasts and immune modulatory/anti-inflammatory activity. Generation of fibroblasts capable of possessing therapeutic activity may be performed in some aspects of the disclosure based on previous protocols that have been reported by other investigators. For example, it has been demonstrated that cultured human adult bronchial fibroblasts- (Br) are similar to other regenerative cells such as mesenchymal cell progenitors isolated from fetal lung (ICIG7) and adult bone marrow (BM212) tissues. Surface immunophenotyping by flow cytometry revealed a similar expression pattern of antigens characteristic of marrow-derived MSCs, including CD34 (-), CD45 (-), CD90/Thy-1 (+), CD73/SH3, SH4 (+), CD105/SH2 (+) and CD166/ALCAM (+) in Br, ICIG7 and BM212 cells. There was one exception, STRO-1 antigen, which was only weakly expressed in Br cells. Analysis of cytoskeleton and matrix composition by immunostaining showed that lung and marrow-derived cells homogeneously expressed vimentin and nestin proteins in intermediate filaments while they were all devoid of epithelial cytokeratins. Additionally, alpha-smooth muscle actin was also present in microfilaments of a low number of cells. All cell types predominantly produced collagen and fibronectin extracellular matrix as evidenced by staining with the monoclonal antibodies for collagen prolyl 4-hydroxylase and fibronectin isoforms containing the extradomain (ED)-A together with ED-B in ICIG7 cells. Br cells similarly to fetal lung and marrow fibroblasts were able to differentiate along the three adipogenic, osteogenic and chondrogenic mesenchymal pathways when cultured under appropriate inducible conditions. Altogether, this data indicates that MSCs are present in human adult lung. They may be actively involved in lung tissue repair under physiological and pathological circumstances [58].

Other types of fibroblasts and conditions for growth may be utilized. For example in one study (59), juvenile foreskin fibroblasts were examined. These cells were shown to share a mesenchymal stem cell phenotype and multi-lineage differentiation potential. Specifically, the investigators demonstrated similar expression patterns for CD14(-), CD29(+), CD31(-), CD34(-), CD44(+), CD45(-), CD71(+), CD73/SH3-SH4(+), CD90/Thy-1(+), CD105/SH2(+), CD133(-) and CD166/ALCAM(+) in well-established adipose tissue derived-stem cells and foreskin fibroblastic cells by flow cytometry. Immunostainings showed fibroblast cells strongly expressed vimentin, fibronectin and collagen, they were moderately expressed for alpha-smooth muscle actin and nestin, while they were negative for epithelial cytokeratins. When cultured under appropriate inducible conditions, both cell types could differentiate along the adipogenic and osteogenic lineages. Additionally, fibroblasts demonstrated a higher proliferation potential than mesenchymal stem cells. These findings are of particular importance, because skin or adipose tissues are easily accessible for cell transplantations in regenerative medicine [59]. Verification of multi-lineage differentiation of foreskin fibroblasts was provided by a study in which foreskin fibroblasts where demonstrated to possess shorter doubling time than MSC, as well as ability to multiply more than 50 doublings without undergoing senescence. The cells were positive for the MSC markers CD90, CD105, CD166, CD73, SH3, and SH4, and could be induced to differentiate into osteocytes, adipocytes, neural cells, smooth muscle cells, Schwann-like cells, and hepatocyte-like cells [60].

In some aspects, fibroblasts are used to differentiate into cells possessing one or more properties of neurons cells (for example, the ability to express GABA), or neuron cells themselves, and/or administration of one or more agents that act as regenerative adjuvants (such as one or more histone deacetylase inhibitors, one or more DNA methyltransferase inhibitors, and/or one or more GSK-3 inhibitors) for said fibroblasts to generate neurons. The fibroblasts in the formulation may display typical fibroblast morphologies when growing in cultured monolayers. Specifically, cells may display an elongated, fusiform or spindle appearance with slender extensions, or cells may appear as larger, flattened stellate cells, which may have cytoplasmic leading edges. A mixture of these morphologies may also be observed. The cells may express proteins characteristic of normal fibroblasts including the fibroblast-specific marker, CD90 (Thy-1), a 35 kDa cell-surface glycoprotein, and the extracellular matrix protein, collagen. In some aspects, an autologous cell therapy product composed of a suspension of autologous fibroblasts, grown from a biopsy of each individual's own skin using standard tissue culture procedures is used for the methods described herein. In one aspect, the fibroblasts are used to create other cell types for tissue repair or regeneration. Generation of fibroblasts has been described previously in the art [61-71].

The fibroblasts may be generated by outgrowth from a biopsy of the recipient's own skin (in the case of autologous preparations), or skin of healthy donors (for allogeneic preparations). In some aspects, fibroblasts are used from young donors. In particular aspects, fibroblasts are transfected with genes to allow for enhanced growth and overcoming of the Hayflick limit. Subsequent to derivation of cells expansion in culture using standard cell culture techniques, skin tissue (dermis and epidermis layers) may be biopsied from a subject's post-auricular area. In certain aspects, the starting material is composed of three 3-mm punch skin biopsies collected using standard aseptic practices. The biopsies are collected by the treating physician, placed into a vial containing sterile phosphate buffered saline (PBS). The biopsies are shipped in a 2-8°C. refrigerated shipper back to the manufacturing facility. In one aspect, after arrival at the manufacturing facility, the biopsy is inspected and, upon acceptance, transferred directly to the manufacturing area. Upon initiation of the process, the biopsy tissue is then washed prior to enzymatic digestion. After washing, a Liberase Digestive Enzyme Solution is added without mincing, and the biopsy tissue is incubated at 37.0 ± 2°C. for one hour. Time of biopsy tissue digestion is a critical process parameter that can affect the viability and growth rate of cells in culture. Liberase is a collagenase/neutral protease enzyme cocktail obtained formulated from Lonza Walkersville, Inc. (Walkersville, Md.) and unformulated from Roche Diagnostics Corp. (Indianapolis, Ind.). Alternatively, other commercially available collagenases may be used, such as Serva Collagenase NB6 (Helidelburg, Germany). After digestion, Initiation Growth Media (IMDM, GA, 10% Fetal Bovine Serum (FBS)) is added to neutralize the enzyme, cells are pelleted by centrifugation and resuspended in 5.0 mL Initiation Growth Media. Alternatively, centrifugation is not performed, with full inactivation of the enzyme occurring by the addition of Initiation Growth Media only. Initiation Growth Media is added prior to seeding of the cell suspension into a T-175 cell culture flask for initiation of cell growth and expansion. A T-75, T-150, T-185 or T-225 flask can be used in place of the T-75 flask.

Cells are incubated at 37 ± 2.0°C. with 5.0 ± 1.0% $CO_2$ and fed with fresh Complete Growth Media every three to five days. All feeds in the process are performed by removing half of the Complete Growth Media and replacing the same volume with fresh media. Alternatively, full feeds can be performed. Cells should not remain in the T-175 flask greater than 30 days prior to passaging. Confluence is monitored throughout the process to ensure adequate seeding densities during culture splitting. When cell confluence is greater than or equal to 40% in the T-175 flask, they are passaged by removing the spent media, washing the cells, and treating with Trypsin-EDTA to release adherent cells in the flask into the solution. Cells are then trypsinized and seeded into a T-500 flask for continued cell expansion. Alternately, one or two T-300 flasks, One Layer Cell Stack (1 CS), One Layer Cell Factory (1 CF) or a Two Layer Cell Stack (2 CS) can be used in place of the T-500 Flask.

Morphology may be evaluated at each passage and prior to harvest to monitor the culture purity throughout the culture purity throughout the process. Morphology may be evaluated by comparing the observed sample with visual standards for morphology examination of cell cultures. The cells display typical fibroblast morphologies when growing in cultured monolayers. Cells may display either an elongated, fusiform or spindle appearance with slender extensions, or appear as larger, flattened stellate cells which may have cytoplasmic leading edges. A mixture of these morphologies may also be observed. Fibroblasts in less confluent areas can be similarly shaped, but randomly oriented. The presence of keratinocytes in cell cultures is also evaluated. Keratinocytes appear round and irregularly shaped and, at higher confluence, they appear organized in a cobblestone formation. At lower confluence, keratinocytes are observable in small colonies. Cells are incubated at $37 \pm 2.0°C$. with $5.0 \pm 1.0\%$ $CO_2$ and passaged every three to five days in the T-500 flask and every five to seven days in the ten layer cell stack (10CS). Cells should not remain in the T-500 flask for more than 10 days prior to passaging.

Quality Control (QC) release testing for safety of the Bulk Drug Substance (the cells or derivatives thereof) includes sterility and endotoxin testing. When cell confluence in the T-500 flask is ~95%, cells are passaged to a 10 CS culture vessel. Alternately, two Five Layer Cell Stacks (5 CS) or a 10 Layer Cell Factory (10 CF) can be used in place of the 10 CS. Passage to the 10 CS is performed by removing the spent media, washing the cells, and treating with Trypsin-EDTA to release adherent cells in the flask into the solution. Cells are then transferred to the 10 CS. Additional Complete Growth Media is added to neutralize the trypsin and the cells from the T-500 flask are pipetted into a 2 L bottle containing fresh Complete Growth Media. The contents of the 2 L bottle are transferred into the 10 CS and seeded across all layers. Cells are then incubated at $37 \pm 2.0°C$. with $5.0 \pm 1.0\%$ $CO_2$ and fed with fresh Complete Growth Media every five to seven days. Cells should not remain in the 10CS for more than 20 days prior to passaging. In one aspect, the passaged dermal fibroblasts are rendered substantially free of immunogenic proteins present in the culture medium by incubating the expanded fibroblasts for a period of time in protein free medium, Primary Harvest When cell confluence in the 10 CS is 95% or more, cells are harvested. Harvesting is performed by removing the spent media, washing the cells, treating with Trypsin-EDTA to release adherent cells into the solution, and adding additional Complete Growth Media to neutralize the trypsin. Cells are collected by centrifugation, resuspended, and in-process QC testing performed to determine total viable cell count and cell viability.

In some aspects, fibroblasts are incubated with one or more growth factors (i.e. mitogenic compounds) under suitable growth conditions to allow for proliferation, and to prepare for differentiation into neuronal cells. Likewise, the fibroblasts of the present disclosure may be incubated with one or more of various differentiation inducers (i.e. inducers or inducing agents), and optionally one or more growth factors, under suitable conditions to allow for differentiation, and optionally propagation, of a variety of cell types. As one of ordinary skill would recognize, there are known compounds that function as both growth factors and differentiation inducers. Growth factors of the disclosure include but are not limited to M-CSF, IL-6, LIF, and IL-12. Examples of compounds functioning as growth factors and/or differentiation inducers include, but are not limited to, lipopolysaccharide (LPS), phorbol 12-myristate 13-acetate (PMA), stem cell growth factor, human recombinant interleukin-2 (IL-2), IL-3, epidermal growth factor (EGF), b-nerve growth factor (bNGF), recombinant human vascular endothelial growth factor 165 isoform (VEGF165), and hepatocyte growth factor (HGF). Useful doses for inducing proliferation of fibroblasts and increasing susceptibility to differentiation by growth and/or differentiation factors are: 0.5 ng/mL-1.0 μg/mL (such as 1.0 μg/mL) for LPS, 1-160 nM (such as 3 nM) for PMA, 500-2400 units/mL (such as 1200 ng/mL) for bNGF, 12.5-100 ng/mL (such as 50 ng/mL for VEGF), 10-200 ng/mL (such as 100 ng/mL) for EGF, and 25-200 ng/mL (such as 50 ng/mL) for HGF.

Induction of differentiation to neurons may be performed using several means. One novel finding is that co-culture with conditioned media from neurons cells is sufficient to induce some degree of differentiation. In some aspects, utilization of dedifferentiating agents, such as valproic acid, enhances differentiation to neurons from fibroblasts in the presence of neuron conditioned media. When neurogenic differentiation is desired, various protocols may be used, in some aspects, protocols useful for differentiating fibroblasts into neurons may be adapted, modified, or replicated using fibroblasts as starting populations. Such protocols are known in the art [72-127].

In some aspects, prior to administration, fibroblasts are contacted with a dedifferentiating agent, such as valproic acid, and/or other agents such as lithium, and/or 5-azacytidine in order to induce expression of one or more markers including, for example, OCT-4, alkaline phosphatase, Sox2, TDGF-1, SSEA-3, SSEA-4, TRA-1-60, and/or TRA-1-80 [128, 129]. Said dedifferentiated cells may be cultured in a multilayer population or embryoid body for a time sufficient for neuron cells to appear in said culture. Said time sufficient for neuronal cells to appear in said culture may comprise at least about 1 week, at least about 2 weeks, at least about 3 weeks, at least about 4 weeks, at least about 5 weeks, at least about 6 weeks, at least about 7 weeks, or at least about 8 weeks. Said multilayer population or embryoid body may be cultured in a medium, including, for example, DMEM. Said medium may comprise, consists essentially of, or consists of EB-DM. Said neuronal cells may be isolated and cultured, thereby producing a population of neurons useful for transplantation. Said isolating may comprise dissociating cells or clumps of cells from the culture enzymatically, chemically, or physically and selecting neuronal cells cells or clumps of cells may comprise neuronal cells. Said embryoid body may be cultured in suspension and/or as an adherent culture (e.g., in suspension followed by adherent culture). Said embryoid body cultured as an adherent culture may produce one or more outgrowths comprising neuronal cells. Said pluripotent stem cells have reduced HLA antigen complexity. Prior to neuronal formation said dedifferentiated fibroblasts cells may be cultured on a matrix which may be selected from the group consisting of laminin, fibronectin, vitronectin, proteoglycan, entactin, collagen, collagen I, collagen IV, collagen VIII, heparan sulfate, Matrigel™ (a soluble preparation from Engelbreth-Holm-Swarm (EHS) mouse sarcoma cells), CellStart, a human basement membrane extract, and any combination thereof. Said matrix may comprise Matrigel™ (a soluble preparation from Engelbreth-Holm-Swarm (EHS) mouse sarcoma cells).

Certain aspects of the present disclosure concern the administration of one or more cellular therapies, such as at least one fibroblasts therapy and/or modified fibroblast therapy, and/or derivatives of fibroblasts (such as exosomes, microvesicles, or apoptotic bodies). A cellular therapy, such as a fibroblast cell therapy may comprise a therapeutically effective amount of cells, such as fibroblasts. The cellular therapy may comprise one or more other suitable compositions that allow for the generation, storage, and/or administration of the cellular therapy. Fibroblasts may be administered to the individual systemically and/or locally. Fibroblasts may be administered to the individual intravenously, intradiscally, epidurally, intrarectally, intra-omentally, intra-arterially, or a combination thereof. Fibroblasts may be administered prior to or simultaneously with the compositions and/or cellular therapies encompassed herein.

There are several methods known in the art for the generation of fibroblasts or obtaining them. In some aspects, the fibroblasts are from omentum, bone marrow, placenta, peripheral blood, cord blood, Wharton's jelly, cerebral spinal fluid, cancer-associated, foreskin, skin, a combination thereof, or any other tissue sufficiently abundant in fibroblasts. The fibroblasts of the present disclosure may comprise fibroblasts selected from the group consisting of skin fibroblasts, hair follicle fibroblasts, adipose fibroblasts, bone marrow fibroblasts, umbilical cord blood fibroblasts, placental fibroblasts, omentum fibroblasts, ovarian tube fibroblasts, peripheral blood fibroblasts, and a combination thereof. In some aspects, fibroblasts of the present disclosure are isolated from tissue comprising fibroblasts selected from the group consisting of skin fibroblasts, hair follicle fibroblasts, adipose fibroblasts, bone marrow fibroblasts, umbilical cord blood fibroblasts, placental fibroblasts, omentum fibroblasts, ovarian tube fibroblasts, peripheral blood fibroblasts, and a combination thereof.

In some aspects, fibroblasts are generated according to protocols previously utilized for treatment of patients utilizing bone marrow-derived MSCs. Specifically, bone marrow is aspirated (10-30 mL) under local anesthesia (with or without sedation) from the posterior iliac crest, collected into sodium heparin containing tubes and transferred to a Good Manufacturing Practices (GMP) clean room. In some aspects, other tissues besides bone marrow are utilized as the starting tissue. For example, in one aspect, dermal fibroblasts are utilized.

Bone marrow cells are washed with a washing solution such as Dulbecco's phosphate-buffered saline (DPBS), RPMI, or PBS supplemented with autologous patient plasma and layered on to 25 mL of Percoll (1.073 g/mL) at a concentration of approximately $1\text{-}2 \times 10^7$ cells/mL. Subsequently the cells are centrifuged at approximately $900 \times g$ for approximately 30 min or a time period sufficient to achieve separation of mononuclear cells from debris and erythrocytes. The cells are then washed with PBS and plated at a density of approximately $1 \times 10^6$ cells per ml in 175 cm$^2$ tissue culture flasks in DMEM with 10% FCS with flasks subsequently being loaded with a minimum of 30 million bone marrow mononuclear cells. The fibroblasts are allowed to adhere for 72 h followed by media changes every 3-4 days. Adherent cells are removed with 0.05% trypsin-EDTA and replated at a density of approximately $1 \times 10^6$ per 175 cm$^2$.

Fibroblasts may be cultured in the presence of a liquid culture medium. Typically, the medium may comprise a basal medium formulation as known in the art. Many basal media formulations can be used to culture fibroblasts herein, including but not limited to Eagle's Minimum Essential Medium (MEM), Dulbecco's Modified Eagle's Medium (DMEM), alpha modified Minimum Essential Medium (alpha-MEM), Basal Medium Essential (BME), Iscove's Modified Dulbecco's Medium (IMDM), BGJb medium, F-12 Nutrient Mixture (Ham), Liebovitz L-15, DMEM/F-12, Essential Modified Eagle's Medium (EMEM), RPMI-1640, and modifications and/or combinations thereof. Compositions of the above basal media are generally known in the art, and it is within the skill of one in the art to modify or modulate concentrations of media and/or media supplements as necessary for the fibroblasts cultured. In some aspects, a culture medium formulation may be explants medium (CEM) which is composed of IMDM supplemented with 10% fetal bovine serum (FBS, Lonza), 100 U/mL penicillin G, 100 μg/mL streptomycin and 2 mmol/L L-glutamine (Sigma-Aldrich). Other aspects may employ further basal media formulations, such as chosen from the ones above.

It is known that under certain conditions fibroblasts are capable of producing interleukin-1 and/or other inflammatory cytokines [130]. The disclosure concerns methods to manipulate fibroblasts to reduce and/or inhibit the production and/or secretion of inflammatory cytokines, such as by gene editing and/or exposure to one or more compositions that aid in the reduction or inhibition of the production and/or secretion of inflammatory cytokines. In certain aspects, fibroblasts are manipulated by gene editing of IL-1 and/or other inflammatory mediators. The inflammatory mediators may be any composition including, for example, interleukin-6, interleukin-7, interleukin-9, interleukin-11, interleukin-12, interleukin-8, interleukin-15, interleukin-17, interleukin-18, interleukin-21, interleukin-23, interleukin-27, interleukin-33, interferon-gamma, TNF-alpha, HMGB-1, or a combination thereof.

Gene editing may comprise genomic manipulation, such as at least one genetic knockout by any method (including CRISPR systems, TALEN systems, recombination systems, or a combination thereof) and/or at least one transgenic insertion. Gene editing may comprise RNA interference, such as RNA interference that targets inflammatory cytokines encompassed herein. In some aspects, gene editing comprises the use of a viral construct. In some aspects, gene editing comprises the use of a non-viral construct.

In some aspects, fibroblasts are pretreated with TNF-alpha in a manner to induce expression of growth factors and/or proliferation such as described in this publication [131, 132]. In some aspects, fibroblasts comprising at least one fibroblast cell therapy are exposed to TNF-alpha prior to administering the fibroblasts. In some aspects, exposure to TNF-alpha prevents and/or reduces the expression and/or secretion of inflammatory cytokines including interleukin-1, interleukin-6, interleukin-7, interleukin-9, interleukin-11, interleukin-12, interleukin-8, interleukin-15, interleukin-17, interleukin-18, interleukin-21, interleukin-23, interleukin-27, interleukin-33, interferon-gamma, TNF-alpha, HMGB-1 and a combination thereof.

In some aspects, fibroblasts comprising at least one fibroblast cell therapy are exposed to at least one inhibitor of protein kinase C, cyclic nucleotide-dependent protein kinases, calmodulin-dependent protein kinases, the Na(+)-H+ antiport system, or a combination thereof prior to administering the fibroblasts. In some aspects, exposure to at least one inhibitor of protein kinase C, cyclic nucleotide-dependent protein kinases, calmodulin-dependent protein kinases, the Na(+)-H+ antiport system, or a combination thereof prevents and/or reduces the expression and/or secretion of inflammatory cytokines including interleukin-1, interleukin-6, interleukin-7, interleukin-9, interleukin-11, interleukin-12, interleukin-8, interleukin-15, interleukin-17, interleukin-18, interleukin-21, interleukin-23, interleukin-27, interleukin-33, interferon-gamma, TNF-alpha, HMGB-1 or a combination thereof.

Methods of the present disclosure may include the incubation with added factors that further enhance generation or function of the T regs. Suitable factors include, without limitation, hormones, proteins, drugs, and/or antibodies. The factors may include, without limitation, at least one of TGF-β, α-MSH, anti-CD46, IL-10, vitamin D3, dexamethasone, rapamycin, and/or IL-2. The factor may be IL-10. The IL-10 may be present at a concentration of about 1 ng/mL to about 100 ng/mL. The IL-10 may be present at a concentration of about 20 ng/mL.

In some aspects, extracorporeal photophoresis used to induce apoptosis. This may involve a photoactivatable composition added to a cell population *ex vivo.* The photosensitive composition may be administered to a cell population comprising blood cells following withdrawal from an individual, subject, recipient, and/or donor, as the case may be, and prior to or contemporaneously with exposure to ultraviolet light. The photosensitive composition may be administered to a cell population comprising whole blood or a fraction thereof, provided that the target blood cells or blood components receive the photosensitive composition. In one aspect, fibroblasts allogenic to an individual receiving a cellular therapy are mixed with autologous blood from the individual and the combination of allogenic fibroblasts and autologous blood is subjected to photopheresis. In another aspect, a portion of the individual's blood, recipient's blood, or donor's blood could first be processed using known methods to substantially remove erythrocytes; the photoactive composition may then be administered to the resulting cell population comprising the enriched PBMC fraction.

## IV. Fibroblasts and Cultured Cells

Aspects of the present disclosure comprise cells useful in therapeutic methods and compositions. Cells disclosed herein include, for example, fibroblasts, stem cells (e.g., hematopoietic stem cells or mesenchymal stem cells), and endothelial progenitor cells. Cells of a given type (e.g., fibroblasts) may be used alone or in combination with cells of other types. For example, fibroblasts may be isolated and provided to a subject alone or in combination with one or more stem cells. In one example, fibroblasts are isolated and provided to a subject together with one or more endothelial progenitor cells. In some aspects, disclosed herein are fibroblasts capable of stimulating tissue regeneration, immune modulation, and/or angiogenesis.

Compositions of the present disclosure may be obtained from isolated fibroblast cells or a population thereof capable of proliferating and differentiating into ectoderm, mesoderm, or endoderm. In some aspects, an isolated fibroblast cell expresses at least one of Oct-4, Nanog, Sox-2, KLF4, c-Myc, Rex-1, GDF-3, LIF receptor, CD105, CD117, CD344 or Stella markers. In some aspects, an isolated fibroblast cell does or does not express at least one of MHC class I, MHC class II, CD45, CD13, CD49c, CD66b, CD73, CD105, or CD90 cell surface proteins. Such isolated fibroblast cells may be used as a source of conditioned media. The cells may be cultured alone, or may by cultured in the presence of other cells in order to further upregulate production of growth factors in the conditioned media.

Fibroblasts may be expanded and utilized by administration themselves, or may be cultured in a growth media in order to obtain conditioned media. The term Growth Medium generally refers to a medium sufficient for the culturing of fibroblasts. In particular, a possible medium for the culturing of the cells of the disclosure herein comprises Dulbecco's Modified Essential Media (DMEM). Another possible medium is DMEM-low glucose (also DMEM-LG herein) (Invitrogen®, Carlsbad, Calif.). The DMEM-low glucose may be supplemented with 15% (v/v) fetal bovine serum (e.g. defined fetal bovine serum, Hyclone™, Logan Utah), antibiotics/antimycotics (such as penicillin including at 100 Units/milliliter), streptomycin (100 milligrams/milliliter), and amphotericin B (0.25 micrograms/milliliter), (Invitrogen®, Carlsbad, Calif.)), and 0.001% (v/v) 2-mercaptoethanol (Sigma®, St. Louis Mo.). In some cases, different growth media are used, or different supplementations are provided, and these are normally indicated as supplementations to Growth Medium. Also relating to the present disclosure, the term standard growth conditions, as used herein refers to culturing of cells at 37°C, in a standard atmosphere comprising 5% $CO_2$, where relative humidity is maintained at about 100%. While the foregoing conditions are useful for culturing, it is to be understood that such conditions are capable of being varied by the skilled artisan who will appreciate the options available in the art for culturing cells, for example, varying the temperature, $CO_2$, relative humidity, oxygen, growth medium, and the like.

Also disclosed herein are cultured cells. Various terms are used to describe cells in culture. Cell culture refers generally to cells taken from a living organism and grown under controlled condition ("in culture" or "cultured"). A primary cell culture is a culture of cells, tissues, or organs taken directly from an organism(s) before the first subculture. Cells are expanded in culture when they are placed in a growth medium under conditions that facilitate cell growth and/or division, resulting in a larger population of the cells. When cells are expanded in culture, the rate of cell proliferation is sometimes measured by the amount of time needed for the cells to double in number, or the "doubling time".

Fibroblast cells used in the disclosed methods can undergo at least 25, 30, 35, or 40 doublings prior to reaching a senescent state. Methods for deriving cells capable of doubling to reach $10^{14}$ cells or more are provided. Examples are those methods which derive cells that can double sufficiently to produce at least about $10^{14}$, $10^{15}$, $10^{16}$, or $10^{17}$ or more cells when seeded at from about $10^3$ to about $10^6$ cells/cm$^2$ in culture. These cell numbers may be produced within 80, 70, or 60 days or less. In one aspect, fibroblast cells used are isolated and expanded, and possess one or more markers selected from a group consisting of CD10, CD13, CD44, CD73, CD90, CD141, PDGFr-alpha, HLA-A, HLA-B, and HLA-C. In some aspects, the fibroblast cells do not produce one or more of CD31, CD34, CD45, CD117, CD141, HLA-DR, HLA-DP, or HLA-DQ.

When referring to cultured cells, including fibroblast cells and vertebrae cells, the term senescence (also "replicative senescence" or "cellular senescence") refers to a property attributable to finite cell cultures; namely, their inability to grow beyond a finite number of population doublings (sometimes referred to as Hayflick's limit). Although cellular senescence was first described using fibroblast-like cells, most normal human cell types that can be grown successfully in culture undergo cellular senescence. The *in vitro* lifespan of different cell types varies, but the maximum lifespan is typically fewer than 100 population doublings (this is the number of doublings for all the cells in the culture to become senescent and thus render the culture unable to divide). Senescence does not depend on chronological time, but rather is measured by the number of cell divisions, or population doublings, the culture has undergone. Thus, cells made quiescent by removing essential growth factors are able to resume growth and division when the growth factors are re-introduced, and thereafter carry out the same number of doublings as equivalent cells grown continuously. Similarly, when cells are frozen in liquid nitrogen after various numbers of population doublings and then thawed and cultured, they undergo substantially the same number of doublings as cells maintained unfrozen in culture. Senescent cells are not dead or dying cells; they are resistant to programmed cell death (apoptosis) and can be maintained in their nondividing state for as long as three years. These cells are alive and metabolically active, but they do not divide.

In some cases, fibroblast cells are obtained from a biopsy, and the donor providing the biopsy may be either the individual to be treated (autologous), or the donor may be different from the individual to be treated (allogeneic). In cases wherein allogeneic fibroblast cells are utilized for an individual, the fibroblast cells may come from one or a plurality of donors.

The fibroblasts may be obtained from a source selected from the group consisting of: dermal fibroblasts; placental fibroblasts; adipose fibroblasts; bone marrow fibroblasts; foreskin fibroblasts; umbilical cord fibroblasts; hair follicle derived fibroblasts; nail derived fibroblasts; endometrial derived fibroblasts; keloid derived fibroblasts; and a combination thereof. In some aspects, fibroblasts are dermal fibroblasts.

In some aspects, fibroblasts are manipulated or stimulated to produce one or more factors. In some aspects, fibroblasts are manipulated or stimulated to produce leukemia inhibitory factor (LIF), brain-derived neurotrophic factor (BDNF), epidermal growth factor receptor (EGF), basic fibroblast growth factor (bFGF), FGF-6, glial-derived neurotrophic factor (GDNF), granulocyte colony-stimulating factor (GCSF), hepatocyte growth factor (HGF), IFN-γ, insulin-like growth factor binding protein (IGFBP-2), IGFBP-6, IL-1ra, IL-6, IL-8, monocyte chemotactic protein (MCP-1), mononuclear phagocyte colony-stimulating factor (M-CSF), neurotrophic factors (NT3), tissue inhibitor of metalloproteinases (TIMP-1), TIMP-2, tumor necrosis factor (TNF-β), vascular endothelial growth factor (VEGF), VEGF-D, urokinase plasminogen activator receptor (uPAR), bone morphogenetic protein 4 (BMP4), IL1-a, IL-3, leptin, stem cell factor (SCF), stromal cell-derived factor-1 (SDF-1), platelet derived growth factor-BB (PDGFBB), transforming growth factors beta (TGFβ-1) and/or TGFβ-3. Factors from manipulated or stimulated fibroblasts may be present in conditioned media and collected for therapeutic use.

In some aspects, fibroblasts are transfected with one or more angiogenic genes to enhance ability to promote neural repair. An "angiogenic gene" describes a gene encoding for a protein or polypeptide capable of stimulating or enhancing angiogenesis in a culture system, tissue, or organism. Examples of angiogenic genes which may be useful in transfection of fibroblasts include activin A, adrenomedullin, aFGF, ALK1, ALK5, ANF, angiogenin, angiopoietin-1, angiopoietin-2, angiopoietin-3, angiopoietin-4, bFGF, B61, bFGF inducing activity, cadherins, CAM-RF, cGMP analogs, ChDI, CLAF, claudins, collagen, connexins, Cox-2, ECDGF (endothelial cell-derived growth factor), ECG, ECI, EDM, EGF, EMAP, endoglin, endothelins, endostatin, endothelial cell growth inhibitor, endothelial cell-viability maintaining factor, endothelial differentiation shpingolipid G-protein coupled receptor-1 (EDG1), ephrins, Epo, HGF, TGF-beta, PD-ECGF, PDGF, IGF, IL8, growth hormone, fibrin fragment E, FGF-5, fibronectin, fibronectin receptor, Factor X, HB-EGF, HBNF, HGF, HUAF, heart derived inhibitor of vascular cell proliferation, IL1, IGF-2 IFN-gamma, α1β1 integrin, α2β1 integrin, K-FGF, LIF, leiomyoma-derived growth factor, MCP-1, macrophage-derived growth factor, monocyte-derived growth factor, MD-ECI, MECIF, MMP2, MMP3, MMP9, urokiase plasminogen activator, neuropilin, neurothelin, nitric oxide donors, nitric oxide synthases (NOSs), notch, occludins, zona occludins, oncostatin M, PDGF, PDGF-B, PDGF receptors, PDGFR-β, PD-ECGF, PAI-2, PD-ECGF, PF4, P1GF, PKR1, PKR2, PPAR-gamma, PPAR-gamma ligands, phosphodiesterase, prolactin, prostacyclin, protein S, smooth muscle cell-derived growth factor, smooth muscle cell-derived migration factor, sphingosine-1-phosphate-1 (SIP1), Syk, SLP76, tachykinins, TGF-beta, Tie 1, Tie2, TGF-β, TGF-β receptors, TIMPs, TNF-α, transferrin, thrombospondin, urokinase, VEGF-A, VEGF-B, VEGF-C, VEGF-D, VEGF-E, VEGF, VEGF(164), VEGI, and EG-VEGF. Fibroblasts transfected with one or more angiogenic factors may be used in the disclosed methods of treatment or prevention of stroke.

Under appropriate conditions, fibroblasts may be capable of producing interleukin-1 (IL-1) and/or other inflammatory cytokines. In some aspects, fibroblasts of the present disclosure are modified (e.g., by gene editing) to prevent or reduce expression of IL-1 or other inflammatory cytokines. For example, in some aspects, fibroblasts are fibroblasts having a deleted or non-functional IL-1 gene, such that the fibroblasts are unable to express IL-1. Such modified fibroblasts may be useful in the therapeutic methods of the present disclosure by having limited pro-inflammatory capabilities when provided to a subject. In some aspects, fibroblasts are treated with (e.g., cultured with) TNF-α, thereby inducing expression of growth factors and/or fibroblast proliferation.

In some aspects, fibroblasts and/or other cells of the present disclosure (e.g., endothelial progenitor cells, mesenchymal stem cells, hematopoietic stem cells, etc.) are treated with (e.g., cultured with) metformin prior to use or administration to a subject. Treatment with metformin may enhance therapeutic efficacy. In some aspects, fibroblasts are preconditioned with metformin to enhance their angiogenic potential prior to therapeutic use. Angiogenic potential may be assessed by measurement of the production of cytokines such as VEGF, EGF, IGF, FGF, and PGF. Angiogenic potential may be quantified directly by injection of cells into animals suffering from ischemic insult. Other assessments of therapeutic benefit of metformin preconditioning includes evaluating cells for: a) resistance to apoptosis; b) production of neurotrophic factors; c) induction of antioxidant enzymes such as superoxide dismutase; and/or d) homing activity, either assessed directly by chemotaxis assays towards a chemoattractant such as SDF-1, or expression of the chemokine receptor CXCR4.

V. Exosomes

Exosomes may be derived from large multivesicular endosomes and secreted into the extracellular milieu. Exosomes may comprise vesicles or a flattened sphere limited by a lipid bilayer. The exosomes may comprise diameters of 20-100 nm. The exosomes may be formed by inward budding of the endosomal membrane. The exosomes may have a density of about 1.13-1.19 g/mL and may float on sucrose gradients. The exosomes may be enriched in cholesterol and sphingomyelin, and lipid raft markers such as GM1, GM3, flotillin and the src protein kinase Lyn. The exosomes may comprise one or more proteins present in fibroblast, such as a protein characteristic or specific to the fibroblasts or fibroblast conditioned media. They may comprise RNA, for example miRNA. The exosomes may possess one or more genes or gene products found in fibroblasts or medium which is conditioned by culture of fibroblasts. The exosomes may comprise molecules secreted by the fibroblasts. Such a exosome, and combinations of any of the molecules comprised therein, including in particular proteins or polypeptides, may be used to supplement the activity of, or in place of, the fibroblasts or medium conditioned by the fibroblasts for the purpose of for example treating or preventing a disease (e.g., stroke). The exosome may comprise a cytosolic protein found in cytoskeleton *e.g.,* tubulin, actin and actin-binding proteins, intracellular membrane fusions and transport, *e.g.,* annexins and rab proteins, signal transduction proteins, *e.g.,* protein kinases, 14-3-3 and heterotrimeric G proteins, metabolic enzymes, *e.g.,* peroxidases, pyruvate and lipid kinases, and enolase-1 and the family of tetraspanins, *e.g.,* CD9, CD63, CD81 and CD82. In particular, the exosome may comprise one or more tetraspanins. The exosomes may comprise mRNA and/or microRNA. The exosome may be used for any of the therapeutic purposes that the fibroblasts or fibroblast conditioned media are used herein.

In one aspect, fibroblast exosomes, or exosomes may be produced by culturing fibroblasts in a medium to condition them. The fibroblasts may be derived from human umbilical tissue derived cells, or other tissues possessing or associated with regenerative features, which possess markers selected from a group comprising of CD90, CD73 and CD105. The medium may comprise DMEM. The DMEM may be such that it does not comprise phenol red. The medium may be supplemented with insulin, transferrin, or selenoprotein (ITS), or any combination thereof. It may comprise FGF2. It may comprise PDGF AB. The concentration of FGF2 may be about 5 ng/mL FGF2. The concentration of PDGF AB may be about 5 ng/mL. The medium may comprise glutamine-penicillin-streptomycin or β-mercaptoethanol, or any combination thereof. The cells may be cultured for about 1, 2, 3, 4, 5, 6, 7, 8, 9, 10 days or more, for example 3 days. The conditioned medium may be obtained by separating the cells from the medium. The conditioned medium may be centrifuged, for example at 500×g. it may be concentrated by filtration through a membrane. The membrane may comprise a >1000 kDa membrame. The conditioned medium may be concentrated about 50 times or more. The conditioned medium may be subject to liquid chromatography such as HPLC. The conditioned medium may be separated by size exclusion. Any size exclusion matrix such as Sepharose may be used. As an example, a TSK Guard column SWXL, 6x40 mm or a TSK gel G4000 SWXL, 7.8x300 mm may be employed. The eluent buffer may comprise any physiological medium such as saline. It may comprise 20 mM phosphate buffer with 150 mM of NaCl at pH 7.2. The chromatography system may be equilibrated at a flow rate of 0.5 mL/min.

The elution mode may be isocratic. UV absorbance at 220 nm may be used to track the progress of elution. Fractions may be examined for dynamic light scattering (DLS) using a quasi-elastic light scattering (QELS) detector. Fractions which are found to exhibit dynamic light scattering may be retained. For example, a fraction which is produced by the general method as described above, and which elutes with a retention time of 11-13 minutes, such as 12 minutes, is found to exhibit dynamic light scattering. The $r_h$ of exosomes in this peak is about 45-55 nm.

Exosomes may be purified using a variety of means known in the art. The fibroblast cell-derived exosomes of the present disclosure can be recovered by carrying out expansion culturing of fibroblast cells in conditioned medium to subconfluency (or confluency), replacing the medium with fresh conditioned medium, culturing for normally 1 to 5 days (e.g., 1 day, 2 to 3 days, or 3 to 4 days) and recovering the exosomes from the culture supernatant. Examples of methods used to recover the fibroblast cell-derived exosomes of the present disclosure include ultracentrifugation, density gradient centrifugation and the use of various types of exosome separation kits (such as the formation of a pellet by centrifugation, immunoprecipitation, purification with magnetic beads, fractionation according to particle size or column adsorption). In some aspects, methods used to recover fibroblast cell-derived exosomes of the present disclosure includes subjecting a culture supernatant of fibroblast cells to ultracentrifugation for 0.5 hours to 2 hours at about 50,000×g to 150,000×g. The method can also include centrifuging the culture supernatant of the fibroblast cells to 0.1 hours to 2 hours at about 100×g to 20,000×g prior to carrying out ultracentrifugation. The fibroblast cell-derived exosomes of the present disclosure can be stored for about 1 week at 4°C, for about 1 month at -20°C or for about 6 months at -80°C, provided it is stored dissolved in a solution such as PBS, and can be stored for about 3 years at 4°C provided it has been freeze-dried. In some aspects, exosomes are purified using ultracentrifugation. In some aspects, exosomes are purified using chromatography. In some aspects, exosomes are purified using affinity purification.

Exosomes of the present disclosure may be derived from one or more cellular sources. In some aspects, exosomes are derived from fibroblasts. In some aspects, exosomes are derived from regenerative fibroblasts. In some aspects, exosomes are derived from stem cells (e.g., hematopoietic stem cells, mesenchymal stem cells, etc.). In some aspects, exosomes are derived by isolating conditioned media from a cell culture. Exosomes, provided alone or as a component of conditioned media, may be used in any of the disclosed therapeutic methods or compositions.

The disclosed methods may comprise administration of exosomes to a subject. Compositions comprising exosomes may comprise about, at least, or at most 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9, 5.0, 5.1, 5.2, 5.3, 5.4, 5.5, 5.6, 5.7, 5.8, 5.9, 6.0, 6.1, 6.2, 6.3, 6.4, 6.5, 6.6, 6.7, 6.8, 6.9, 7.0, 7.1, 7.2, 7.3, 7.4, 7.5, 7.6, 7.7, 7.8, 7.9, 8.0, 8.1, 8.2, 8.3, 8.4, 8.5, 8.6, 8.7, 8.8, 8.9, 9.0, 9.1, 9.2, 9.3, 9.4, 9.5, 9.6, 9.7, 9.8, 9.9, 10.0, 10.5, 11.0, 11.5, 12.0, 12.5, 13.0, 13.5, 14.0, 14.5, 15.0, 15.5, 16.0, 16.5, 17.0, 17.5, 18.0, 18.5, 19.0. 19.5, 20.0, 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 105, 110, 115, 120, 125, 130, 135, 140, 145, 150, 155, 160, 165, 170, 175, 180, 185, 190, 195, 200, 205, 210, 215, 220, 225, 230, 235, 240, 245, 250, 255, 260, 265, 270, 275, 280, 285, 290, 295, 300, 305, 310, 315, 320, 325, 330, 335, 340, 345, 350, 355, 360, 365, 370, 375, 380, 385, 390, 395, 400, 410, 420, 425, 430, 440, 445, 450, 460, 470, 475, 480, 490, 500, 510, 520, 525, 530, 540, 550, 560, 570, 575, 580, 590, 600, 610, 620, 625, 630, 640, 650, 660, 670, 675, 680, 690, 700, 710, 720, 725, 730, 740, 750, 760, 770, 775, 780, 790, 800, 810, 820, 825, 830, 840, 850, 860, 870, 875, 880, 890, 900, 910, 920, 925, 930, 940, 950, 960, 970, 975, 980, 990, 1000, 1100, 1200, 1300, 1400, 1500, 1600, 1700, 1800, 1900, 2000, 2100, 2200, 2300, 2400, 2500, 2600, 2700, 2800, 2900, 3000, 3100, 3200, 3300, 3400, 3500, 3600, 3700, 3800, 3900, 4000, 4100, 4200, 4300, 4400, 4500, 4600, 4700, 4800, 4900, 5000, 6000, 7000, 8000, 9000, 10000 nanograms (ng), micrograms ($\mu$g), milligrams (mg), or grams of exosomes, or any range derivable therein. Exosomes may be administered at a dose based on a subject's weight, expressed as ng/kg, mg/kg, or g/kg.

In some aspects, exosomes for use in the current disclosure are purified by culturing fibroblasts using means known in the art for preserving viability and proliferative ability of fibroblasts. The disclosure may be applied both for individualized autologous exosome preparations and for exosome preparations obtained from established cell lines, for experimental or biological use. In particular aspects, the use of chromatography separation methods are employed for preparing membrane vesicles, particularly to separate the membrane vesicles from potential biological contaminants, wherein said microvesicles are exosomes, and cells utilized for generating said exosomes are fibroblast cells.

In one aspect, a strong or weak anion exchange may be performed. In addition, in a specific aspect, the chromatography is performed under pressure. Thus, it may consist of high performance liquid chromatography (HPLC). Different types of supports may be used to perform the anion exchange chromatography. These may include cellulose, poly(styrene-divinylbenzene), agarose, dextran, acrylamide, silica, ethylene glycol-methacrylate co-polymer, or mixtures thereof, e.g., agarose-dextran mixtures. To illustrate this, it is possible to mention the different chromatography equipment composed of supports as mentioned above, particularly the following gels: SOURCE. POROS®. SEPHAROSE®, SEPHADEX®, TRISACRYL®, TSK-GEL SW OR PW®, SUPERDEX®TOYOPEARL HW and SEPHACRYL®, for example, which are suitable for the application of this disclosure. Therefore, in a specific aspect, this disclosure relates to a method of preparing membrane vesicles, particularly exosomes, from a biological sample such as a tissue culture containing fibroblasts, comprising at least one step during which the biological sample is treated by anion exchange chromatography on a support selected from cellulose, poly(styrene-divinylbenzene), silica, acrylamide, agarose, dextran, ethylene glycol-methacrylate co-polymer, alone or in mixtures, optionally functionalized.

In addition, to improve the chromatographic resolution, within the scope of the disclosure, supports in bead form may be used. These beads have a homogeneous and calibrated diameter, with a sufficiently high porosity to enable the penetration of the objects under chromatography (i.e. the exosomes). In this way, given the diameter of exosomes (generally between 50 and 100 nm), to apply the disclosure, high porosity gels may be used, particularly between 10 nm and 5 $\mu$m, including between approximately 20 nm and approximately 2 $\mu$m, including between about 100 nm and about 1 $\mu$m. For the anion exchange chromatography, the support used must be functionalized using a group capable of interacting with an anionic molecule. Generally, this group is composed of an amine which may be ternary or quaternary, which defines a weak or strong anion exchanger, respectively. Within the scope of this disclosure, it is particularly advantageous to use a strong anion exchanger. In this way, according to the disclosure, a chromatography support as described above, functionalized with quaternary amines, is used. Therefore, according to a specific aspect of the disclosure, the anion exchange chromatography is performed on a support functionalized with a quaternary amine. This support may be selected from poly(styrene-divinylbenzene), acrylamide, agarose, dextran and silica, alone or in mixtures, and functionalized with a quaternary amine. Examples of supports functionalized with a quaternary amine include the gels SOURCEQ. MONO Q, Q SEPHAROSE®, POROS® HQ and POROS® QE, FRACTOGEL®TMAE type gels and TOYOPEARL SUPER®Q gels.

In some aspects, a support to perform the anion exchange chromatography comprises poly(styrene-divinylbenzene). An example of this type of gel which may be used within the scope of this disclosure is SOURCE Q gel, particularly SOURCE 15 Q (Pharmacia). This support offers the advantage of very large internal pores, thus offering low resistance to the circulation of liquid through the gel, while enabling rapid diffusion of the exosomes to the functional groups, which are particularly important parameters for exosomes given their size. The biological compounds retained on the column may be eluted in different ways, particularly using the passage of a saline solution gradient of increasing concentration, e.g. from 0 to 2 M. A sodium chloride solution may particularly be used, in concentrations varying from 0 to 2 M, for example. The different fractions purified in this way are detected by measuring their optical density (OD) at the column outlet using a continuous spectro-photometric reading. As an indication, under the conditions used in the examples, the fractions comprising the membrane vesicles were eluted at an ionic strength comprised between approximately 350 and 700 mM, depending on the type of vesicles.

Different types of columns may be used to perform this chromatographic step, according to requirements and the volumes to be treated. For example, depending on the preparations, it is possible to use a column from approximately 100 $\mu$L up to 10 mL or greater. In this way, the supports available have a capacity which may reach 25 mg of proteins/ml, for example. For this reason, a 100 $\mu$L column has a capacity of approximately 2.5 mg of proteins which, given the samples in question, allows the treatment of culture supernatants of approximately 2 L (which, after concentration by a factor of 10 to 20, for example, represent volumes of 100 to 200 mL per preparation). It is understood that higher volumes may also be treated, by increasing the volume of the column, for example. In addition, to perform this disclosure, it is also possible to combine the anion exchange chromatography step with a gel permeation chromatography step. In this way, according to a specific aspect of the disclosure, a gel permeation chromatography step is added to the anion exchange step, either before or after the anion exchange chromatography step. In a particular aspect, the permeation chromatography step takes place after the anion exchange step. In addition, in a specific variant, the anion exchange chromatography step is replaced by the gel permeation chromatography step. The present application demonstrates that membrane vesicles may also be purified using gel permeation liquid chromatography, particularly when this step is combined with an anion exchange chromatography or other treatment steps of the biological sample, as described in detail below.

To perform the gel permeation chromatography step, a support selected from silica, acrylamide, agarose, dextran, ethylene glycol-methacrylate co-polymer or mixtures thereof, e.g., agarose-dextran mixtures, may be used. As an illustration, for gel permeation chromatography, a support such as SUPERDEX®200HR (Pharmacia), TSK G6000 (TosoHaas) or SEPHACRYL® S (Pharmacia) may be used. The process according to the disclosure may be applied to different biological samples. In particular, these may consist of a biological fluid from a subject (bone marrow, peripheral blood, etc.), a culture supernatant, a cell lysate, a pre-purified solution or any other composition comprising membrane vesicles.

In this respect, in a specific aspect of the disclosure, the biological sample is a culture supernatant of membrane vesicle-producing fibroblast cells.

In addition, according to an aspect of the disclosure, the biological sample is treated, prior to the chromatography step, to be enriched with membrane vesicles (enrichment stage). In this way, in a specific aspect, this disclosure relates to a method of preparing membrane vesicles from a biological sample, characterized in that it comprises at least an enrichment step, to prepare a sample enriched with membrane vesicles, and a step during which the sample is treated by anion exchange chromatography and/or gel permeation chromatography.

In one aspect, the biological sample is a culture supernatant treated so as to be enriched with membrane vesicles. In particular, the biological sample may be composed of a pre-purified solution obtained from a culture supernatant of a population of membrane vesicle-producing cells or from a biological fluid, by treatments such as centrifugation, clarification, ultrafiltration, nanofiltration and/or affinity chromatography, particularly with clarification and/or ultrafiltration and/or affinity chromatography. Therefore, certain methods of preparing membrane vesicles according to this disclosure comprise the following steps: a) culturing a population of membrane vesicle (e.g. exosome) producing cells under conditions enabling the release of vesicles, b) a step of enrichment of the sample in membrane vesicles, and c) an anion exchange chromatography and/or gel permeation chromatography treatment of the sample.

As indicated above, the sample (e.g. supernatant) enrichment step may comprise one or more centrifugation, clarification, ultrafiltration, nanofiltration and/or affinity chromatography steps on the supernatant. In a first specific aspect, the enrichment step comprises (i) the elimination of cells and/or cell debris (clarification), possibly followed by (ii) a concentration and/or affinity chromatography step. In another specific aspect, the enrichment step comprises an affinity chromatography step, optionally preceded by a step of elimination of cells and/or cell debris (clarification). In some aspects, an enrichment step according to this disclosure comprises (i) the elimination of cells and/or cell debris (clarification), (ii) a concentration and (iii) an affinity chromatography. The cells and/or cell debris may be eliminated by centrifugation of the sample, for example, at a low speed, such as below 1000×g, between 100 and 700×g, for example. In some aspects, centrifugation conditions during this step are approximately 300×g or 600×g for a period between 1 and 15 minutes, for example.

The cells and/or cell debris may also be eliminated by filtration of the sample, possibly combined with the centrifugation described above. The filtration may particularly be performed with successive filtrations using filters with a decreasing porosity. For this purpose, filters with a porosity above 0.2 $\mu$m, e.g. between 0.2 and 10 $\mu$m, may be used. It is particularly possible to use a succession of filters with a porosity of 10 $\mu$m, 1 $\mu$m, 0.5 $\mu$m followed by 0.22 $\mu$m.

A concentration step may also be performed, in order to reduce the volumes of sample to be treated during the chromatography stages. In this way, the concentration may be obtained by centrifugation of the sample at high speeds, e.g. between 10,000 and 100,000×g, to cause the sedimentation of the membrane vesicles. This may consist of a series of differential centrifugations, with the last centrifugation performed at approximately 70,000×g. The membrane vesicles in the pellet obtained may be taken up with a smaller volume and in a suitable buffer for the subsequent steps of the process. The concentration step may also be performed by ultrafiltration. In fact, this ultrafiltration allows both to concentrate the supernatant and perform an initial purification of the vesicles. According to one aspect, the biological sample (e.g., the supernatant) is subjected to an ultrafiltration, such as a tangential ultrafiltration. Tangential ultrafiltration consists of concentrating and fractionating a solution between two compartments (filtrate and retentate), separated by membranes of determined cut-off thresholds. The separation is carried out by applying a flow in the retentate compartment and a transmembrane pressure between this compartment and the filtrate compartment. Different systems may be used to perform the ultrafiltration, such as spiral membranes (Millipore, Amicon), flat membranes or hollow fibres (Amicon, Millipore, Sartorius, Pall, GF, Sepracor). Within the scope of the disclosure, the use of membranes with a cut-off threshold below 1000 kDa, such as between 300 kDa and 1000 kDa, or such as between 300 kDa and 500 kDa, is advantageous.

The affinity chromatography step can be performed in various ways, using different chromatographic support and material. It is advantageously a non-specific affinity chromatography, aimed at retaining (i.e., binding) certain contaminants present within the solution, without retaining the objects of interest (i.e., the exosomes). It is therefore a negative selection. In certain aspects, an affinity chromatography on a dye is used, allowing the elimination (i.e., the retention) of contaminants such as proteins and enzymes, for instance albumin, kinases, deshydrogenases, clotting factors, interferons, lipoproteins, or also co-factors, etc. In certain aspects, the support used for this chromatography step is a support as used for the ion exchange chromatography, functionalized with a dye. As specific example, the dye may be selected from Blue SEPHAROSE®(Pharmacia), YELLOW 86, GREEN 5 and BROWN 10 (Sigma). The support may be agarose. It should be understood that any other support and/or dye or reactive group allowing the retention (binding) of contaminants from the treated biological sample can be used in the instant disclosure.

In one aspect, a membrane vesicle preparation process within the scope of this disclosure comprises the following steps: a) the culture of a population of membrane vesicle (e.g. exosome) producing cells under conditions enabling the release of vesicles, b) the treatment of the culture supernatant with at least one ultrafiltration or affinity chromatography step, to produce a biological sample enriched with membrane vesicles (e.g. with exosomes), and c) an anion exchange chromatography and/or gel permeation chromatography treatment of the biological sample. In certain aspects, step b) above comprises a filtration of the culture supernatant, followed by an ultrafiltration, such as tangential. In another aspect, step b) above comprises a clarification of the culture supernatant, followed by an affinity chromatography on dye, such as on Blue SEPHAROSE®.

In addition, after step c), the material harvested may, if applicable, be subjected to one or more additional treatment and/or filtration stages d), particularly for sterilisation purposes. For this filtration treatment stage, filters with a diameter less than or equal to 0.3 $\mu$m, or less than or equal to 0.25 $\mu$m may be used. Such filters have a diameter of 0.22 $\mu$m, for example. After step d), the material obtained is, for example, distributed into suitable devices such as bottles, tubes, bags, syringes, etc., in a suitable storage medium. The purified vesicles obtained in this way may be stored cold, frozen or used extemporaneously. Therefore, a specific preparation process within the scope of the disclosure comprises at least the following steps: c) an anion exchange chromatography and/or gel permeation chromatography treatment of the biological sample, and d) a filtration step, particularly sterilizing filtration, of the material harvested after stage c). In a first variant, the process according to the disclosure comprises: c) an anion exchange chromatography treatment of the biological sample, and d) a filtration step, particularly sterilizing filtration, on the material harvested after step c).

In another variant, the process according to the disclosure comprises: c) a gel permeation chromatography treatment of the biological sample, and d) a filtration step, particularly sterilizing filtration, on the material harvested after step c). According to a third variant, the process according to the disclosure comprises: c) an anionic exchange treatment of the biological sample followed or preceded by gel permeation chromatography, and d) a filtration step, particularly sterilizing filtration, on the material harvested after step c).

In some aspects, apoptotic bodies of cells are utilized to enhance the activity and/or engraftment of at least one cellular therapy, including at least one fibroblast therapy. Apoptotic bodies may be generated from any cell undergoing apoptosis. Apoptosis may be induced in vitro using known methods in the art, including treatment with photosensitizers followed by irradiation, gamma irradiation, X-radiation; induction of mitotic arrest; and/or exposure to ozone gas. Apoptosis-characterizing features may include, but are not limited to, surface exposure of phosphatidylserine, as detected by standard, accepted methods of detection such as Annexin V staining; alterations in mitochondrial membrane permeability measured by standard methods; DNA fragmentation as measured by accepted methods of analyzing DNA fragmentation such as the appearance of DNA laddering on agarose gel electrophoresis following extraction of DNA from the cells or by in situ labeling. In some aspects, apoptotic bodies, such as allogeneic fibroblast apoptotic bodies, may be administered alone, or may be administered together with a tolerogenic adjuvant such as immature dendritic cells, T regulatory cells, mesenchymal stem cells, fibroblasts, and/or gene modified cells.

Exosomes and/or apoptotic bodies encompassed herein may be administered to an individual, including any individual encompassed herein. Exosomes and/or apoptotic bodies may be administered to the individual systemically and/or locally. Exosomes and/or apoptotic bodies may be administered to the individual intravenously, intradiscally, epidurally, intrarectally, intra-omentally, intra-arterially or a combination thereof. Exosomes and/or apoptotic bodies may be administered prior to or simultaneously with the compositions and/or cellular therapies encompassed herein.

## VI. Administration of Therapeutic Compositions

The therapy provided herein may comprise administration of a therapeutic agents alone or in combination. Therapies may be administered in any suitable manner known in the art. For example, a first and second treatment may be administered sequentially (at different times) or concurrently (at the same time). In some aspects, the first and second treatments are administered in a separate composition. In some aspects, the first and second treatments are in the same composition.

Aspects of the disclosure relate to compositions and methods comprising therapeutic compositions. The different therapies may be administered in one composition or in more than one composition, such as 2 compositions, 3 compositions, or 4 compositions. Various combinations of the agents may be employed.

The therapeutic agents of the disclosure may be administered by the same route of administration or by different routes of administration. In some aspects, the cancer therapy is administered intravenously, intramuscularly, subcutaneously, topically, orally, transdermally, intraperitoneally, intraorbitally, by implantation, by inhalation, intrathecally, intraventricularly, or intranasally. In some aspects, the antibiotic is administered intravenously, intramuscularly, subcutaneously, topically, orally, transdermally, intraperitoneally, intraorbitally, by implantation, by inhalation, intrathecally, intraventricularly, or intranasally. The appropriate dosage may be determined based on the type of disease to be treated, severity and course of the disease, the clinical condition of the individual, the individual's clinical history and response to the treatment, and the discretion of the attending physician.

The treatments may include various "unit doses." Unit dose is defined as containing a predetermined-quantity of the therapeutic composition. The quantity to be administered, and the particular route and formulation, is within the skill of determination of those in the clinical arts. A unit dose need not be administered as a single injection but may comprise continuous infusion over a set period of time. In some aspects, a unit dose comprises a single administrable dose.

In some aspects, about 50 million to 500 million fibroblast cells are administered to the subject. For example, about 50 million to about 100 million fibroblast cells, about 50 million to about 200 million fibroblast cells, about 50 million to about 300 million fibroblast cells, about 50 million to about 400 million fibroblast cells, about 100 million to about 200 million fibroblast cells, about 100 million to about 300 million fibroblast cells, about 100 million to about 400 million fibroblast cells, about 100 million to about 500 million fibroblast cells, about 200 million to about 300 million fibroblast cells, about 200 million to about 400 million fibroblast cells, about 200 million to about 500 million fibroblast cells, about 300 million to about 400 million fibroblast cells, about 300 million to about 500 million fibroblast cells, about 400 million to about 500 million fibroblast cells, about 50 million fibroblast cells, about 100 million fibroblast cells, about 150 million fibroblast cells, about 200 million fibroblast cells, about 250 million fibroblast cells, about 300 million fibroblast cells, about 350 million fibroblast cells, about 400 million fibroblast cells, about 450 million fibroblast cells or about 500 million fibroblast cells may be administered to the subject.

The quantity to be administered, both according to number of treatments and unit dose, depends on the treatment effect desired. An effective dose is understood to refer to an amount necessary to achieve a particular effect. In the practice in certain aspects, it is contemplated that doses in the range from 10 mg/kg to 200 mg/kg can affect the protective capability of these agents. Thus, it is contemplated that doses include doses of about 0.1, 0.5, 1, 5, 10, 15, 20, 25, 30, 35, 40, 45, 50, 55, 60, 65, 70, 75, 80, 85, 90, 100, 105, 110, 115, 120, 125, 130, 135, 140, 145, 150, 155, 160, 165, 170, 175, 180, 185, 190, 195, and 200, 300, 400, 500, 1000 $\mu$g/kg, mg/kg, $\mu$g/day, or mg/day or any range derivable therein. Furthermore, such doses can be administered at multiple times during a day, and/or on multiple days, weeks, or months.

In certain aspects, the effective dose of the pharmaceutical composition is one which can provide a blood level of about 1 $\mu$M to 150 $\mu$M. In another aspect, the effective dose provides a blood level of about 4 $\mu$M to 100 $\mu$M.; or about 1 $\mu$M to 100 $\mu$M; or about 1 $\mu$M to 50 $\mu$M; or about 1 $\mu$M to 40 $\mu$M; or about 1 $\mu$M to 30 $\mu$M; or about 1 $\mu$M to 20 $\mu$M; or about 1 $\mu$M to 10 $\mu$M; or about 10 $\mu$M to 150 $\mu$M; or about 10 $\mu$M to 100 $\mu$M; or about 10 $\mu$M to 50 $\mu$M; or about 25 $\mu$M to 150 $\mu$M; or about 25 $\mu$M to 100 $\mu$M; or about 25 $\mu$M to 50 $\mu$M; or about 50 $\mu$M to 150 $\mu$M; or about 50 $\mu$M to 100 $\mu$M (or any range derivable therein). In other aspects, the dose can provide the following blood level of the agent that results from a therapeutic agent being administered to a subject: about, at least about, or at most about 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, or 100 $\mu$M or any range derivable therein. In certain aspects, the therapeutic agent that is administered to a subject is metabolized in the body to a metabolized therapeutic agent, in which case the blood levels may refer to the amount of that agent. Alternatively, to the extent the therapeutic agent is not metabolized by a subject, the blood levels discussed herein may refer to the unmetabolized therapeutic agent.

Precise amounts of the therapeutic composition also depend on the judgment of the practitioner and are peculiar to each individual. Factors affecting dose include physical and clinical state of the patient, the route of administration, the intended goal of treatment (alleviation of symptoms versus cure) and the potency, stability and toxicity of the particular therapeutic substance or other therapies a subject may be undergoing.

It will be understood by those skilled in the art and made aware that dosage units of $\mu$g/kg or mg/kg of body weight can be converted and expressed in comparable concentration units of $\mu$g/mL or mM (blood levels), such as 4 $\mu$M to 100 $\mu$M. It is also understood that uptake is species and organ/tissue dependent. The applicable conversion factors and physiological assumptions to be made concerning uptake and concentration measurement are well-known and would permit those of skill in the art to convert one concentration measurement to another and make reasonable comparisons and conclusions regarding the doses, efficacies and results described herein.

## VII. Kits of the Disclosure

Any of the cellular and/or non-cellular compositions described herein or similar thereto may be comprised in a kit. In a non-limiting example, one or more reagents for use in methods for preparing fibroblasts or derivatives thereof (*e.g.,* exosomes derived from fibroblasts) may be comprised in a kit. Such reagents may include cells, vectors, one or more growth factors, vector(s) one or more costimulatory factors, media, enzymes, buffers, nucleotides, salts, primers, compounds, and so forth. The kit components are provided in suitable container means.

Some components of the kits may be packaged either in aqueous media or in lyophilized form. The container means of the kits will generally include at least one vial, test tube, flask, bottle, syringe or other container means, into which a component may be placed and suitably aliquoted. Where there are more than one component in the kit, the kit also will generally contain a second, third or other additional container into which the additional components may be separately placed. However, various combinations of components may be comprised in a vial. The kits of the present disclosure also will typically include a means for containing the components in close confinement for commercial sale. Such containers may include injection or blow molded plastic containers into which the desired vials are retained.

When the components of the kit are provided in one and/or more liquid solutions, the liquid solution is an aqueous solution, with a sterile aqueous solution being particularly useful. In some cases, the container means may itself be a syringe, pipette, and/or other such like apparatus, or may be a substrate with multiple compartments for a desired reaction.

Some components of the kit may be provided as dried powder(s). When reagents and/or components are provided as a dry powder, the powder can be reconstituted by the addition of a suitable solvent. It is envisioned that the solvent may also be provided in another container means. The kits may also comprise a second container means for containing a sterile acceptable buffer and/or other diluent.

In specific aspects, reagents and materials include primers for amplifying desired sequences, nucleotides, suitable buffers or buffer reagents, salt, and so forth, and in some cases the reagents include apparatus or reagents for isolation of a particular desired cell(s).

In particular aspects, there are one or more apparatuses in the kit suitable for extracting one or more samples from an individual. The apparatus may be a syringe, fine needles, scalpel, and so forth.

## EXAMPLES

The following examples are included to demonstrate particular aspects of the disclosure.

## EXAMPLE 1: FIBROBLAST CONDITIONED MEDIA PROTECTS FROM IN VITRO DEATH OF NEURON CELL LINE PC-12 IN GLUCOSE/OXYGEN DEPRIVATION

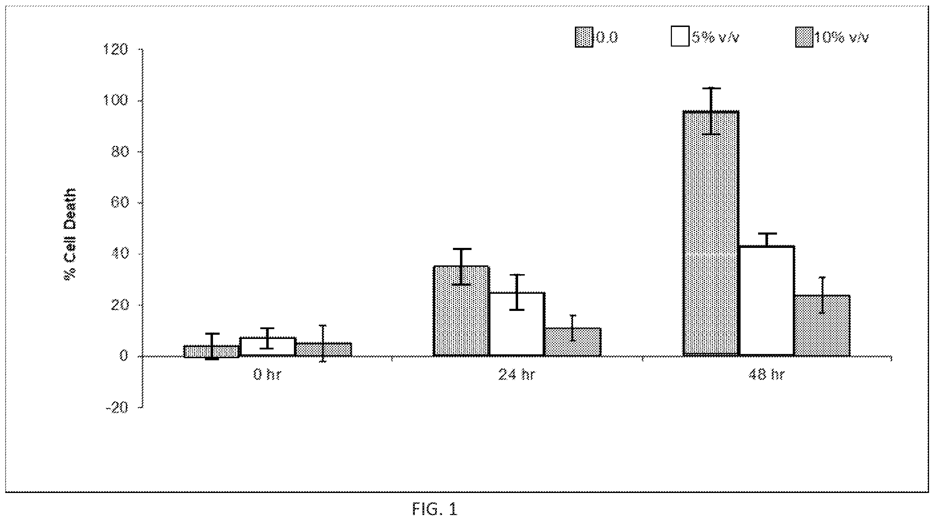

Foreskin fibroblasts where obtained from American Type Culture Collection (ATCC) and cultured according to the manufacturer's instructions. Conditioned media was obtained from 50-75% confluent fibroblast cultures after 24 hours of culture with conditioned media lacking glucose. Media was centrifuged to ensure lack of cellular debris or cells. Centrifugation was performed at 500$\times$g for 10 minutes. Condition media was added to cultures of PC-12 cells that were depleted of glucose and oxygen by culture in anoxic conditions in glucose free media with addition of 0%, 5%, or 10% volume per volume. Cells were incubated for 1, 24, or 48 hours and cell death was assessed by trypan blue staining. As observed in FIG. 1, a protective effect was noted.

EXAMPLE 2: FIBROBLAST CONDITIONED MEDIA PROTECT FROM IN VITRO DEATH OF PRIMARY NEURONS IN GLUCOSE/OXYGEN DEPRIVATION

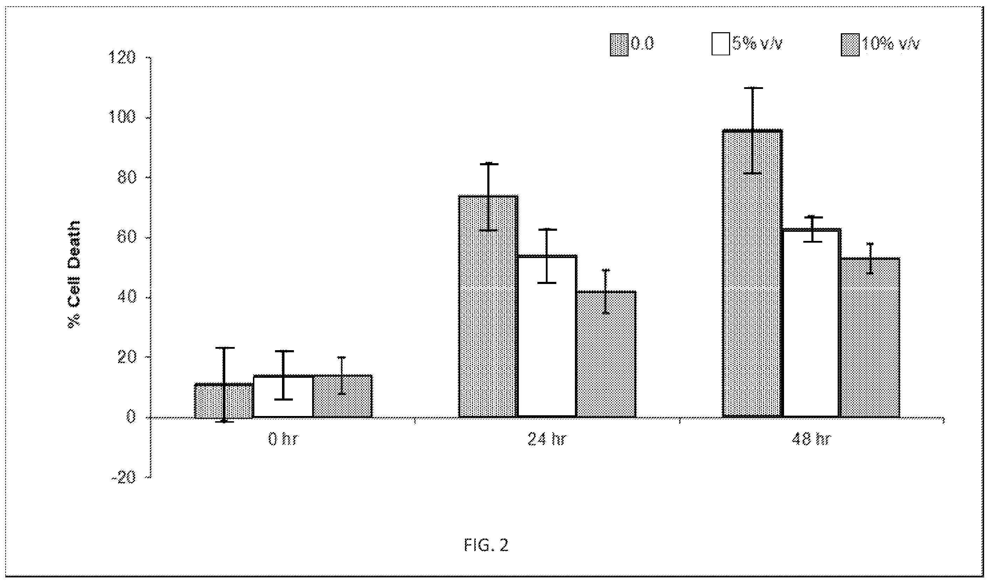

Foreskin fibroblasts where obtained from ATCC and cultured according to the manufacturer's instructions. Conditioned media was obtained from 50-75% confluent fibroblast cultures after 24 hours of culture with conditioned media lacking glucose. Media was centrifuged to ensure lack of cellular debris or cells. Centrifugation was performed at 500×g for 10 minutes. Condition media was added to cultures of primary neurons (ATCC) that were depleted of glucose and oxygen by culture in anoxic conditions in glucose free media with addition of 0%, 5%, or 10% volume per volume. Cells were incubated for 1, 24, or 48 hours and cell death was assessed by trypan blue staining. As observed in FIG. 2, a protective effect was noted.

EXAMPLE 3: FIBROBLAST EXOSOMES PROTECT FROM IN VITRO DEATH OF NEURON CELL LINE PC-12 IN GLUCOSE/OXYGEN DEPRIVATION

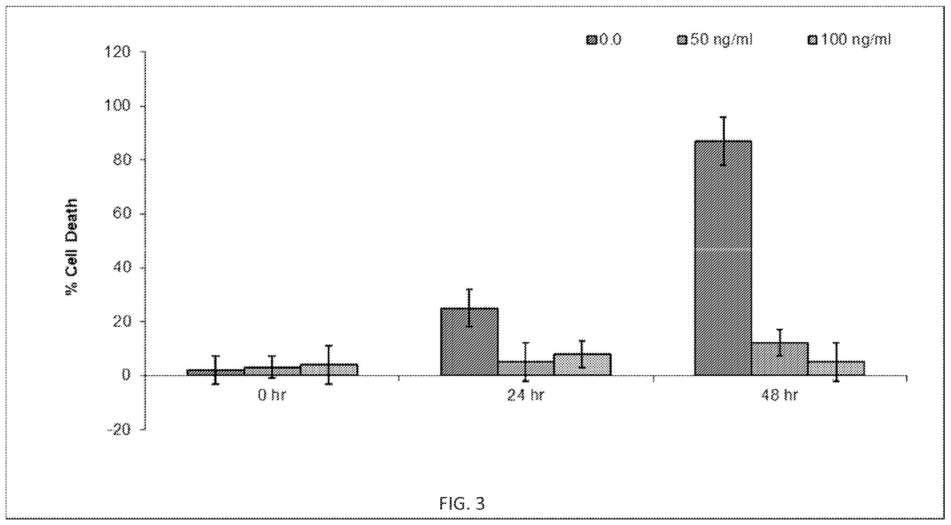

Foreskin fibroblasts were obtained from ATCC and cultured according to the manufacturer's instructions. Conditioned media was obtained from 50-75% confluent fibroblast cultures after 24 hours of culture with conditioned media lacking glucose. Media was centrifuged to ensure lack of cellular debris or cells. Centrifugation was performed at 500×g for 10 minutes. A further centrifugation step (100,000×g for 3 hours) was performed to concentrate exosomes was performed. Exosomes were diluted at the indicated concentrations and added to PC-12 cells incubated for 1, 24, or 48 hours and cell death was assessed by trypan blue staining. As observed in FIG. 3, a protective effect was noted.

EXAMPLE 4: FIBROBLAST EXOSOMES PROTECT FROM IN VITRO DEATH OF PRIMARY NEURONS IN GLUCOSE/OXYGEN DEPRIVATION

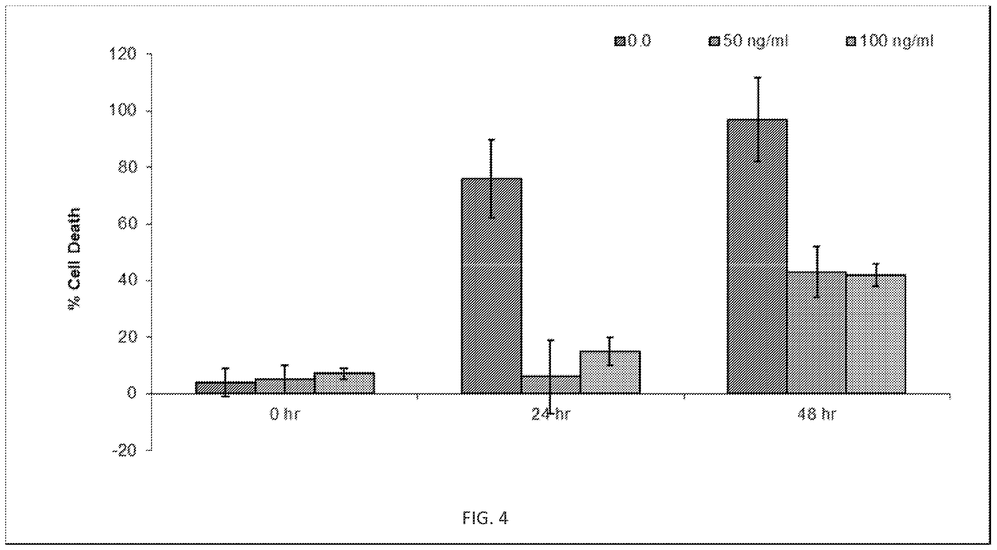

Foreskin fibroblasts were obtained from ATCC and cultured according to the manufacturer's instructions. Conditioned media was obtained from 50-75% confluent fibroblast cultures after 24 hours of culture with conditioned media lacking glucose. Media was centrifuged to ensure lack of cellular debris or cells. Centrifugation was performed at 500×g for 10 minutes. A further centrifugation step (100,000×g for 3 hours) was performed to concentrate exosomes was performed. Exosomes were diluted at the indicated concentrations and added to primary cells incubated for 1, 24, or 48 hours and cell death was assessed by trypan blue staining. As observed in FIG. 4, a protective effect was noted.

EXAMPLE 5: SYNERGY OF FIBROBLAST EXOSOMES WITH METFORMIN FOR NEUROPROTECTION: PC-12

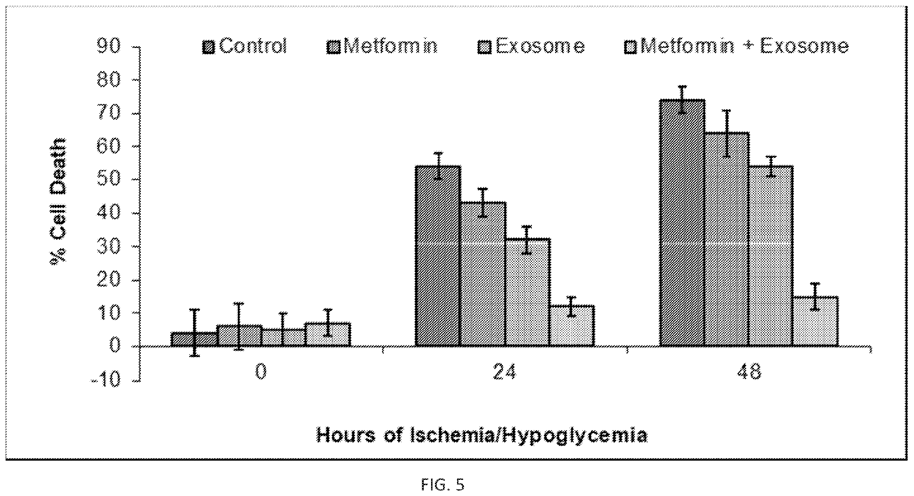

Foreskin fibroblasts were obtained from ATCC and cultured according to the manufacturer's instructions. Conditioned media was obtained from 50-75% confluent fibroblast cultures after 24 hours of culture with conditioned media lacking glucose. Media was centrifuged to ensure lack of cellular debris or cells. Centrifugation was performed at 500×g for 10 minutes. A further centrifugation step (100,000×g for 3 hours) was performed to concentrate exosomes was performed. Exosomes were diluted at 20ng/mL and added to PC-12 cells incubated for 1, 24, or 48 hours. Additionally, metformin (5 $\mu$Mol) and metformin with exosomes were added. Cell death was assessed by trypan blue staining. As observed in FIG. 5, a protective effect was noted in the combination of metformin and exosomes.

EXAMPLE 6: SYNERGY OF FIBROBLAST EXOSOMES WITH METFORMIN FOR NEUROPROTECTION: PRIMARY NEURONS

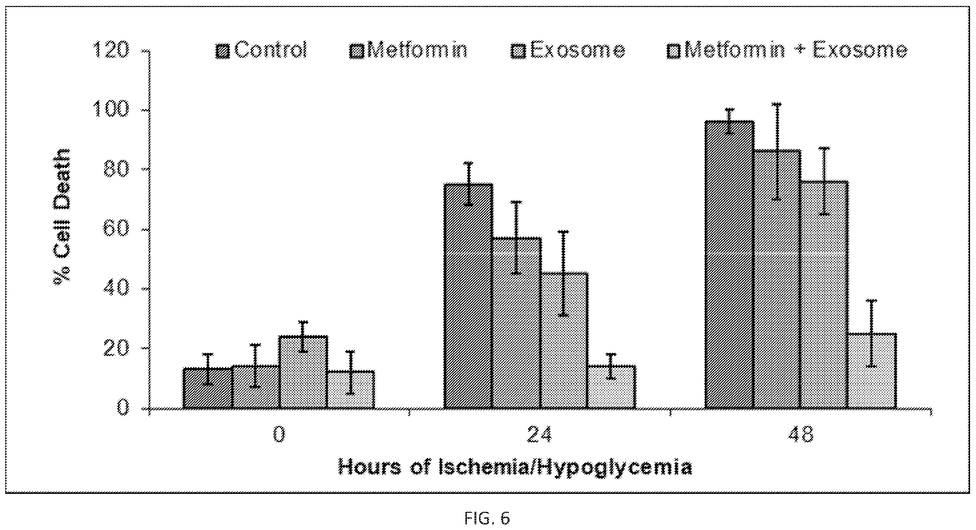

Foreskin fibroblasts were obtained from ATCC and cultured according to the manufacturer's instructions. Conditioned media was obtained from 50-75% confluent fibroblast cultures after 24 hours of culture with conditioned media lacking glucose. Media was centrifuged to ensure lack of cellular debris or cells. Centrifugation was performed at 500×g for 10 minutes. A further centrifugation step (100,000×g for 3 hours) was performed to concentrate exosomes was performed. Exosomes were diluted at the indicated concentrations (20 ng/mL) and added to primary neuron cells incubated for 1, 24, or 48 hours. Additionally, metformin (5 $\mu$Mol) and metformin with exosomes were added. Cell death was assessed by trypan blue staining. As observed in FIG. 6, a protective effect was noted in the combination of metformin and exosomes.

EXAMPLE 7: SYNERGY OF FIBROBLAST EXOSOMES WITH MINOCYCLINE FOR NEUROPROTECTION: PC12

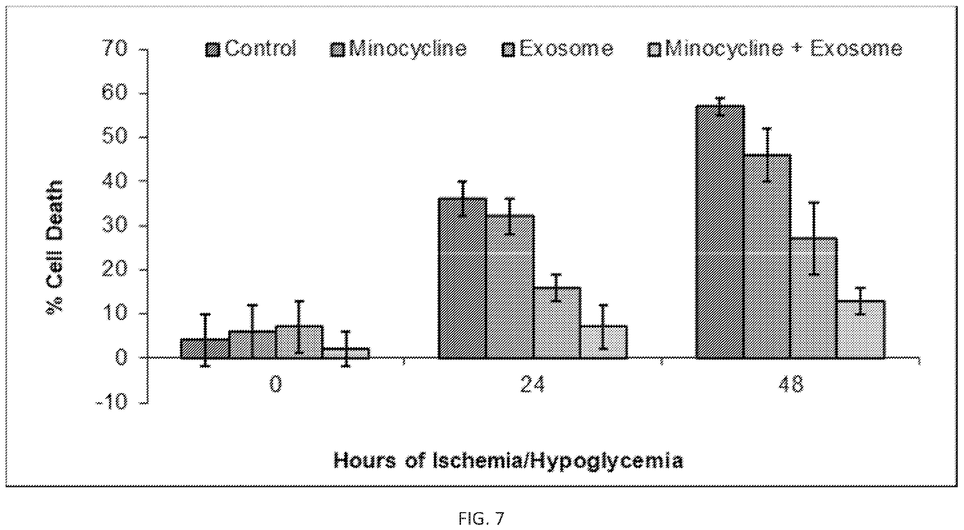

Foreskin fibroblasts were obtained from ATCC and cultured according to the manufacturer's instructions. Conditioned media was obtained from 50-75% confluent fibroblast cultures after 24 hours of culture with conditioned media lacking glucose. Media was centrifuged to ensure lack of cellular debris or cells. Centrifugation was performed at 500×g for 10 minutes. A further centrifugation step (100,000×g for 3 hours) was performed to concentrate exosomes was performed. Exosomes were diluted at the indicated concentrations (20ng/mL) and added to PC-12 cells incubated for 1, 24, or 48 hours. Additionally, minocycline (1 $\mu$Mol) and minocycline with exosomes were added. Cell death was assessed by trypan blue staining. As observed in FIG. 7, a protective effect was noted in the combination of metformin and exosomes.

EXAMPLE 8: SYNERGY OF FIBROBLAST EXOSOMES WITH MINOCYCLINE FOR NEUROPROTECTION: PRIMARY NEURONS

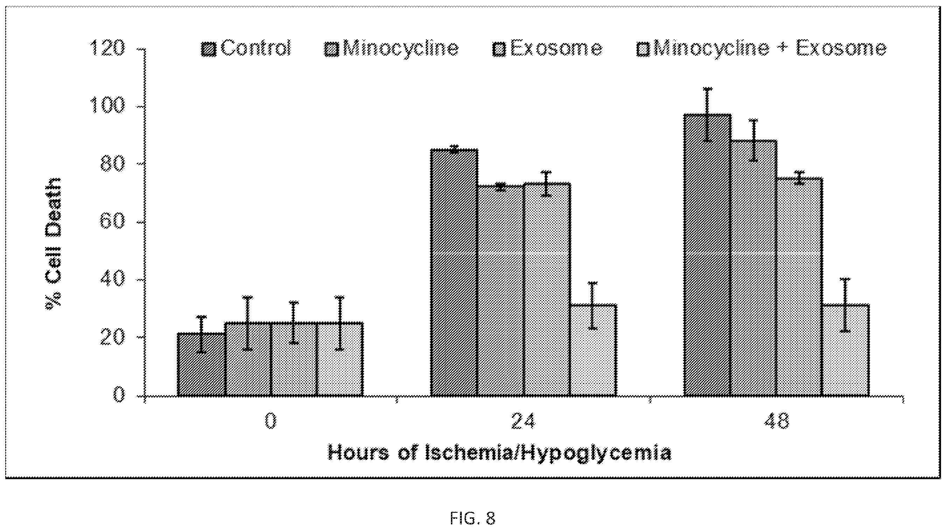

Foreskin fibroblasts were obtained from ATCC and cultured according to the manufacturer's instructions. Conditioned media was obtained from 50-75% confluent fibroblast cultures after 24 hours of culture with conditioned media lacking glucose. Media was centrifuged to ensure lack of cellular debris or cells. Centrifugation was performed at 500×g for 10 minutes. A further centrifugation step (100,000×g for 3 hours) was performed to concentrate exosomes was performed. Exosomes were diluted at the indicated concentrations (20 ng/mL) and added to primary neuron cells incubated for 1, 24, or 48 hours. Additionally, minocycline (1 μMol) and minocycline with exosomes were added. Cell death was assessed by trypan blue staining (FIG. 8). In specific aspects, a protective effect is provided with the combination of minocycline and exosomes.

EXAMPLE 9: REDUCTION IN STROKE INFARCT VOLUME BY AMNIOTIC FIBROBLASTS

Amniotic fibroblasts were generated by purification of mononuclear cells from 5 ml amniocentesis derived fluid. Briefly, fresh amniotic fluid was harvested from women undergoing routine amniocentesis at 16 to 21 weeks of pregnancy (second trimester). Second trimester amniotic fluid contained approximately 1-2 x 104 live cells per ml. The cells were pelleted in a clinical centrifuge and resuspended in 15 mL medium. The medium was composed of low glucose Dulbecco Modified Eagle's Medium (GIBCO, Carlsbad, Calif.) and MCDB 201 medium (SIGMA, Saint Louis, Mo.) at a one to one ratio and contained 2% Defined Fetal Calf Serum (HYCLONE, Logan, Utah), 1x insulin-transferrin-selenium, linoleic-acid-bovine-serum-albumin (ITS+1, SIGMA), 1 nanomolar dexamethasone (Sigma), 100 μm ascorbic acid 2-phosphate (Sigma), 4 μm/mL gentamycin, 10 ng/mL of rhEGF (R&D Systems, Minneapolis, Minn.), 10 ng/mL rrPDGF-BB (R&D) and 10 ng/mL rhFGF-basic (R&D).

Cells were selected for CD73, and/or CD56 by magnetic activated cell sorting following manufacturer's instructions (Miltenyi Biotec). The wells of 6-well culture dishes were prepared for cell plating by coating for one hour at room temperature with 2.5 mL of fibronectin (stock of 10 μg fibronectin/mL of sterile water) immediately prior to cell plating. The fibronectin solution was removed prior to cell plating and the wells were not washed after removal of the fibronectin solution. The cells were then seeded in 2.5 mL of medium in each well.Cells were transferred to successively larger fibronectin-coated flasks/vessels. To perform cell transfer, the cells were grown to a subconfluent state of approximately 40% confluence and were detached with 0.25% Trypsin-EDTA and replated at a 1:3 or 1: 12 dilution under the same culture conditions.

Adult male Sprague-Dawley rats weighing about 270 to 400 grams were used for the stroke model. Right MCAO was performed by intraluminal vascular occlusion with the monofilament method. Rats were initially anesthetized by confinement to a sealed box with 5% isoflurane, and general anesthesia was maintained by facial mask with 1.5% isoflurane and a mixture of 70% $N_2O$ and 30% $O_2$. Body temperature was measured by rectal measurement and maintained at 37 °C by using a heating pad. In the supine position, bilateral arms, legs, and tail were fixed on a plate. A linear midline vertical skin incision was made on the neck above the sternal notch after the hair was shaved. After dissecting the right sternocleidomastoid and omohyoid muscles, exposure of the right common carotid artery and carotid bifurcation were achieved. Ligation of the common carotid artery, external carotid artery, and pterygopalatine artery was carried out with 5-0 silk. After that, the bilateral tips of a 20-mm-sized 4-0 monofilament were heated near an alcohol lamp flame, producing oval-shaped tips. A tiny incision was made with the sharp needle tip on the common carotid artery, and the monofilament was introduced through the common carotid artery to the internal carotid artery and advanced 20 mm to locate the tip of the monofilament on the proximal middle cerebral artery (MCA). Internal carotid artery ligation followed monofilament introduction and skin suture.

40 MCAO rats were randomly assigned to one of four groups: control (n = 10), Amniotic Fluid Unseparated Fibroblasts (n = 10), CD73 selected fibroblasts (n = 10), or aCD56 selected amniotic stem cells (n = 10). An intravenous phosphate-buffered saline (PBS) injection (100 μL) was carried out 72 h after MCAO via the tail vein. 1.5 × 106 cells were injected.

Stroke Infract Volume was assessed 28 days after infarct. Rats were sacrificed and their brains were removed and sliced into 2-mm-thick coronal sections from bregma by using a rat brain mold. Sliced brain sections were soaked in a 2% solution of 2,3,5-triphenyl tetrazolium chloride (Sigma-Aldrich) with PBS for 30 min at a temperature of 37 °C. The volume of infarction in each coronal section was measured by MetaMorph image analyzing software (Molecular Devices, San Jose, CA, USA). To avoid overestimation of infarction volume due to brain edema, the corrected infarction volume (CIV) was obtained by the following formula:

$$CIV=[LT-(RT-RI)]×d,$$

where LT is the volume of the left hemisphere, RT is the volume of the right hemisphere, RI is the volume of the infarcted area, and d is the slice thickness (in millimeters).

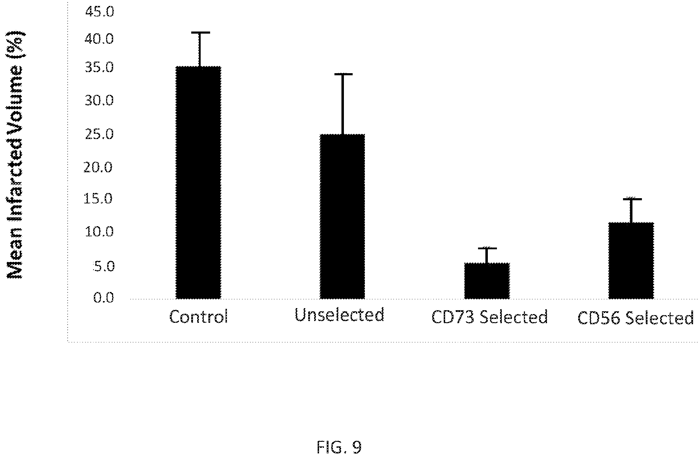

Total CIV was calculated by the sum of all slices, and the percentage of total infarcted volume was measured. CD73 selected fibroblasts had the smallest mean infarcted volume of the four groups (FIG. 9).

REFERENCES

1. Feigin, V.L., et al., Worldwide stroke incidence and early case fatality reported in 56 population-based studies: a systematic review. Lancet Neurol, 2009. 8(4): p. 355-69.

2. Broderick, J.P., et al., Guidelines for the management of spontaneous intracerebral hemorrhage: A statement for healthcare professionals from a special writing group of the Stroke Council, American Heart Association. Stroke, 1999. 30(4): p. 905-15.

3. Wang, J. and S.E. Tsirka, Tuftsin fragment 1-3 is beneficial when delivered after the induction of intracerebral hemorrhage. Stroke, 2005. 36(3): p. 613-8.

4. Hijioka, M., et al., Therapeutic effect of nicotine in a mouse model of intracerebral hemorrhage. J Pharmacol Exp Ther, 2011.

5. Gu, Y., C.M. Dee, and J. Shen, Interaction of free radicals, matrix metalloproteinases and caveolin-1 impacts blood-brain barrier permeability. Front Biosci (Schol Ed), 2011. 3: p. 1216-31.

6. Ma, Q., et al., Vascular adhesion protein-1 inhibition provides antiinflammatory protection after an intracerebral hemorrhagic stroke in mice. J Cereb Blood Flow Metab, 2011. 31(3): p. 881-93.

7. Lekic, T., et al., Protective effect of melatonin upon neuropathology, striatal function, and memory ability after intracerebral hemorrhage in rats. J Neurotrauma, 2010. 27(3): p. 627-37.

8. MacLellan, C.L., et al., Intracerebral hemorrhage models in rat: comparing collagenase to blood infusion. J Cereb Blood Flow Metab, 2008. 28(3): p. 516-25.

9. Masuda, T., et al., Increase in neurogenesis and neuroblast migration after a small intracerebral hemorrhage in rats. Neurosci Lett, 2007. 425(2): p. 114-9.

10. Hua, Y., et al., Thrombin and brain recovery after intracerebral hemorrhage. Stroke, 2009. 40(3 Suppl): p. S88-9.

11. Yang, S., et al., Effects of thrombin on neurogenesis after intracerebral hemorrhage. Stroke, 2008. 39(7): p. 2079-84.

12. Bolander, H.G., et al., Treatment of spontaneous intracerebral haemorrhage. A retrospective analysis of 74 consecutive cases with special reference to computertomographic data. Acta Neurochir (Wien), 1983. 67(1-2): p. 19-28.

13. Diringer, M.N., Intracerebral hemorrhage: pathophysiology and management. Crit Care Med, 1993. 21(10): p. 1591-603.

14. Liao, W., et al., Therapeutic benefit of human umbilical cord derived mesenchymal stromal cells in intracerebral hemorrhage rat: implications of anti-inflammation and angiogenesis. Cell Physiol Biochem, 2009. 24(3-4): p. 307-16.

15. Fatar, M., et al., Lipoaspirate-derived adult mesenchymal stem cells improve functional outcome during intracerebral hemorrhage by proliferation of endogenous progenitor cells stem cells in intracerebral hemorrhages. Neurosci Lett, 2008. 443(3): p. 174-8.

16. Krenzlin, H., et al., The cerebral thrombin system is activated after intracerebral hemorrhage and contributes to secondary lesion growth and poor neurological outcome in C57Bl/6 mice. J Neurotrauma, 2019.

17. Wang, Y., et al., Simvastatin accelerates hematoma resolution after intracerebral hemorrhage in a PPARgamma-dependent manner. Neuropharmacology, 2018. 128: p. 244-254.

18. Jung, K.H., et al., HMG-CoA reductase inhibitor, atorvastatin, promotes sensorimotor recovery, suppressing acute inflammatory reaction after experimental intracerebral hemorrhage. Stroke, 2004. 35(7): p. 1744-9.

19. Lu, D., et al., Atorvastatin reduction of intracranial hematoma volume in rats subjected to controlled cortical impact. J Neurosurg, 2004. 101(5): p. 822-5.

20. Karki, K., et al., Simvastatin and atorvastatin improve neurological outcome after experimental intracerebral hemorrhage. Stroke, 2009. 40(10): p. 3384-9.

21. Todo, T., M. Usui, and K. Takakura, Treatment of severe intraventricular hemorrhage by intraventricular infusion of urokinase. J Neurosurg, 1991. 74(1): p. 81-6.

22. Aydin, I.H., et al., The effect of urokinase on experimental intracerebral haematomas. Zentralbl Neurochir, 1994. 55(1): p. 29-34.

23. Naff, N.J., et al., Treatment of intraventricular hemorrhage with urokinase : effects on 30-Day survival. Stroke, 2000. 31(4): p. 841-7.

24. Findlay, J.M., et al., Intracisternal recombinant tissue plasminogen activator after aneurysmal subarachnoid hemorrhage. J Neurosurg, 1991. 75(2): p. 181-8.

25. von Kummer, R. and W. Hacke, Safety and efficacy of intravenous tissue plasminogen activator and heparin in acute middle cerebral artery stroke. Stroke, 1992. 23(5): p. 646-52.

26. Mayfrank, L., et al., Effect of recombinant tissue plasminogen activator on clot lysis and ventricular dilatation in the treatment of severe intraventricular haemorrhage. Acta Neurochir (Wien), 1993. 122(1-2): p. 32-8.

27. Nasser, J.A., et al., Stereotactic fibrinolysis of spontaneous intracerebral hematoma using infusion of recombinant tissue plasminogen activator. Arq Neuropsiquiatr, 2002. 60(2-B): p. 362-6.

28. Rohde, V., et al., Fibrinolysis therapy achieved with tissue plasminogen activator and aspiration of the liquefied clot after experimental intracerebral hemorrhage: rapid reduction in hematoma volume but intensification of delayed edema formation. J Neurosurg, 2002. 97(4): p. 954-62.

29. Seifert, V., et al., Endothelin concentrations in patients with aneurysmal subarachnoid hemorrhage. Correlation with cerebral vasospasm, delayed ischemic neurological deficits, and volume of hematoma. J Neurosurg, 1995. 82(1): p. 55-62.

30. Zimmermann, M., Endothelin in cerebral vasospasm. Clinical and experimental results. J Neurosurg Sci, 1997. 41(2): p. 139-51.

31. Alioglu, Z., et al., Increased plasma endothelin-1 levels in patients with intracerebral hemorrhage. J Stroke Cerebrovasc Dis, 2000. 9(4): p. 176-80.

32. Lyden, P.D., C. Jackson-Friedman, and L. Lonzo-Doktor, Medical therapy for intracerebral hematoma with the gamma-aminobutyric acid-A agonist muscimol. Stroke, 1997. 28(2): p. 387-91.

33. Xi, G., et al., Brain edema after intracerebral hemorrhage: the effects of systemic complement depletion. Acta Neurochir Suppl, 2002. 81: p. 253-6.

34. Rynkowski, M.A., et al., C3a receptor antagonist attenuates brain injury after intracerebral hemorrhage. J Cereb Blood Flow Metab, 2009. 29(1): p. 98-107.

35. Kitaoka, T., et al., Delayed argatroban treatment reduces edema in a rat model of intracerebral hemorrhage. Stroke, 2002. 33(12): p. 3012-8.

36. Kitaoka, T., et al., Effect of delayed argatroban treatment on intracerebral hemorrhage-induced edema in the rat. Acta Neurochir Suppl, 2003. 86: p. 457-61.

37. Terai, K., et al., Effect of AMPA receptor antagonist YM872 on cerebral hematoma size and neurological recovery in the intracerebral hemorrhage rat model. Eur J Pharmacol, 2003. 467(1-3): p. 95-101.

38. Lema, P.P., C. Girard, and P. Vachon, High doses of methylprednisolone are required for the treatment of collagenase-induced intracerebral hemorrhage in rats. Can J Vet Res, 2005. 69(4): p. 253-9.

39. Strbian, D., et al., Mast cell blocking reduces brain edema and hematoma volume and improves outcome after experimental intracerebral hemorrhage. J Cereb Blood Flow Metab, 2007. 27(4): p. 795-802.

40. Sinn, D.I., et al., Combined neuroprotective effects of celecoxib and memantine in experimental intracerebral hemorrhage. Neurosci Lett, 2007. 411(3): p. 238-42.

41. Sinn, D.I., et al., Proteasomal inhibition in intracerebral hemorrhage: neuroprotective and anti-inflammatory effects of bortezomib. Neurosci Res, 2007. 58(1): p. 12-8.

42. Al-Senani, F.M., et al., Proteasome Inhibitor Reduces Astrocytic iNOS Expression and Functional Deficit after Experimental Intracerebral Hemorrhage in Rats. Transl Stroke Res, 2012. 3(1): p. 146-53.

43. Thiex, R., et al., Addition of intravenous N-methyl-D-aspartate receptor antagonists to local fibrinolytic therapy for the optimal treatment of experimental intracerebral hemorrhages. J Neurosurg, 2007. 106(2): p. 314-20.

44. Gaberel, T., et al., Immunotherapy blocking the tissue plasminogen activator-dependent activation of N-methyl-D-aspartate glutamate receptors improves hemorrhagic stroke outcome. Neuropharmacology, 2013. 67: p. 267-71.

45. Mehdiratta, M., et al., Association between serum ferritin level and perihematoma edema volume in patients with spontaneous intracerebral hemorrhage. Stroke, 2008. 39(4): p. 1165-70.

46. Zhou, F., G. Chen, and J. Zhang, Edaravone reduces brain oedema and attenuates cell death after intracerebral haemorrhage in mice. Brain Inj, 2009. 23(4): p. 353-7.

47. Nakamura, T., et al., Serine protease inhibitor attenuates intracerebral hemorrhage-induced brain injury and edema formation in rat. Acta Neurochir Suppl, 2010. 106: p. 307-10.

48. Chun, H.J., et al., Effects of statin and deferoxamine administration on neurological outcomes in a rat model of intracerebral hemorrhage. Neurol Sci, 2012. 33(2): p. 289-96.

49. Auriat, A.M., et al., Ferric iron chelation lowers brain iron levels after intracerebral hemorrhage in rats but does not improve outcome. Exp Neurol, 2012. 234(1): p. 136-43.

50. Hatakeyama, T., et al., Deferoxamine reduces neuronal death and hematoma lysis after intracerebral hemorrhage in aged rats. Transl Stroke Res, 2013. 4(5): p. 546-53.

51. Laskowitz, D.T., et al., The apoE-mimetic peptide, COG1410, improves functional recovery in a murine model of intracerebral hemorrhage. Neurocrit Care, 2012. 16(2): p. 316-26.

52. Matsushita, H., et al., Natural and synthetic retinoids afford therapeutic effects on intracerebral hemorrhage in mice. Eur J Pharmacol, 2012. 683(1-3): p. 125-31.

53. James, M.L., et al., TT-301 inhibits microglial activation and improves outcome after central nervous system injury in adult mice. Anesthesiology, 2012. 116(6): p. 1299-311.

54. Sheng, S.P., et al., Xenon neuroprotection in experimental stroke: interactions with hypothermia and intracerebral hemorrhage. Anesthesiology, 2012. 117(6): p. 1262-75.

55. King, M.D., C.H. Alleyne, Jr., and K.M. Dhandapani, TNF-alpha receptor antagonist, R-7050, improves neurological outcomes following intracerebral hemorrhage in mice. Neurosci Lett, 2013. 542: p. 92-6.

56. Lei, B., et al., Tumor necrosis factor alpha antagonism improves neurological recovery in murine intracerebral hemorrhage. J Neuroinflammation, 2013. 10: p. 103.

57. Lee, S.H., et al., Effects of celecoxib on hematoma and edema volumes in primary intracerebral hemorrhage: a multicenter randomized controlled trial. Eur J Neurol, 2013. 20(8): p. 1161-9.

58. Sabatini, F., et al., Human bronchial fibroblasts exhibit a mesenchymal stem cell phenotype and multilineage differentiating potentialities. Lab Invest, 2005. 85(8): p. 962-71.

59. Lorenz, K., et al., Multilineage differentiation potential of human dermal skin-derived fibroblasts. Exp Dermatol, 2008. 17(11): p. 925-32.

60. Huang, H.I., et al., Multilineage differentiation potential of fibroblast-like stromal cells derived from human skin. Tissue Eng Part A, 2010. 16(5): p. 1491-501.

61. Gibbs, D.A., et al., A clinical trial of fibroblast transplantation for the treatment of mucopolysaccharidoses. J Inherit Metab Dis, 1983. 6(2): p. 62-81.

62. Akle, C., et al., Transplantation of amniotic epithelial membranes in patients with mucopolysaccharidoses. Exp Clin Immunogenet, 1985. 2(1): p. 43-8.

63. Wetzels, A.M., et al., The effects of human skin fibroblast monolayers on human sperm motility and mouse zygote development. Hum Reprod, 1992. 7(6): p. 852-6.

64. Hansbrough, J.F., C. Dore, and W.B. Hansbrough, Clinical trials of a living dermal tissue replacement placed beneath meshed, split-thickness skin grafts on excised burn wounds. J Burn Care Rehabil, 1992. 13(5): p. 519-29.

65. Sabolinski, M.L., et al., Cultured skin as a 'smart material'for healing wounds: experience in venous ulcers. Biomaterials, 1996. 17(3): p. 311-20.

66. Eaglstein, W.H., et al., Acute excisional wounds treated with a tissue-engineered skin (Apligraf). Dermatol Surg, 1999. 25(3): p. 195-201.

67. Noordenbos, J., C. Dore, and J.F. Hansbrough, Safety and efficacy of TransCyte for the treatment of partial-thickness burns. J Burn Care Rehabil, 1999. 20(4): p. 275-81.

68. Chang, D.W., et al., Can a tissue-engineered skin graft improve healing of lower extremity foot wounds after revascularization? Ann Vasc Surg, 2000. 14(1): p. 44-9.

69. McGuire, M.K., et al., A pilot study to evaluate a tissue-engineered bilayered cell therapy as an alternative to tissue from the palate. J Periodontol, 2008. 79(10): p. 1847-56.

70. Kirsner, R.S., et al., Spray-applied cell therapy with human allogeneic fibroblasts and keratinocytes for the treatment of chronic venous leg ulcers: a phase 2, multicentre, double-blind, randomised, placebo-controlled trial. Lancet, 2012. 380(9846): p. 977-85.

71. Lantis, J.C., 2nd, et al., The influence of patient and wound variables on healing of venous leg ulcers in a randomized controlled trial of growth-arrested allogeneic keratinocytes and fibroblasts. J Vasc Surg, 2013. 58(2): p. 433-9.

72. Zhao, L.R., et al., Human bone marrow stem cells exhibit neural phenotypes and ameliorate neurological deficits after grafting into the ischemic brain of rats. Exp Neurol, 2002. 174(1): p. 11-20.

73. Ashjian, P.H., et al., In vitro differentiation of human processed lipoaspirate cells into early neural progenitors. Plast Reconstr Surg, 2003. 111(6): p. 1922-31.

74. Wislet-Gendebien, S., et al., Regulation of neural markers nestin and GFAP expression by cultivated bone marrow stromal cells. J Cell Sci, 2003. 116(Pt 16): p. 3295-302.

75. Kang, S.K., et al., Improvement of neurological deficits by intracerebral transplantation of human adipose tissue-derived stromal cells after cerebral ischemia in rats. Exp Neurol, 2003. 183(2): p. 355-66.

76. Jeong, J.A., et al., Rapid neural differentiation of human cord blood-derived mesenchymal stem cells. Neuroreport, 2004. 15(11): p. 1731-4.

77. Wislet-Gendebien, S., et al., Nestin-positive mesenchymal stem cells favour the astroglial lineage in neural progenitors and stem cells by releasing active BMP4. BMC Neurosci, 2004. 5: p. 33.

78. Long, X., et al., Neural cell differentiation in vitro from adult human bone marrow mesenchymal stem cells. Stem Cells Dev, 2005. 14(1): p. 65-9.

79. Alexanian, A.R., Neural stem cells induce bone-marrow-derived mesenchymal stem cells to generate neural stem-like cells via juxtacrine and paracrine interactions. Exp Cell Res, 2005. 310(2): p. 383-91.

80. Arnhold, S., et al., Human bone marrow, stroma cells display certain neural characteristics and integrate in the subventricular compartment after injection into the liquor system. Eur J Cell Biol, 2006. 85(6): p. 551-65.

81. Moviglia, G.A., et al., Autoreactive T cells induce in vitro BM mesenchymal stem cell transdifferentiation to neural stem cells. Cytotherapy, 2006. 8(3): p. 196-201.

82. Rivera, F.J., et al., Adult hippocampus derived soluble factors induce a neuronal-like phenotype in mesenchymal stem cells. Neurosci Lett, 2006. 406(1-2): p. 49-54.

83. El-Badri, N.S., et al., Cord blood mesenchymal stem cells: Potential use in neurological disorders. Stem Cells Dev, 2006. 15(4): p. 497-506.

84. Zhang, W., et al., Combination of adenoviral vector-mediated neurotrophin-3 gene transfer and retinoic acid promotes adult bone marrow cells to differentiate into neuronal phenotypes. Neurosci Lett, 2006. 408(2): p. 98-103.

85. Mareschi, K., et al., Neural differentiation of human mesenchymal stem cells: Evidence for expression of neural markers and eag K+ channel types. Exp Hematol, 2006. 34(11): p. 1563-72.

86. Kim, S., et al., Neural differentiation potential of peripheral blood- and bone-marrow-derived precursor cells. Brain Res, 2006. 1123(1): p. 27-33.

87. Kingham, P.J., et al., Adipose-derived stem cells differentiate into a Schwann cell phenotype and promote neurite outgrowth in vitro. Exp Neurol, 2007. 207(2): p. 267-74.

88. Greco, S.J., et al., An interdisciplinary approach and characterization of neuronal cells transdifferentiated from human mesenchymal stem cells. Stem Cells Dev, 2007. 16(5): p. 811-26.

89. Chu, Q., et al., Astrocytes facilitate the growth and differentiation of co-cultured mesenchymal stem cells. J Huazhong Univ Sci Technolog Med Sci, 2008. 28(3): p. 333-6.

90. Yang, Y., et al., NRSF silencing induces neuronal differentiation of human mesenchymal stem cells. Exp Cell Res, 2008. 314(11-12): p. 2257-65.

91. Yang, J., et al., Dorsal root ganglion neurons induce transdifferentiation of mesenchymal stem cells along a Schwann cell lineage. Neurosci Lett, 2008. 445(3): p. 246-51.

92. Cheng, Z., et al., Targeted induction of differentiation of human bone mesenchymal stem cells into neuron-like cells. J Huazhong Univ Sci Technolog Med Sci, 2009. 29(3): p. 296-9.

93. Zhang, S., et al., Stem cells modified by brain-derived neurotrophic factor to promote stem cells differentiation into neurons and enhance neuromotorfunction after brain injury. Chin J Traumatol, 2009. 12(4): p. 195-9.

94. Jurga, M., et al., Generation of functional neural artificial tissue from human umbilical cord blood stem cells. Tissue Eng Part C Methods, 2009. 15(3): p. 365-72.

95. Wang, L., et al., Differentiation of human bone marrow mesenchymal stem cells grown in terpolyesters of 3-hydroxyalkanoates scaffolds into nerve cells. Biomaterials, 2010. 31(7): p. 1691-8.

96. Yang, L.L., et al., Differentiation of human bone marrow-derived mesenchymal stem cells into neural-like cells by co-culture with retinal pigmented epithelial cells. Int J Ophthalmol, 2010. 3(1): p. 23-7.

97. Ding, Y., et al., Bone marrow mesenchymal stem cells and electroacupuncture downregulate the inhibitor molecules and promote the axonal regeneration in the transected spinal cord of rats. Cell Transplant, 2011. 20(4): p. 475-91.

98. Egea, V., et al., TNF-alpha respecifies human mesenchymal stem cells to a neural fate and promotes migration toward experimental glioma. Cell Death Differ, 2011. 18(5): p. 853-63.

99. Manochantr, S., et al., Isolation, characterization and neural differentiation potential of amnion derived mesenchymal stem cells. J Med Assoc Thai, 2010. 93 Suppl 7: p. S183-91.

100. Khoo, M.L., et al., Transplantation of neuronal-primed human bone marrow mesenchymal stem cells in hemiparkinsonian rodents. PLoS One, 2011. 6(5): p. e19025.

101. Liu, Z., et al., Cocaine- and amphetamine-regulated transcript promotes the differentiation of mouse bone marrow-derived mesenchymal stem cells into neural cells. BMC Neurosci, 2011. 12: p. 67.

102. Cho, H., et al., Neural differentiation of umbilical cord mesenchymal stem cells by subsonic vibration. Life Sci, 2012. 90(15-16): p. 591-9.

103. Ding, Y., et al., Electroacupuncture promotes the differentiation of transplanted bone marrow mesenchymal stem cells overexpressing TrkC into neuron-like cells in transected spinal cord of rats. Cell Transplant, 2013. 22(1): p. 65-86.

104. Qin, X., W. Han, and Z. Yu, Neuronal-like differentiation of bone marrow-derived mesenchymal stem cells induced by striatal extracts from a rat model of Parkinson's disease. Neural Regen Res, 2012. 7(34): p. 2673-80.

105. Martini, M.M., et al., Human placenta-derived mesenchymal stem cells acquire neural phenotype under the appropriate niche conditions. DNA Cell Biol, 2013. 32(2): p. 58-65.

106. Lo Furno, D., et al., Differentiation of human adipose stem cells into neural phenotype by neuroblastoma- or olfactory ensheathing cells-conditioned medium. J Cell Physiol, 2013. 228(11): p. 2109-18.

107. Singh, S.P., N.K. Tripathy, and S. Nityanand, Comparison of phenotypic markers and neural differentiation potential of multipotent adult progenitor cells and mesenchymal stem cells. World J Stem Cells, 2013. 5(2): p. 53-60.

108. Zhao, T., et al., Combined treatment with platelet-rich plasma and brain-derived neurotrophic factor-overexpressing bone marrow stromal cells supports axonal remyelination in a rat spinal cord hemisection model. Cytotherapy, 2013. 15(7): p. 792-804.

109. Xiong, N., et al., bFGFpromotes the differentiation and effectiveness of human bone marrow mesenchymal stem cells in a rotenone model for Parkinson's disease. Environ Toxicol Pharmacol, 2013. 36(2): p. 411-422.

110. Hu, W., et al., New methods for inducing the differentiation of amniotic-derived mesenchymal stem cells into motor neuron precursor cells. Tissue Cell, 2013. 45(5): p. 295-305.

111. Zhao, Y., et al., Exogenous and endogenous therapeutic effects of combination Sodium Ferulate and bone marrow stromal cells (BMSCs) treatment enhance neurogenesis after rat focal cerebral ischemia. Metab Brain Dis, 2013. 28(4): p. 655-66.

112. Jeong, S.G., et al., Valproic acid promotes neuronal differentiation by induction of neuroprogenitors in human bone-marrow mesenchymal stromal cells. Neurosci Lett, 2013. 554: p. 22-7.

113. Oh, S.H., et al., Mesenchymal Stem Cells Increase Hippocampal Neurogenesis and Neuronal Differentiation by Enhancing the Wnt Signaling Pathway in an Alzheimer's Disease Model. Cell Transplant, 2015. 24(6): p. 1097-109.

114. Berg, J., et al., Human adipose-derived mesenchymal stem cells improve motor functions and are neuroprotective in the 6-hydroxydopamine-rat model for Parkinson's disease when cultured in monolayer cultures but suppress hippocampal neurogenesis and hippocampal memory function when cultured in spheroids. Stem Cell Rev Rep, 2015. 11(1): p. 133-49.

115. Zhu, T., et al., GDNF and NT-3 induce progenitor bone mesenchymal stem cell differentiation into neurons in fetal gut culture medium. Cell Mol Neurobiol, 2015. 35(2): p. 255-64.

116. Razavi, S., et al., Effect of T3 hormone on neural differentiation of human adipose derived stem cells. Cell Biochem Funct, 2014. 32(8): p. 702-10.

117. Joe, I.S., S.G. Jeong, and G.W. Cho, Resveratrol-induced SIRT1 activation promotes neuronal differentiation of human bone marrow mesenchymal stem cells. Neurosci Lett, 2015. 584: p. 97-102.

118. Manochantr, S., et al., The expression of neurogenic markers after neuronal induction of chorion-derived mesenchymal stromal cells. Neurol Res, 2015. 37(6): p. 545-52.

119. Mu, M.W., Z.Y. Zhao, and C.G. Li, Comparative study of neural differentiation of bone marrow mesenchymal stem cells by different induction methods. Genet Mol Res, 2015. 14(4): p. 14169-76.

120. Rafieemehr, H., M. Kheirandish, and M. Soleimani, Improving the neuronal differentiation efficiency of umbilical cord blood-derived mesenchymal stem cells cultivated under appropriate conditions. Iran J Basic Med Sci, 2015. 18(11): p. 1100-6.

121. Nan, C., et al., Tetramethylpyrazine induces differentiation of human umbilical cord-derived mesenchymal stem cells into neuron-like cells in vitro. Int J Oncol, 2016. 48(6): p. 2287-94.

122. Javanmard, F., M. Azadbakht, and M. Pourmoradi, The effect of hydrostatic pressure on staurosporine-induced neural differentiation in mouse bone marrowderived mesenchymal stem cells. Bratisl Lek Listy, 2016. 117(5): p. 283-9.

123. Joe, I.S. and G.W. Cho, PDE4 Inhibition by Rolipram Promotes Neuronal Differentiation in Human Bone Marrow Mesenchymal Stem Cells. Cell Reprogram, 2016. 18(4): p. 224-9.

124. Shahbazi, A., et al., Rapid Induction of Neural Differentiation in Human Umbilical Cord Matrix Mesenchymal Stem Cells by cAMP-elevating Agents. Int J Mol Cell Med, 2016. 5(3): p. 167-177.

125. Bonilla-Porras, A.R., C. Velez-Pardo, and M. Jimenez-Del-Rio, Fast transdifferentiation of human Wharton'sjelly mesenchymal stem cells into neurospheres and nerve-like cells. J Neurosci Methods, 2017. 282: p. 52-60.

126. Zarrinpour, V., Z. Hajebrahimi, and M. Jafarinia, Expression pattern of neurotrophins and their receptors during neuronal differentiation of adipose-derived stem cells in simulated microgravity condition. Iran J Basic Med Sci, 2017. 20(2): p. 178-186.

127. Guo, L., et al., Resveratrol Induces Differentiation of Human Umbilical Cord Mesenchymal Stem Cells into Neuron-Like Cells. Stem Cells Int, 2017. 2017: p. 1651325.

128. Huang, Y., et al., Histone deacetylase inhibitor significantly improved the cloning efficiency of porcine somatic cell nuclear transfer embryos. Cell Reprogram, 2011. 13(6): p. 513-20.

129. Marquez-Curtis, L.A., et al., Migration, proliferation, and differentiation of cord blood mesenchymal stromal cells treated with histone deacetylase inhibitor valproic Acid. Stem Cells Int, 2014. 2014: p. 610495.

130. Yamato, K., Z. el-Hajjaoui, and H.P. Koeffler, Regulation of levels of IL-1 mRNA in human fibroblasts. J Cell Physiol, 1989. 139(3): p. 610-6.

131. Zucali, J.R., C. Morse, and C.A. Dinarello, The role of protein kinase C in interleukin 1 and tumor necrosis factor alpha induction of fibroblasts to produce and release granulocyte-macrophage colony-stimulating activity. Exp Hematol, 1990. 18(8): p. 888-92.

132. Hori, T., et al., Prostaglandins antagonize fibroblast proliferation stimulated by tumor necrosis factor. Biochem Biophys Res Commun, 1991. 174(2): p. 758-66.

133. Rosenberg, G.A., et al., Collagenase-induced intracerebral hemorrhage in rats. Stroke, 1990. 21(5): p. 801-7.

134. Manaenko, A., et al., Arginine-vasopressin V1a receptor inhibition improves neurologic outcomes following an intracerebral hemorrhagic brain injury. Neurochem Int, 2011. 58(4): p. 542-8.

135. Manaenko, A., et al., Effect of gap junction inhibition on intracerebral hemorrhage-induced brain injury in mice. Neurol Res, 2009. 31(2): p. 173-8.

136. Manaenko, A., et al., Geldanamycin reduced brain injury in mouse model of intracerebral hemorrhage. Acta Neurochir Suppl, 2011. 111: p. 161-5.

**Claims**

1. Exosomes derived from fibroblasts for use in a method of treating or preventing a stroke in a subject, the method comprising providing to the subject an effective amount of said exosomes derived from fibroblasts.

2. The exosomes derived from fibroblasts for use according to claim 1, wherein:

   (a) the stroke is an ischemic stroke, or hemorrhagic stroke; and/or
   (b) the fibroblasts are plastic-adherent fibroblasts; and/or
   (c) the fibroblasts are in a proliferative state; and/or
   (d) the fibroblasts are allogenic, xenogenic, or autologous fibroblasts; and/or
   (e) the fibroblasts are fibroblasts isolated from placenta, cord blood, peripheral blood, omentum, hair follicle, skin, bone marrow, adipose tissue, or Wharton's Jelly; and/or
   (f) the fibroblasts induce neuroregeneration in the subject; and/or
   (g) the fibroblasts induce immune modulation in the subject, optionally wherein the immune modulation comprises enhancing production of a cytokine associated with neuroprotection in the subject, further optionally wherein the cytokine is interleukin-10, interleukin-4, interleukin-13, interleukin-35, or TGF-$\beta$.

3. The exosomes derived from fibroblasts for use according to claim 1 or claim 2, wherein the exosomes derived from fibroblasts are provided to the subject prior to the onset of the stroke, thereby preventing the stroke.

4. The exosomes derived from fibroblasts for use according to claim 1 or claim 2, wherein:

   (a) the exosomes derived from fibroblasts are provided to the subject subsequent to the onset of the stroke, thereby treating the stroke, optionally wherein:

      (I) the exosomes derived from fibroblasts reduce production of IL-17 from microglial cells in the subject; and/or
      (II) the exosomes derived from fibroblasts reduce production of TNF-$\alpha$ from microglial cells in the subject; and/or
      (III) the exosomes derived from fibroblasts reduce neuroinflammation in the subject, further optionally wherein the neuroinflammation is reduced in an ischemic penumbra of the stroke; and/or

   (b) the method further comprises providing a TNF-$\alpha$ inhibitor to the subject, optionally wherein the TNF-$\alpha$ inhibitor is melatonin, cycloheximide, auranofin, sodium aurothiomalate, Leukotriene B4, interleukin-4, interleukin-13, polymyxin B, bile acids, interleukin-6, lactulose, oxpentifylline, mometasone, glucocorticoids, colchicine, chloroquine, FK-506, berberine, resveratrol, pterostilbene, vitamin A, vitamin C, cyclosporine, phosphodiesterase inhibitors such as vinpocetine, milrinone, CI-930, rolipram, nitroquazone, zaprinast, synthetic lipid A, amrinone, N-acetylcysteine, dithiocarbamates and metal chelators, exosurf synthetic surfactant, dehydroepiandrosterone, delta-tetrahydrocannabinol, phosphatidylserine, TCV-309, a PAF antagonist, thalidomide, a cytochrome p450 inhibitor, cytochalasin D, ketamine, TGF-beta, interleukin-10, pentoxifylline, BRL 61,063, a calcium antagonist, curcumin, kappa-selective opioid agonist U50,488H (trans-3,4-dichloro-N-methyl-N-[7-(1-pyrrolidinyl)cyclohexyl]benzene-acetamide methanesulfonate), alendronate, tetrandrine, sulfasalazine, epinephrine, BMS-182123, adenosine, E3330, nicotine, IVIG, cardiotrophin-1, KB-R7785, CGRP, ligustrazine, dexanabinol, iloprost, activated protein C, a growth hormone, spermine, FR-167653, gm-6001, estradiol, aspirin, or amiodarone; and/or
   (c) the method further comprises providing to the subject an agent capable of inhibiting responsiveness to TNF-$\alpha$, optionally wherein the agent capable of inhibiting responsiveness to TNF-$\alpha$ is ibuprofen, indomethacin, Nedocromil sodium, cromolyn (sodium cromoglycate), a spleen derived factor, pentoxifylline, NG-methyl-L-arginine, dexamethasone, chlorpromazine, activated alpha 2 macroglobulin, serum amyloid A protein, a neutrophil derived proteolytic enzyme, phentolamine, propranolol, a leukotriene inhibitor, nordihydroguaiaretic acid, genistein, butylated hydroxyanisole, CNI-1493, quercetin, gabexate mesylate, SM-12502, monoclonal nonspecific suppressor factor (MNSF), pyrrolidine dithiocarbamate (PDTC), or aprotinin; and/or
   (d) the exosomes derived from fibroblasts stimulate proliferation of neural progenitor cells in the subject, optionally wherein the neural progenitor cells are in the dentate gyrus, or subventricular zone of the subject; and/or
(e) the method further comprises providing an anti-apoptotic agent to the subject, optionally wherein:

    (I) the anti-apoptotic agent is a caspase inhibitor, further optionally wherein the caspase inhibitor is a caspase-3 inhibitor or a caspase-9 inhibitor; and/or
    (II) the anti-apoptotic agent is EGF, FGF, carbon monoxide, FEDVI peptide, or TGF-β; and/or

(f) the method further comprises providing an additional agent to the subject, wherein the additional agent is n-acetylcysteine, ascorbic acid, alpha lipoic acid, human chorionic gonadotropin, VEGF, TNF-α, retinoic acid, alpha tocopherol, interleukin-3, G-CSF, GM-CSF, leukemia inhibitory factor, placental growth factor, angiopoietin, hydrogenated water, or NGF; and/or
(g) the method further comprises providing endothelial progenitor cells to the subject, optionally wherein:

    (I) the endothelial progenitor cells are derived from the subject, or are allogenic; and/or
    (II) the endothelial progenitor cells are derived from peripheral blood, mobilized peripheral blood, bone marrow, adipose derived stromal vascular fraction, cord blood, Wharton's jelly, or placenta; and/or

(h) the method further comprises mobilizing endothelial progenitor cells in the subject, optionally wherein mobilizing the endothelial progenitor cells comprises:

    (I) administration of G-CSF to the subject; and/or
    (II) administration of GM-CSF to the subject; and/or
    (III) administration of IL-3 to the subject; and/or
    (IV) administration of TPO to the subject; and/or
    (V) administration of FLT3 ligand (FL) to the subject; and/or
    (VI) administration of G-CSF to the subject; and/or

(i) the method further comprises providing a regenerative cell to the subject, optionally wherein the regenerative cell is a stem cell, further optionally wherein the stem cell is a hematopoietic stem cell, further optionally wherein:

    (I) the hematopoietic stem cell expresses CD34, CD133, or c-kit; and/or
    (II) the hematopoietic stem cell does not express one or more of CD38 and thrombopoietin receptor; and/or
    (III) the hematopoietic stem cell is an autologous, allogenic, or xenogenic hematopoietic stem cell; and/or
    (IV) the hematopoietic stem cell is derived from adipose, bone marrow, peripheral blood, mobilized peripheral blood, or cord blood.

5. The exosomes derived from fibroblasts for use according to any of claims 1-4, further comprising providing to the subject mesenchymal stem cells.

6. The exosomes derived from fibroblasts for use according to claim 5, wherein:

    (a) the mesenchymal stem cell expresses CD90, CD105, or CD73; and/or
    (b) the mesenchymal stem cell does not express one or more of HLA, CD34, or CD14; and/or
    (c) the mesenchymal stem cell is plastic adherent; and/or
    (d) the mesenchymal stem cell is allogenic, or autologous to the subject; and/or
    (e) the mesenchymal stem cell is derived from adipose, bone marrow, peripheral blood, mobilized peripheral blood, menstrual blood, fallopian tube, or cord blood.

7. The exosomes derived from fibroblasts for use according to any of claims 1-6, further comprising providing to the subject an effective amount of exosomes derived from one or more stem cells, wherein the one or more stem cells comprise hematopoietic stem cells, mesenchymal stem cells, or a combination thereof.

8. The exosomes derived from fibroblasts for use according to claim 7, wherein:

    (a) the exosomes are derived from the one or more stem cells via:

        (I) ultracentrifugation and/or wherein the exosomes are derived from the fibroblasts; or
        (II) chromatography; or
        (III) affinity purification; and/or

    (b) an outer surface of the exosomes comprises phosphatidylserine, CD9, CD19, or a tetraspanin protein.

9. The exosomes derived from fibroblasts for use according to any of claims 7-8, further comprising stimulating the one or more stem cells to secrete the exosomes.

10. The exosomes derived from fibroblasts for use according to claim 9, wherein the stimulating comprises culturing the one or more stem cells in hypoxic conditions.

11. The exosomes derived from fibroblasts for use according to claim 10, wherein the hypoxic conditions comprise between 0.01% and 10% oxygen, optionally wherein the hypoxic conditions comprise 3% oxygen.

12. The exosomes derived from fibroblasts for use according to claim 10 or claim 11, wherein the one or more stem cells are cultured in the hypoxic conditions for less than 14 days, optionally wherein the one or more stem cells are cultured in the hypoxic conditions for about 4 days.

13. The exosomes derived from fibroblasts for use according to any of claims 1-12, wherein:

(a) the method further comprises culturing the fibroblasts with metformin prior to providing the exosomes derived from fibroblasts to the subject; and/or
(b) the exosomes derived from fibroblasts stimulate production of an angiogenic cytokine in the subject, optionally wherein the angiogenic cytokine is VEGF, FGF-1, FGF-2, or IGF-1; and/or
(c) the exosomes derived from fibroblasts stimulate production of a neurogenic cytokine in the subject, optionally wherein the neurogenic cytokine is BDNF, NGF, or CNTF; and/or
(d) the method further comprises transfecting the fibroblasts with one or more angiogenic genes prior to providing the exosomes derived from fibroblasts to the subject, optionally wherein the one or more angiogenic genes comprise activin A, adrenomedullin, aFGF, ALK1, ALK5, ANF, angiogenin, angiopoietin-1, angiopoietin-2, angiopoietin-3, angiopoietin-4, bFGF, B61, bFGF inducing activity, cadherins, CAM-RF, cGMP analogs, ChDI, CLAF, claudins, collagen, connexins, Cox-2, ECDGF (endothelial cell-derived growth factor), ECG, ECI, EDM, EGF, EMAP, endoglin, endothelins, endostatin, endothelial cell growth inhibitor, endothelial cell-viability maintaining factor, endothelial differentiation sphingolipid G-protein coupled receptor-1 (EDG1), ephrins, Epo, HGF, TGF-beta, PD-ECGF, PDGF, IGF, IL8, growth hormone, fibrin fragment E, FGF-5, fibronectin, fibronectin receptor, Factor X, HB-EGF, HBNF, HGF, HUAF, heart derived inhibitor of vascular cell proliferation, IL1, IGF-2 IFN-gamma, $\alpha 1\beta 1$ integrin, $\alpha 2\beta 1$ integrin, K-FGF, LIF, leiomyoma-derived growth factor, MCP-1, macrophage-derived growth factor, monocyte-derived growth factor, MD-ECI, MECIF, MMP2, MMP3, MMP9, urokiase plasminogen activator, neuropilin, neurothelin, nitric oxide donors, nitric oxide synthases (NOSs), notch, occludins, zona occludins, oncostatin M, PDGF, PDGF-B, PDGF receptors, PDGFR-$\beta$, PD-ECGF, PAI-2, PD-ECGF, PF4, P1GF, PKR1, PKR2, PPAR-gamma, PPAR-gamma ligands, phosphodiesterase, prolactin, prostacyclin, protein S, smooth muscle cell-derived growth factor, smooth muscle cell-derived migration factor, sphingosine-1-phosphate-1 (SIP1), Syk, SLP76, tachykinins, TGF-beta, Tie 1, Tie2, TGF-$\beta$, TGF-$\beta$ receptors, TIMPs, TNF-$\alpha$, transferrin, thrombospondin, urokinase, VEGF-A, VEGF-B, VEGF-C, VEGF-D, VEGF-E, VEGF, VEGF(164), VEGI, EG-VEGF, or a combination thereof; and/or (e) the method comprises providing to the subject exosomes derived from fibroblasts, optionally wherein the exosomes are derived after culturing the fibroblasts in serum-free media.

## Patentansprüche

1. Aus Fibroblasten abgeleitete Exosome für die Verwendung in einem Verfahren zum Behandeln oder Vorbeugen eines Schlaganfalls bei einem Subjekt, wobei das Verfahren das Bereitstellen einer wirksamen Menge der aus Fibroblasten abgeleiteten Exosome an das Subjekt umfasst.

2. Aus Fibroblasten abgeleitete Exosome für die Verwendung nach Anspruch 1, wobei:

(a) der Schlaganfall ein ischämischer Schlaganfall oder ein hämorrhagischer Schlaganfall ist; und/oder
(b) die Fibroblasten plastisch-adhärente Fibroblasten sind; und/oder
(c) die Fibroblasten sich in einem proliferativen Zustand befinden; und/oder
(d) die Fibroblasten allogene, xenogene oder autologe Fibroblasten sind; und/oder
(e) die Fibroblasten Fibroblasten sind, die aus Plazenta, Nabelschnurblut, peripherem Blut, Omentum, Haarfollikel, Haut, Knochenmark, Fettgewebe oder Wharton-Sulze isoliert wurden; und/oder
(f) die Fibroblasten eine Neuroregeneration in dem Subjekt induzieren; und/oder

(g) die Fibroblasten eine Immunmodulation in dem Subjekt induzieren, wobei die Immunmodulation optional das Verbessern der Produktion eines der Neuroprotektion in dem Subjekt zugeordneten Zytokins umfasst, wobei das Zytokin optional Interleukin-10, Interleukin-4, Interleukin-13, Interleukin-35 oder TGF-β ist.

3. Aus Fibroblasten abgeleitete Exosome für die Verwendung nach Anspruch 1 oder 2, wobei die aus Fibroblasten abgeleiteten Exosome dem Subjekt vor dem Auftreten des Schlaganfalls bereitgestellt werden, wodurch der Schlaganfall verhindert wird.

4. Aus Fibroblasten abgeleitete Exosome für die Verwendung nach Anspruch 1 oder 2, wobei:

(a) die aus Fibroblasten abgeleiteten Exosome dem Subjekt nach dem Auftreten des Schlaganfalls bereitgestellt werden, wodurch der Schlaganfall behandelt wird, wobei optional:

(I) die aus Fibroblasten abgeleiteten Exosome die Produktion von IL-17 aus Mikrogliazellen in dem Subjekt reduzieren; und/oder

(II) die aus Fibroblasten abgeleiteten Exosome die Produktion von TNF-α aus Mikrogliazellen in dem Subjekt reduzieren; und/oder

(III) die aus Fibroblasten abgeleiteten Exosome die Neuroinflammation bei dem Subjekt reduzieren, ferner optional die Neuroinflammation in einer ischämischen Penumbra des Schlaganfalls reduziert wird; und/oder

(b) das Verfahren ferner das Bereitstellen eines TNF-α-Inhibitors an das Subjekt umfasst, wobei optional der TNF-α-Inhibitor Melatonin, Cycloheximid, Auranofin, Natriumaurothiomalat, Leukotrien B4, Interleukin-4, Interleukin-13, Polymyxin B, Gallensäuren, Interleukin-6, Lactulose, Oxpentifyllin, Mometason, Glukokortikoide, Colchicin, Chloroquin, FK-506, Berberin, Resveratrol, Pterostilben, Vitamin A, Vitamin C, Cyclosporin, Phosphodiesterase-Inhibitoren wie Vinpocetin, Milrinon, CI-930, Rolipram, Nitroquazon, Zaprinast, synthetisches Lipid A, Amrinon, N-Acetylcystein, Dithiocarbamate und Metallchelatoren, synthetisches Exosurf-Tensid, Dehydroepiandrosteron, Delta-Tetrahydrocannabinol, Phosphatidylserin, TCV-309, ein PAF-Antagonist, Thalidomid, ein Cytochrom-P450-Inhibitor, Cytochalasin D, Ketamin, TGF-beta, Interleukin-10, Pentoxifyllin, BRL 61,063, ein Calciumantagonist, Curcumin, der kappa-selektive Opioidagonist U50,488H (trans-3,4-Dichlor-N-methyl-N-[7-(1-pyrrolidinyl)cyclohexyl]benzolacetamidmethansulfonat), Alendronat, Tetrandrin, Sulfasalazin, Epinephrin, BMS-182123, Adenosin, E3330, Nikotin, IVIG, Cardiotrophin-1, KB-R7785, CGRP, Ligustrazin, Dexanabinol, Iloprost, aktiviertes Protein C, ein Wachstumshormon, Spermin, FR-167653, gm-6001, Estradiol, Aspirin oder Amiodaron ist; und/oder

(c) das Verfahren ferner das Bereitstellen eines Mittels an das Subjekt umfasst, das in der Lage ist, die Reaktionsfähigkeit auf TNF-α zu hemmen, optional wobei das Mittel, das in der Lage ist, die Reaktionsfähigkeit auf TNF-α zu hemmen, Ibuprofen, Indomethacin, Nedocromilnatrium, Cromolyn (Natriumcromoglycat), ein aus der Milz abgeleiteter Faktor, Pentoxifyllin, NG-Methyl-L-Arginin, Dexamethason, Chlorpromazin, aktiviertes Alpha-2-Makroglobulin, Serumamyloid-A-Protein, ein aus Neutrophilen abgeleitetes proteolytisches Enzym, Phentolamin, Propranolol, ein Leukotrien-Inhibitor, Nordihydroguaiaretsäure, Genistein, butyliertes Hydroxyanisol, CNI-1493, Quercetin, Gabexatmesylat, SM-12502, monoklonaler unspezifischer Suppressor-Faktor (MNSF), Pyrrolidindithiocarbamat (PDTC) oder Rotinin ist; und/oder

(d) die aus Fibroblasten abgeleiteten Exosome die Proliferation neuraler Vorläuferzellen in dem Subjekt stimulieren, wobei sich optional die neuralen Vorläuferzellen in dem Gyrus dentatus oder in der subventrikulären Zone des Subjekts befinden; und/oder

(e) das Verfahren ferner das Bereitstellen eines anti-apoptotischen Mittels an das Subjekt umfasst, wobei optional:

(I) das anti-apoptotische Mittel ein Caspase-Inhibitor ist, wobei ferner optional das anti-apoptotische Mittel ein Caspase-3-Inhibitor oder ein Caspase-9-Inhibitor ist; und/oder

(II) das anti-apoptotische Mittel EGF, FGF, Kohlenmonoxid, FEDVI-Peptid oder TGF-β ist; und/oder

(f) das Verfahren das Bereitstellen eines zusätzlichen Mittels an das Subjekt umfasst, wobei das zusätzliche Mittel N-Acetylcystein, Ascorbinsäure, Alpha-Liponsäure, humanes Choriongonadotropin, VEGF, TNF-α, Retinsäure, Alpha-Tocopherol, Interleukin-3, G-CSF, GM-CSF, Leukämie-hemmender Faktor, Plazenta-Wachstumsfaktor, Angiopoietin, hydriertes Wasser oder NGF ist; und/oder

(g) das Verfahren ferner das Bereitstellen von Endothelvorläuferzellen an das Subjekt umfasst, wobei optional:

(I) die endothelialen Vorläuferzellen von dem Subjekt abgeleitet oder allogen sind; und/oder

(II) die endothelialen Vorläuferzellen aus peripherem Blut, mobilisiertem peripherem Blut, Knochenmark, aus Fettgewebe abgeleiteter stromaler vaskulärer Fraktion, Nabelschnurblut, Wharton-Sulze oder Plazenta abgeleitet sind; und/oder

(h) das Verfahren ferner das Mobilisieren von endothelialen Vorläuferzellen in dem Subjekt umfasst, wobei optional das Mobilisieren der endothelialen Vorläuferzellen umfasst:

(I) die Verabreichung von G-CSF an das Subjekt; und/oder
(II) die Verabreichung von GM-CSF an das Subjekt; und/oder
(III) die Verabreichung von IL-3 an das Subjekt; und/oder
(IV) die Verabreichung von TPO an das Subjekt; und/oder
(V) Verabreichung von FLT3-Ligand (FL) an das Subjekt; und/oder
(VI) die Verabreichung von G-CSF an das Subjekt; und/oder

(i) das Verfahren ferner das Bereitstellen einer regenerativen Zelle an das Subjekt umfasst, wobei optional die regenerative Zelle eine Stammzelle ist, wobei ferner optional die Stammzelle eine hämatopoetische Stammzelle ist, wobei ferner optional:

(I) die hämatopoetische Stammzelle CD34, CD133 oder c-Kit exprimiert; und/oder
(II) die hämatopoetische Stammzelle eines oder mehrere von CD38 und Thrombopoietinrezeptor nicht exprimiert; und/oder
(III) die hämatopoetische Stammzelle eine autologe, allogene oder xenogene hämatopoetische Stammzelle ist; und/oder
(IV) die hämatopoetische Stammzelle aus Fettgewebe, Knochenmark, peripherem Blut, mobilisiertem peripherem Blut oder Nabelschnurblut abgeleitet ist.

5. Aus Fibroblasten abgeleitete Exosome für die Verwendung nach einem der Ansprüche 1-4, ferner umfassend das Bereitstellen von mesenchymalen Stammzellen an das Subjekt.

6. Aus Fibroblasten abgeleitete Exosome für die Verwendung nach Anspruch 5, wobei:

(a) die mesenchymale Stammzelle CD90, CD105 oder CD73 exprimiert; und/oder
(b) die mesenchymale Stammzelle eines oder mehrere der Moleküle HLA, CD34 oder CD14 nicht exprimiert; und/oder
(c) die mesenchymale Stammzelle plastisch-adhärent ist; und/oder
(d) die mesenchymale Stammzelle allogen oder autolog zu dem Subjekt ist; und/oder
(e) die mesenchymale Stammzelle aus Fettgewebe, Knochenmark, peripherem Blut, mobilisiertem peripherem Blut, Menstruationsblut, Eileiter oder Nabelschnurblut abgeleitet wird.

7. Aus Fibroblasten abgeleitete Exosome für die Verwendung nach einem der Ansprüche 1-6, ferner umfassend das Bereitstellen einer wirksamen Menge an Exosomen, die aus einer oder mehreren Stammzellen abgeleitet werden, an das Subjekt, wobei die eine oder die mehreren Stammzellen hämatopoetische Stammzellen, mesenchymale Stammzellen oder eine Kombination davon umfassen.

8. Aus Fibroblasten abgeleitete Exosome für die Verwendung nach Anspruch 7, wobei:

(a) die Exosome aus der einen oder den mehreren Stammzellen abgeleitet werden durch:

(I) Ultrazentrifugation und/oder wobei die Exosome aus den Fibroblasten abgeleitet werden; oder
(II) Chromatographie; oder
(III) Affinitätsreinigung; und/oder

(b) eine Oberfläche der Exosome Phosphatidylserin, CD9, CD19 oder ein Tetraspanin-Protein umfasst.

9. Aus Fibroblasten abgeleitete Exosome für die Verwendung nach einem der Ansprüche 7-8, ferner umfassend das Stimulieren der einen oder der mehreren Stammzellen, um die Exosome zu sekretieren.

10. Aus Fibroblasten abgeleitete Exosome für die Verwendung nach Anspruch 9, wobei das Stimulieren das Kultivieren der einen oder der mehreren Stammzellen unter hypoxischen Bedingungen umfasst.

11. Aus Fibroblasten abgeleitete Exosome für die Verwendung nach Anspruch 10, wobei die hypoxischen Bedingungen zwischen 0,01 % und 10 % Sauerstoff umfassen, optional wobei die hypoxischen Bedingungen 3 % Sauerstoff umfassen.

12. Aus Fibroblasten abgeleitete Exosome für die Verwendung nach Anspruch 10 oder 11, wobei die eine oder die mehreren Stammzellen weniger als 14 Tage unter hypoxischen Bedingungen kultiviert werden, optional wobei die eine oder die mehreren Stammzellen etwa 4 Tage unter hypoxischen Bedingungen kultiviert werden.

13. Aus Fibroblasten abgeleitete Exosome für die Verwendung nach einem der Ansprüche 1-12, wobei:

(a) das Verfahren ferner das Kultivieren der Fibroblasten mit Metformin vor dem Bereitstellen der aus Fibroblasten abgeleiteten Exosome an das Subjekt umfasst; und/oder
(b) die aus Fibroblasten abgeleiteten Exosome die Produktion eines angiogenen Zytokins in dem Subjekt stimulieren, wobei optional das angiogene Zytokin VEGF, FGF-1, FGF-2 oder IGF-1 ist; und/oder
(c) die aus Fibroblasten abgeleiteten Exosome die Produktion eines neurogenen Zytokins in dem Subjekt stimulieren, wobei das neurogene Zytokin optional BDNF, NGF oder CNTF ist; und/oder
(d) das Verfahren ferner das Transfizieren der Fibroblasten mit einem oder mehreren angiogenen Genen umfasst, bevor die von Fibroblasten abgeleiteten Exosome dem Subjekt bereitgestellt werden, wobei optional das eine oder die mehreren angiogenen Gene Activin A, Adrenomedullin, aFGF, ALK1, ALK5, ANF, Angiogenin, Angiopoietin-1, Angiopoietin-2, Angiopoietin-3, Angiopoietin-4, bFGF, B61, bFGF-induzierende Aktivität, Cadherine, CAM-RF, cGMP-Analoga, ChDI, CLAF, Claudine, Kollagen, Connexine, Cox-2, ECDGF (Endothelzell-abgeleiteten Wachstumsfaktor), ECG, ECI, EDM, EGF, EMAP, Endoglin, Endotheline, Endostatin, Endothelzellwachstuminhibitor, Faktor für die Aufrechterhaltung der Endothelzellviabilität, G-Protein-gekoppelten Endothel-Differenzierungssphingolipid-Rezeptor-1 (EDG1), Ephrine, Epo, HGF, TGF-beta, PD-ECGF, PDGF, IGF, IL8, Wachstumshormon, Fibrin-Fragment E, FGF-5, Fibronectin, Fibronectin-Rezeptor, Faktor X, HB-EGF, HBNF, HGF, HUAF, Herz-abgeleiteten Inhibitor der vaskulären Zellproliferation, IL1, IGF-2 IFN-gamma, $\alpha1\beta1$-Integrin, $\alpha2\beta1$-Integrin, K-FGF, LIF, Leiomyom-abgeleiteten Wachstumsfaktor, MCP-1, Makrophagen-abgeleiteten Wachstumsfaktor, Monozyten-abgeleiteten Wachstumsfaktor, MD-ECI, MECIF, MMP2, MMP3, MMP9, Urokinase-Plasminogenaktivator, Neuropilin, Neurothelin, Stickstoffmonoxidspender, Stickstoffmonoxidsynthasen (NOSs), Notch, Occludine, Zona-Occludine, Oncostatin M, PDGF, PDGF-B, PDGF-Rezeptoren, PDGFR-$\beta$, PD-ECGF, PAI-2, PD-ECGF, PF4, P1GF, PKR1, PKR2, PPAR-gamma, PPAR-Gamma-Liganden, Phosphodiesterase, Prolaktin, Prostacyclin, Protein S, aus glatten Muskelzellen abgeleiteten Wachstumsfaktor, aus glatten Muskelzellen abgeleiteten Migrationsfaktor, Sphingosin-1-Phosphat-1 (SIP1), Syk, SLP76, Tachykinine, TGF-beta, Tie 1, Tie2, TGF-$\beta$, TGF-$\beta$-Rezeptoren, TIMPs, TNF-$\alpha$, Transferrin, Thrombospondin, Urokinase, VEGF-A, VEGF-B, VEGF-C, VEGF-D, VEGF-E, VEGF, VEGF(164), VEGI, EG-VEGF oder eine Kombination davon umfassen; und/oder
(e) das Verfahren das Bereitstellen von aus Fibroblasten abgeleiteten Exosomen an das Subjekt umfasst, wobei die Exosome optional nach dem Kultivieren der Fibroblasten in serumfreien Medien abgeleitet werden.

**Revendications**

1. Exosomes dérivés de fibroblastes pour utilisation dans une méthode de traitement ou de prévention d'un accident vasculaire cérébral chez un sujet, la méthode comprenant l'administration d'une quantité efficace desdits exosomes dérivés de fibroblastes au sujet.

2. Exosomes dérivés de fibroblastes pour utilisation selon la revendication 1, étant entendu que

(a) l'accident vasculaire cérébral est un accident vasculaire cérébral ischémique ou hémorragique ; et/ou
(b) les fibroblastes sont des fibroblastes aptes à adhérer au plastique ; et/ou
(c) les fibroblastes sont dans un état prolifératif ; et/ou
(d) les fibroblastes sont des fibroblastes allogéniques, xénogéniques ou autologues ; et/ou
(e) les fibroblastes sont des fibroblastes isolés du placenta, du sang de cordon, du sang périphérique, de l'omentum, du follicule pileux, de la peau, de la moelle osseuse, du tissu adipeux ou de la gelée de Wharton ; et/ou
(f) les fibroblastes provoquent une neurodégénérescence chez le sujet ; et/ou
(g) les fibroblastes provoquent une modulation immunitaire chez le sujet, ladite modulation immunitaire comprenant éventuellement l'amélioration de la production d'une cytokine associée à la neuroprotection chez le sujet, ladite cytokine étant en outre éventuellement une interleukine-10, interleukine-4, interleukine-13, interleukine-35 ou TGF-$\beta$.

3. Exosomes dérivés de fibroblastes pour utilisation selon la revendication 1 ou 2, lesdits exosomes dérivés de fibroblastes étant administrés au sujet avant la survenue de l'accident vasculaire cérébral, prévenant ainsi l'accident vasculaire cérébral.

4. Exosomes dérivés de fibroblastes pour utilisation selon la revendication 1 ou 2, étant entendu que :

(a) les exosomes dérivés de fibroblastes sont administrés au sujet après la survenue de l'accident vasculaire cérébral, traitant ainsi l'accident vasculaire cérébral, lesdits exosomes dérivés de fibroblastes :

(I) réduisant éventuellement la production d'IL-17 par les cellules microgliales chez le sujet, et/ou

(II) réduisant éventuellement la production de TNF-α par les cellules microgliales chez le sujet, et/ou

(III) réduisant éventuellement une neuroinflammation chez le sujet, ladite neuroinflammation étant en outre éventuellement réduite dans une pénombre ischémique de l'accident vasculaire cérébral ; et/ou

(b) la méthode comprend en outre l'administration d'un inhibiteur du TNF-α chez le sujet, ledit inhibiteur du TNF-α étant éventuellement la mélatonine, le cycloheximide, l'auranofine, l'aurothiomalate de sodium, le leucotriène B4, l'interleukine-4, l'interleukine-13, la polymyxine B, les acides biliaires, l'interleukine-6, le lactulose, l'oxpentifylline, la mométasone, les glucocorticoïdes, la colchicine, la chloroquine, le FK-506, la berbérine, le resvératrol, le ptérostilbène, la vitamine A, la vitamine C, la cyclosporine, les inhibiteurs de la phosphodiestérase tels que la vinpocétine, la milrinone, le CI-930, le rolipram, la nitroquazone, le zaprinast, le lipide synthétique A, l'amrinone, la N-acétylcystéine, les dithiocarbamates et les chélateurs de métaux, le tensioactif synthétique Exosurf, la déshydroépiandrostérone, le delta-tétrahydrocannabinol, la phosphatidylsérine, le TCV-309, un antagoniste du PAF, le thalidomide, un inhibiteur du cytochrome p450, la cytochalasine D, la kétamine, le TGF-bêta, l'interleukine-10, la pentoxifylline, le BRL 61,063, un antagoniste du calcium, la curcumine, un agoniste opioïde à sélectivité kappa U50,488H (méthanesulfonate de trans-3,4,-dichloro-N-méthyl-N-[7-(1-pyrrolidinyl)cyclohexyl]benzène-acétamide), l'alendronate, la tétrandrine, la sulfasalazine, l'épinéphrine, le BMS-182123, l'adénosine, l'E3330, la nicotine, l'IVIG, la cardiotrophine-1, le KB-R7785, la CGRP, la ligstrazine, le dexanabinol, l'iloprost, la protéine C activée, une hormone de croissance, la spermine, le FR-167653, le gm-6001, l'estradiol, l'aspirine ou l'amiodarone ;

et/ou

(c) la méthode comprend en outre l'administration au sujet d'un agent capable d'inhiber la réactivité au TNF-α, ledit agent capable d'inhiber la réactivité au TNF-α étant éventuellement l'ibuprofène, l'indométhacine, le nédocromil sodique, la cromolyne (cromoglycate de sodium), un facteur dérivé de la rate, la pentoxifylline, la NG-méthyl-L-arginine, la dexaméthasone, la chlorpromazine, l'alpha-2-macroglobuline activée, la protéine sérique amyloïde A, une enzyme protéolytique dérivée des neutrophiles, la phentolamine, le propanolol, un inhibiteur du leucotriène, l'acide nordihydroguaïarétique, la génistéine, l'hydroxyanisole butylé, le CNI-1493, la quercétine, le mésylate de gabexate, le SM-12502, le facteur de suppression monoclonal non spécifique (MNSF), le dithiocarbonate de pyrrolidine (PDTC) ou l'aprotinine ; et/ou

(d) les exosomes dérivés des fibroblastes stimulent la prolifération des cellules progénitrices neuronales chez le sujet, lesdites cellules progénitrices neuronales étant éventuellement dans le gyrus denté ou une zone sous-ventriculaire du sujet ; et/ou

(e) la méthode comprend en outre l'administration d'un agent anti-apoptotique au sujet ; ledit agent anti-apoptotique étant éventuellement :

(I) un inhibiteur de la caspase, ledit inhibiteur de la caspase étant éventuellement en outre un inhibiteur de la caspase-3 ou un inhibiteur de la caspase-9, et/ou

(II) un EGF, FGF, monoxyde de carbone, peptide FEDVI, ou TGF-β ; et/ou

(f) la méthode comprend en outre l'administration d'un agent supplémentaire au sujet, ledit agent supplémentaire étant une n-acétylcystéine, l'acide ascorbique, l'acide alpha-lipoïque, la gonadotrophine chorionique humaine, le VEGF, le TNF-α, l'acide rétinoïque, l'alpha-tocophérol, l'interleukine-3, le G-CSF, le GM-CSF, le facteur inhibiteur de la leucémie, le facteur de croissance placentaire, l'angiopoïétine, l'eau hydrogénée ou le NGF ; et/ou

(g) la méthode comprend en outre l'administration de cellules progénitrices endothéliales au sujet, lesdites cellules progénitrices endothéliales étant éventuellement :

(I) dérivées du sujet ou allogéniques, et/ou

(II) dérivées du sang périphérique, du sang périphérique mobilisé, de la moelle osseuse, de la fraction vasculaire stromale dérivée des tissus adipeux, du sang de cordon, de la gelée de Wharton ou du placenta ; et/ou

(h) la méthode comprend en outre la mobilisation des cellules progénitrices endothéliales chez le sujet, ladite mobilisation des cellules progénitrices endothéliales comprenant éventuellement :

    (I) l'administration de G-CSF au sujet, et/ou
    (II) l'administration de GM-CSF au sujet, et/ou
    (III) l'administration d'IL-3 au sujet, et/ou
    (IV) l'administration de TPO au sujet, et/ou
    (V) l'administration de FLT3-ligand (FL) au sujet, et/ou
    (VI) l'administration de G-CSF au sujet ; et/ou

(i) la méthode comprend en outre l'administration d'une cellule régénératrice au sujet, ladite cellule régénératrice étant en outre éventuellement une cellule souche, ladite cellule souche étant en outre éventuellement une cellule souche hématopoïétique, ladite cellule souche hématopoïétique :

    (I) exprimant en outre éventuellement CD34, CD133 ou c-kit, et/ou
    (II) n'exprimant en outre éventuellement pas CD38 et/ou un récepteur de la thrombopoïétine, et/ou
    (III) étant en outre éventuellement une cellule souche hématopoïétique autologue, allogénique ou xénogénique, et/ou
    (IV) étant en outre éventuellement dérivée du tissu adipeux, de la moelle osseuse, du sang périphérique, du sang périphérique mobilisé ou du sang de cordon.

5. Exosomes dérivés de fibroblastes pour utilisation selon l'une quelconque des revendications 1 à 4, comprenant en outre l'administration de cellules souches mésenchymateuses au sujet.

6. Exosomes dérivés de fibroblastes pour utilisation selon la revendication 5, étant entendu que :

    (a) la cellule souche mésenchymateuse exprime CD90, CD105 ou CD73 ; et/ou
    (b) la cellule souche mésenchymateuse n'exprime pas HLA, CD34 et/ou CD14 ; et/ou
    (c) la cellule souche mésenchymateuse adhère au plastique ; et/ou
    (d) la cellule souche mésenchymateuse est allogénique ou autologue au sujet ; et/ou
    (e) la cellule souche mésenchymateuse est dérivée du tissu adipeux, de la moelle osseuse, du sang périphérique, du sang périphérique mobilisé, du sang menstruel, de la trompe de Fallope ou du sang de cordon.

7. Exosomes dérivés de fibroblastes pour utilisation selon l'une quelconque des revendications 1 à 6, comprenant en outre l'administration au sujet d'une quantité efficace d'exosomes dérivés d'une ou plusieurs cellules souches, la ou lesdites cellules souches comprenant des cellules souches hématopoïétiques, des cellules souches mésenchymateuses ou une combinaison de celles-ci.

8. Exosomes dérivés de fibroblastes pour utilisation selon la revendication 7, étant entendu que :

    (a) les exosomes sont dérivés d'une ou plusieurs cellules souches par :

        (I) ultracentrifugation et/ou lesdits exosomes sont dérivés des fibroblastes, ou
        (II) une chromatographie, ou
        (III) une purification par affinité ; et/ou

    (b) une surface extérieure des exosomes comprend la phosphatidylsérine, CD9, CD19 ou la protéine tétraspanine.

9. Exosomes dérivés de fibroblastes pour utilisation selon l'une quelconque des revendications 7 et 8, comprenant en outre la stimulation de la ou desdites cellules souches de façon qu'elles sécrètent les exosomes.

10. Exosomes dérivés de fibroblastes pour utilisation selon la revendication 9, étant entendu que la stimulation comprend la mise en culture de la ou desdites cellules souches dans des conditions hypoxiques.

11. Exosomes dérivés de fibroblastes pour utilisation selon la revendication 10, étant entendu que les conditions hypoxiques comprennent entre 0,01 % et 10 % d'oxygène, lesdites conditions hypoxiques comprenant éventuellement 3 % d'oxygène.

12. Exosomes dérivés de fibroblastes pour utilisation selon la revendication 10 ou 11, dans lesquels la ou les cellules souches sont mises en culture dans lesdites conditions hypoxiques pendant moins de 14 jours, ladite ou lesdites cellules souches étant éventuellement mises en culture dans lesdites conditions hypoxiques pendant environ 4 jours.

13. Exosomes dérivés de fibroblastes pour utilisation selon l'une quelconque des revendications 1 à 12, étant entendu que

    (a) la méthode comprend en outre la mise en culture des fibroblastes avec de la metformine avant l'administration des exosomes dérivés de fibroblastes au sujet ; et/ou

(b) les exosomes dérivés de fibroblastes stimulent la production d'une cytokine angiogénique chez le sujet, ladite cytokine angiogénique étant éventuellement un VEGF, FGF-1, FGF-2 ou IGF-1 ; et/ou

(c) les exosomes dérivés de fibroblastes stimulent la production d'une cytokine neurogénique chez le sujet, ladite cytokine neurogénique étant éventuellement un BDNF, NGF ou CNTF ; et/ou

(d) la méthode comprend en outre la transfection des fibroblastes avec un ou plusieurs gènes angiogéniques avant l'administration des exosomes dérivés de fibroblastes au sujet, le ou lesdits gènes angiogéniques comprenant éventuellement l'activine A, l'adrénomédulline, l'aFGF, l'ALK1, l'ALK5, l'ANF, l'angiogénine, l'angiopoïétine-1, l'angiopoïétine-2, l'angiopoïétine-3, l'angiopoïétine-4, le bFGF, B61, bFGF, activité induisant le bFGF, les cadhérines, CAM-RF, les analogues du cGMP, ChDI, CLAF, les claudines, le collagène, les connexines, Cox-2, l'ECDGF (facteur de croissance dérivé des cellules endothéliales), ECG, ECI, EDM, EGF, EMAP, l'endogline, les endothélines, l'endostatine, l'inhibiteur de croissance des cellules endothéliales, le facteur de maintien de la viabilité des cellules endothéliales, le récepteur 1 couplé à une protéine G sphingolipide de différenciation endothéliale (EDG1), les éphrines, l'Epo, le HGF, le TGF-bêta, le PD-ECGF, le PDGF, l'IGF, l'IL8, l'hormone de croissance, le fragment E de la fibrine, le FGF-5, la fibronectine, le récepteur de la fibronectine, le facteur X, le HB-EGF, le HBNF, le HGF, le HUAF, l'inhibiteur de la prolifération des cellules vasculaires dérivé du cœur, l'IL1, l'IGF-2 IFN-gamma, l'intégrine $\alpha1\beta1$, l'intégrine $\alpha2\beta2$, le K-FGF, le LIF, le facteur de croissance dérivé du léiomyome, la MCP-1, le facteur de croissance dérivé des macrophages, le facteur de croissance dérivé des monocytes, MD-ECI, MECIF, MMP2, MMP3, MMP9, l'urokinase plasminogène activateur, la neuropiline, la neurothéline, les donneurs d'oxyde nitrique, les synthases de l'oxyde nitrique (NOS), Notch, les occludines, les occludines du zona, l'oncostatine M, le PDGF, le PDGF-B, les récepteurs du PDGF, le PDGFR-$\beta$, le PD-ECGF, le PAI-2, le PD-ECGF, le PF4, le P1GF, le PKR1, le PKR2, le PPAR-gamma, les ligands de PPAR-gamma, la phosphodiestérase, la prolactine, la prostacyline, la protéine S, le facteur de croissance dérivé des cellules musculaires lisses, le facteur de migration dérivé des cellules musculaires lisses, le sphingosine-1-phosphate-1 (SIP1), Syk, SLP76, les tachykinines, le TGF-bêta, Tie1, Tie2, le TGF-$\beta$, les récepteurs du TGF-$\beta$, le TIMP, le TNF-$\alpha$, la transferrine, la thrombospondine, l'urokinase, le VEGF-A, le VEGF-B, le VEGF-C, le VEGF-D, le VEGF-E, le VEGF, le VEGF(164), le VEGI, l'EG-VEGF ou une combinaison de ceux-ci ; et/ou

(e) la méthode comprend l'administration au sujet d'exosomes dérivés de fibroblastes, lesdits exosomes étant éventuellement dérivés après la mise en culture des fibroblastes dans un milieu sans sérum.

FIG. 1

FIG. 2

FIG. 3

FIG. 4

FIG. 5

FIG. 6

FIG. 7

FIG. 8

FIG. 9

## REFERENCES CITED IN THE DESCRIPTION

**Patent documents cited in the description**

- WO 2020093051 A1

**Non-patent literature cited in the description**

- **TASSEW et al.** *Cell Reports*, 01 July 2017, vol. 20 (1), 99-111
- **ARBELÁEZ-QUINTERO et al.** *Stroke Research and Treatment*, 28 May 2017, vol. 2017, 1-13
- **SHENG et al.** *Frontiers in Neurology*, 20 December 2018, vol. 9
- **CHOU et al.** *Brain and Behavior*, vol. 9 (5), 1-11
- **CHEN et al.** *Journal of Neuropathology and Experimental Neurology*, vol. 71 (12), 1123-1136
- **FEIGIN, V.L. et al.** Worldwide stroke incidence and early case fatality reported in 56 population-based studies: a systematic review. *Lancet Neurol*, 2009, vol. 8 (4), 355-69
- **BRODERICK, J.P. et al.** Guidelines for the management of spontaneous intracerebral hemorrhage: A statement for healthcare professionals from a special writing group of the Stroke Council, American Heart Association. *Stroke*, 1999, vol. 30 (4), 905-15
- **WANG, J.** ; **S.E. TSIRKA.** Tuftsin fragment 1-3 is beneficial when delivered after the induction of intracerebral hemorrhage. *Stroke*, 2005, vol. 36 (3), 613-8
- **HIJIOKA, M. et al.** Therapeutic effect of nicotine in a mouse model of intracerebral hemorrhage. *J Pharmacol Exp Ther*, 2011
- Interaction of free radicals, matrix metalloproteinases and caveolin-1 impacts blood-brain barrier permeability. **GU, Y** ; **C.M. DEE** ; **J. SHEN**. Front Biosci. 2011, vol. 3, 1216-31
- **MA, Q. et al.** Vascular adhesion protein-1 inhibition provides antiinflammatory protection after an intracerebral hemorrhagic stroke in mice. *J Cereb Blood Flow Metab*, 2011, vol. 31 (3), 881-93
- **LEKIC, T. et al.** Protective effect of melatonin upon neuropathology, striatal function, and memory ability after intracerebral hemorrhage in rats. *J Neurotrauma*, 2010, vol. 27 (3), 627-37
- **MACLELLAN, C.L. et al.** Intracerebral hemorrhage models in rat: comparing collagenase to blood infusion. *J Cereb Blood Flow Metab*, 2008, vol. 28 (3), 516-25

- **MASUDA, T. et al.** Increase in neurogenesis and neuroblast migration after a small intracerebral hemorrhage in rats. *Neurosci Lett*, 2007, vol. 425 (2), 114-9
- **HUA, Y. et al.** Thrombin and brain recovery after intracerebral hemorrhage. *Stroke*, 2009, vol. 40 (3), S88-9
- **YANG, S. et al.** Effects of thrombin on neurogenesis after intracerebral hemorrhage. *Stroke*, 2008, vol. 39 (7), 2079-84
- **BOLANDER, H.G. et al.** Treatment of spontaneous intracerebral haemorrhage. A retrospective analysis of 74 consecutive cases with special reference to computertomographic data. *Acta Neurochir (Wien)*, 1983, vol. 67 (1-2), 19-28
- **DIRINGER, M.N**. Intracerebral hemorrhage: pathophysiology and management. *Crit Care Med*, 1993, vol. 21 (10), 1591-603
- **LIAO, W. et al.** Therapeutic benefit of human umbilical cord derived mesenchymal stromal cells in intracerebral hemorrhage rat: implications of anti-inflammation and angiogenesis.. *Cell Physiol Biochem*, 2009, vol. 24 (3-4), 307-16
- **FATAR, M. et al.** Lipoaspirate-derived adult mesenchymal stem cells improve functional outcome during intracerebral hemorrhage by proliferation of endogenous progenitor cells stem cells in intracerebral hemorrhages.. *Neurosci Lett*, 2008, vol. 443 (3), 174-8
- **KRENZLIN, H. et al.** The cerebral thrombin system is activated after intracerebral hemorrhage and contributes to secondary lesion growth and poor neurological outcome in C57Bl/6 mice. *J Neurotrauma*, 2019
- **WANG, Y. et al.** Simvastatin accelerates hematoma resolution after intracerebral hemorrhage in a PPAR-gamma-dependent manner. *Neuropharmacology*, 2018, vol. 128, 244-254
- **JUNG, K.H. et al.** HMG-CoA reductase inhibitor, atorvastatin, promotes sensorimotor recovery, suppressing acute inflammatory reaction after experimental intracerebral hemorrhage. *Stroke*, 2004, vol. 35 (7), 1744-9

- **LU, D. et al.** Atorvastatin reduction of intracranial hematoma volume in rats subjected to controlled cortical impact. *J Neurosurg*, 2004, vol. 101 (5), 822-5
- **KARKI, K. et al.** Simvastatin and atorvastatin improve neurological outcome after experimental intracerebral hemorrhage. *Stroke*, 2009, vol. 40 (10), 3384-9
- **TODO, T** ; **M. USUI** ; **K. TAKAKURA**. Treatment of severe intraventricular hemorrhage by intraventricular infusion of urokinase. *J Neurosurg*, 1991, vol. 74 (1), 81-6
- **AYDIN, I.H. et al.** The effect of urokinase on experimental intracerebral haematomas. *Zentralbl Neurochir*, 1994, vol. 55 (1), 29-34
- **NAFF, N.J. et al.** Treatment of intraventricular hemorrhage with urokinase : effects on 30-Day survival. *Stroke*, 2000, vol. 31 (4), 841-7
- **FINDLAY, J.M. et al.** Intracisternal recombinant tissue plasminogen activator after aneurysmal subarachnoid hemorrhage. *J Neurosurg*, 1991, vol. 75 (2), 181-8
- **VON KUMMER, R.** ; **W. HACKE**. Safety and efficacy of intravenous tissue plasminogen activator and heparin in acute middle cerebral artery stroke. *Stroke*, 1992, vol. 23 (5), 646-52
- **MAYFRANK, L. et al.** Effect of recombinant tissue plasminogen activator on clot lysis and ventricular dilatation in the treatment of severe intraventricular haemorrhage. *Acta Neurochir (Wien)*, 1993, vol. 122 (1-2), 32-8
- **NASSER, J.A. et al.** Stereotactic fibrinolysis of spontaneous intracerebral hematoma using infusion of recombinant tissue plasminogen activator. *Arq Neuropsiquiatr*, 2002, vol. 60 (2-B), 362-6
- **ROHDE, V. et al.** Fibrinolysis therapy achieved with tissue plasminogen activator and aspiration of the liquefied clot after experimental intracerebral hemorrhage: rapid reduction in hematoma volume but intensification of delayed edema formation. *J Neurosurg*, 2002, vol. 97 (4), 954-62
- **SEIFERT, V. et al.** Endothelin concentrations in patients with aneurysmal subarachnoid hemorrhage. Correlation with cerebral vasospasm, delayed ischemic neurological deficits, and volume of hematoma. *J Neurosurg*, 1995, vol. 82 (1), 55-62
- **ZIMMERMANN, M**. Endothelin in cerebral vasospasm. Clinical and experimental results. *J Neurosurg Sci*, 1997, vol. 41 (2), 139-51
- **ALIOGLU, Z. et al.** Increased plasma endothelin-1 levels in patients with intracerebral hemorrhage. *J Stroke Cerebrovasc Dis*, 2000, vol. 9 (4), 176-80
- **LYDEN, P.D** ; **C. JACKSON-FRIEDMAN** ; **L. LONZO-DOKTOR**. Medical therapy for intracerebral hematoma with the gamma-aminobutyric acid-A agonist muscimol. *Stroke*, 1997, vol. 28 (2), 387-91
- **XI, G. et al.** Brain edema after intracerebral hemorrhage: the effects of systemic complement depletion.. *Acta Neurochir Suppl*, 2002, vol. 81, 253-6
- **RYNKOWSKI, M.A. et al.** C3a receptor antagonist attenuates brain injury after intracerebral hemorrhage. *J Cereb Blood Flow Metab*, 2009, vol. 29 (1), 98-107
- **KITAOKA, T. et al.** Delayed argatroban treatment reduces edema in a rat model of intracerebral hemorrhage. *Stroke*, 2002, vol. 33 (12), 3012-8
- **KITAOKA, T. et al.** Effect of delayed argatroban treatment on intracerebral hemorrhage-induced edema in the rat. *Acta Neurochir Suppl*, 2003, vol. 86, 457-61
- **TERAI, K. et al.** Effect of AMPA receptor antagonist YM872 on cerebral hematoma size and neurological recovery in the intracerebral hemorrhage rat model.. *Eur J Pharmacol*, 2003, vol. 467 (1-3), 95-101
- **LEMA, P.P** ; **C. GIRARD** ; **P. VACHON**. High doses of methylprednisolone are required for the treatment of collagenase-induced intracerebral hemorrhage in rats. *Can J Vet Res*, 2005, vol. 69 (4), 253-9
- **STRBIAN, D. et al.** Mast cell blocking reduces brain edema and hematoma volume and improves outcome after experimental intracerebral hemorrhage. *J Cereb Blood Flow Metab*, 2007, vol. 27 (4), 795-802
- **SINN, D.I. et al.** Combined neuroprotective effects of celecoxib and memantine in experimental intracerebral hemorrhage. *Neurosci Lett*, 2007, vol. 411 (3), 238-42
- **SINN, D.I. et al.** Proteasomal inhibition in intracerebral hemorrhage: neuroprotective and anti-inflammatory effects of bortezomib.. *Neurosci Res*, 2007, vol. 58 (1), 12-8
- **AL-SENANI, F.M. et al.** Proteasome Inhibitor Reduces Astrocytic iNOS Expression and Functional Deficit after Experimental Intracerebral Hemorrhage in Rats. *Transl Stroke Res*, 2012, vol. 3 (1), 146-53
- **THIEX, R. et al.** Addition of intravenous N-methyl-D-aspartate receptor antagonists to local fibrinolytic therapy for the optimal treatment of experimental intracerebral hemorrhages. *J Neurosurg*, 2007, vol. 106 (2), 314-20
- **GABEREL, T. et al.** Immunotherapy blocking the tissue plasminogen activator-dependent activation of N-methyl-D-aspartate glutamate receptors improves hemorrhagic stroke outcome. *Neuropharmacology*, 2013, vol. 67, 267-71
- **MEHDIRATTA, M. et al.** Association between serum ferritin level and perihematoma edema volume in patients with spontaneous intracerebral hemorrhage. *Stroke*, 2008, vol. 39 (4), 1165-70

- **ZHOU, F** ; **G. CHEN** ; **J. ZHANG**. Edaravone reduces brain oedema and attenuates cell death after intracerebral haemorrhage in mice. *Brain Inj*, 2009, vol. 23 (4), 353-7
- **NAKAMURA, T. et al.** Serine protease inhibitor attenuates intracerebral hemorrhage-induced brain injury and edema formation in rat.. *Acta Neurochir Suppl*, 2010, vol. 106, 307-10
- **CHUN, H.J. et al.** Effects of statin and deferoxamine administration on neurological outcomes in a rat model of intracerebral hemorrhage. *Neurol Sci*, 2012, vol. 33 (2), 289-96
- **AURIAT, A.M. et al.** Ferric iron chelation lowers brain iron levels after intracerebral hemorrhage in rats but does not improve outcome. *Exp Neurol*, 2012, vol. 234 (1), 136-43
- **HATAKEYAMA, T. et al.** Deferoxamine reduces neuronal death and hematoma lysis after intracerebral hemorrhage in aged rats. *Transl Stroke Res*, 2013, vol. 4 (5), 546-53
- **LASKOWITZ, D.T. et al.** The apoE-mimetic peptide, COG1410, improves functional recovery in a murine model of intracerebral hemorrhage. *Neurocrit Care*, 2012, vol. 16 (2), 316-26
- **MATSUSHITA, H. et al.** Natural and synthetic retinoids afford therapeutic effects on intracerebral hemorrhage in mice.. *Eur J Pharmacol*, 2012, vol. 683 (1-3), 125-31
- **JAMES, M.L. et al.** TT-301 inhibits microglial activation and improves outcome after central nervous system injury in adult mice. *Anesthesiology*, 2012, vol. 116 (6), 1299-311
- **SHENG, S.P. et al.** Xenon neuroprotection in experimental stroke: interactions with hypothermia and intracerebral hemorrhage. *Anesthesiology*, 2012, vol. 117 (6), 1262-75
- **KING, M.D** ; **C.H. ALLEYNE, JR.** ; **K.M. DHANDA-PANI**. TNF-alpha receptor antagonist, R-7050, improves neurological outcomes following intracerebral hemorrhage in mice. *Neurosci Lett*, 2013, vol. 542, 92-6
- **LEI, B et al.** Tumor necrosis factor alpha antagonism improves neurological recovery in murine intracerebral hemorrhage. *J Neuroinflammation*, 2013, vol. 10, 103
- **LEE, S.H. et al.** Effects of celecoxib on hematoma and edema volumes in primary intracerebral hemorrhage: a multicenter randomized controlled trial. *Eur J Neurol*, 2013, vol. 20 (8), 1161-9
- **SABATINI, F. et al.** Human bronchial fibroblasts exhibit a mesenchymal stem cell phenotype and multilineage differentiating potentialities. *Lab Invest*, 2005, vol. 85 (8), 962-71
- **LORENZ, K. et al.** Multilineage differentiation potential of human dermal skin-derived fibroblasts. *Exp Dermatol*, 2008, vol. 17 (11), 925-32
- **HUANG, H.I. et al.** Multilineage differentiation potential of fibroblast-like stromal cells derived from human skin. *Tissue Eng Part A*, 2010, vol. 16 (5), 1491-501
- **GIBBS, D.A. et al.** A clinical trial of fibroblast transplantation for the treatment of mucopolysaccharidoses. *J Inherit Metab Dis*, 1983, vol. 6 (2), 62-81
- **AKLE, C. et al.** Transplantation of amniotic epithelial membranes in patients with mucopolysaccharidoses. *Exp Clin Immunogenet*, 1985, vol. 2 (1), 43-8
- **WETZELS, A.M. et al.** The effects of human skin fibroblast monolayers on human sperm motility and mouse zygote development. *Hum Reprod*, 1992, vol. 7 (6), 852-6
- **HANSBROUGH, J.F** ; **C. DORE** ; **W.B. HANSBROUGH**. Clinical trials of a living dermal tissue replacement placed beneath meshed, split-thickness skin grafts on excised burn wounds.. *J Burn Care Rehabil*, 1992, vol. 13 (5), 519-29
- **SABOLINSKI, M.L. et al.** Cultured skin as a 'smart material'for healing wounds: experience in venous ulcers. *Biomaterials*, 1996, vol. 17 (3), 311-20
- **EAGLSTEIN, W.H. et al.** Acute excisional wounds treated with a tissue-engineered skin (Apligraf). *Dermatol Surg*, 1999, vol. 25 (3), 195-201
- **NOORDENBOS, J.** ; **C. DORE** ; **J.F. HANSBROUGH**. Safety and efficacy of TransCyte for the treatment of partial-thickness burns.. *J Burn Care Rehabil*, 1999, vol. 20 (4), 275-81
- **CHANG, D.W. et al.** Can a tissue-engineered skin graft improve healing of lower extremity foot wounds after revascularization?. *Ann Vasc Surg*, 2000, vol. 14 (1), 44-9
- **MCGUIRE, M.K. et al.** A pilot study to evaluate a tissue-engineered bilayered cell therapy as an alternative to tissue from the palate. *J Periodontol*, 2008, vol. 79 (10), 1847-56
- **KIRSNER, R.S. et al.** Spray-applied cell therapy with human allogeneic fibroblasts and keratinocytes for the treatment of chronic venous leg ulcers: a phase 2, multicentre, double-blind, randomised, placebo-controlled trial. *Lancet*, 2012, vol. 380 (9846), 977-85
- **LANTIS, J.C. et al.** The influence of patient and wound variables on healing of venous leg ulcers in a randomized controlled trial of growth-arrested allogeneic keratinocytes and fibroblasts. *J Vasc Surg*, 2013, vol. 58 (2), 433-9
- **ZHAO, L.R. et al.** Human bone marrow stem cells exhibit neural phenotypes and ameliorate neurological deficits after grafting into the ischemic brain of rats. *Exp Neurol*, 2002, vol. 174 (1), 11-20
- **ASHJIAN, P.H et al.** In vitro differentiation of human processed lipoaspirate cells into early neural progenitors. *Plast Reconstr Surg*, 2003, vol. 111 (6), 1922-31

- **WISLET-GENDEBIEN, S. et al.** Regulation of neural markers nestin and GFAP expression by cultivated bone marrow stromal cells. *J Cell Sci*, 2003, vol. 116, 3295-302
- **KANG, S.K. et al.** Improvement of neurological deficits by intracerebral transplantation of human adipose tissue-derived stromal cells after cerebral ischemia in rats. *Exp Neurol*, 2003, vol. 183 (2), 355-66
- **JEONG, J.A. et al.** Rapid neural differentiation of human cord blood-derived mesenchymal stem cells. *Neuroreport*, 2004, vol. 15 (11), 1731-4
- **WISLET-GENDEBIEN, S. et al.** Nestin-positive mesenchymal stem cells favour the astroglial lineage in neural progenitors and stem cells by releasing active BMP4.. *BMC Neurosci*, 2004, vol. 5, 33
- **LONG, X. et al.** Neural cell differentiation in vitro from adult human bone marrow mesenchymal stem cells. *Stem Cells Dev*, 2005, vol. 14 (1), 65-9
- **ALEXANIAN, A.R.** Neural stem cells induce bone-marrow-derived mesenchymal stem cells to generate neural stem-like cells via juxtacrine and paracrine interactions. *Exp Cell Res*, 2005, vol. 310 (2), 383-91
- **ARNHOLD, S. et al.** Human bone marrow, stroma cells display certain neural characteristics and integrate in the subventricular compartment after injection into the liquor system. *Eur J Cell Biol*, 2006, vol. 85 (6), 551-65
- **MOVIGLIA, G.A. et al.** Autoreactive T cells induce in vitro BM mesenchymal stem cell transdifferentiation to neural stem cells. *Cytotherapy*, 2006, vol. 8 (3), 196-201
- **RIVERA, F.J. et al.** Adult hippocampus derived soluble factors induce a neuronal-like phenotype in mesenchymal stem cells. *Neurosci Lett*, 2006, vol. 406 (1-2), 49-54
- **EL-BADRI, N.S. et al.** Cord blood mesenchymal stem cells: Potential use in neurological disorders. *Stem Cells Dev*, 2006, vol. 15 (4), 497-506
- **ZHANG, W. et al.** Combination of adenoviral vector-mediated neurotrophin-3 gene transfer and retinoic acid promotes adult bone marrow cells to differentiate into neuronal phenotypes. *Neurosci Lett*, 2006, vol. 408 (2), 98-103
- **MARESCHI, K. et al.** Neural differentiation of human mesenchymal stem cells: Evidence for expression of neural markers and eag K+ channel types. *Exp Hematol*, 2006, vol. 34 (11), 1563-72
- **KIM, S. et al.** Neural differentiation potential of peripheral blood- and bone-marrow-derived precursor cells. *Brain Res*, 2006, vol. 1123 (1), 27-33
- **KINGHAM, P.J. et al.** Adipose-derived stem cells differentiate into a Schwann cell phenotype and promote neurite outgrowth in vitro. *Exp Neurol*, 2007, vol. 207 (2), 267-74
- **GRECO, S.J. et al.** An interdisciplinary approach and characterization of neuronal cells transdifferentiated from human mesenchymal stem cells. *Stem Cells Dev*, 2007, vol. 16 (5), 811-26
- **CHU, Q. et al.** Astrocytes facilitate the growth and differentiation of co-cultured mesenchymal stem cells. *J Huazhong Univ Sci Technolog Med Sci*, 2008, vol. 28 (3), 333-6
- **YANG, Y. et al.** NRSF silencing induces neuronal differentiation of human mesenchymal stem cells. *Exp Cell Res*, 2008, vol. 314 (11-12), 2257-65
- **YANG, J. et al.** Dorsal root ganglion neurons induce transdifferentiation of mesenchymal stem cells along a Schwann cell lineage. *Neurosci Lett*, 2008, vol. 445 (3), 246-51
- **CHENG, Z. et al.** Targeted induction of differentiation of human bone mesenchymal stem cells into neuron-like cells. *J Huazhong Univ Sci Technolog Med Sci*, 2009, vol. 29 (3), 296-9
- **ZHANG, S. et al.** Stem cells modified by brain-derived neurotrophic factor to promote stem cells differentiation into neurons and enhance neuromotorfunction after brain injury. *Chin J Traumatol*, 2009, vol. 12 (4), 195-9
- **JURGA, M. et al.** Generation of functional neural artificial tissue from human umbilical cord blood stem cells. *Tissue Eng Part C Methods*, 2009, vol. 15 (3), 365-72
- **WANG, L. et al.** Differentiation of human bone marrow mesenchymal stem cells grown in terpolyesters of 3-hydroxyalkanoates scaffolds into nerve cells. *Biomaterials*, 2010, vol. 31 (7), 1691-8
- **YANG, L.L. et al.** Differentiation of human bone marrow-derived mesenchymal stem cells into neural-like cells by co-culture with retinal pigmented epithelial cells. *Int J Ophthalmol*, 2010, vol. 3 (1), 23-7
- **DING, Y. et al.** Bone marrow mesenchymal stem cells and electroacupuncture downregulate the inhibitor molecules and promote the axonal regeneration in the transected spinal cord of rats. *Cell Transplant*, 2011, vol. 20 (4), 475-91
- **EGEA, V. et al.** TNF-alpha respecifies human mesenchymal stem cells to a neural fate and promotes migration toward experimental glioma. *Cell Death Differ*, 2011, vol. 18 (5), 853-63
- **MANOCHANTR, S. et al.** Isolation, characterization and neural differentiation potential of amnion derived mesenchymal stem cells. *J Med Assoc Thai*, 2010, vol. 93 (7), S183-91
- **KHOO, M.L. et al.** Transplantation of neuronal-primed human bone marrow mesenchymal stem cells in hemiparkinsonian rodents. *PLoS One*, 2011, vol. 6 (5), e19025
- **LIU, Z. et al.** Cocaine- and amphetamine-regulated transcript promotes the differentiation of mouse bone marrow-derived mesenchymal stem cells into neural cells. *BMC Neurosci*, 2011, vol. 12, 67

- **CHO, H. et al.** Neural differentiation of umbilical cord mesenchymal stem cells by subsonic vibration. *Life Sci*, 2012, vol. 90 (15-16), 591-9
- **DING, Y. et al.** Electroacupuncture promotes the differentiation of transplanted bone marrow mesenchymal stem cells overexpressing TrkC into neuron-like cells in transected spinal cord of rats. *Cell Transplant*, 2013, vol. 22 (1), 65-86
- **QIN, X.** ; **W. HAN** ; **Z. YU**. Neuronal-like differentiation of bone marrow-derived mesenchymal stem cells induced by striatal extracts from a rat model of Parkinson's disease. *Neural Regen Res*, 2012, vol. 7 (34), 2673-80
- **MARTINI, M.M. et al.** Human placenta-derived mesenchymal stem cells acquire neural phenotype under the appropriate niche conditions. *DNA Cell Biol*, 2013, vol. 32 (2), 58-65
- **LO FURNO, D. et al.** Differentiation of human adipose stem cells into neural phenotype by neuroblastoma- or olfactory ensheathing cells-conditioned medium.. *J Cell Physiol*, 2013, vol. 228 (11), 2109-18
- **SINGH, S.P.** ; **N.K. TRIPATHY** ; **S. NITYANAND**. Comparison of phenotypic markers and neural differentiation potential of multipotent adult progenitor cells and mesenchymal stem cells. *World J Stem Cells*, 2013, vol. 5 (2), 53-60
- **ZHAO, T. et al.** Combined treatment with platelet-rich plasma and brain-derived neurotrophic factor-overexpressing bone marrow stromal cells supports axonal remyelination in a rat spinal cord hemi-section model. *Cytotherapy*, 2013, vol. 15 (7), 792-804
- **XIONG, N. et al.** bFGFpromotes the differentiation and effectiveness of human bone marrow mesenchymal stem cells in a rotenone model for Parkinson's disease. *Environ Toxicol Pharmacol*, 2013, vol. 36 (2), 411-422
- **HU, W. et al.** New methods for inducing the differentiation of amniotic-derived mesenchymal stem cells into motor neuron precursor cells. *Tissue Cell*, 2013, vol. 45 (5), 295-305
- **ZHAO, Y. et al.** Exogenous and endogenous therapeutic effects of combination Sodium Ferulate and bone marrow stromal cells (BMSCs) treatment enhance neurogenesis after rat focal cerebral ischemia. *Metab Brain Dis*, 2013, vol. 28 (4), 655-66
- **JEONG, S.G. et al.** Valproic acid promotes neuronal differentiation by induction of neuroprogenitors in human bone-marrow mesenchymal stromal cells. *Neurosci Lett*, 2013, vol. 554, 22-7
- **OH, S.H. et al.** Mesenchymal Stem Cells Increase Hippocampal Neurogenesis and Neuronal Differentiation by Enhancing the Wnt Signaling Pathway in an Alzheimer's Disease Model. *Cell Transplant*, 2015, vol. 24 (6), 1097-109
- **BERG, J. et al.** Human adipose-derived mesenchymal stem cells improve motor functions and are neuroprotective in the 6-hydroxydopamine-rat model for Parkinson's disease when cultured in monolayer cultures but suppress hippocampal neurogenesis and hippocampal memory function when cultured in spheroids. *Stem Cell Rev Rep*, 2015, vol. 11 (1), 133-49
- **ZHU, T. et al.** GDNF and NT-3 induce progenitor bone mesenchymal stem cell differentiation into neurons in fetal gut culture medium. *Cell Mol Neurobiol*, 2015, vol. 35 (2), 255-64
- **RAZAVI, S. et al.** Effect of T3 hormone on neural differentiation of human adipose derived stem cells. *Cell Biochem Funct*, 2014, vol. 32 (8), 702-10
- **JOE, I.S** ; **S.G. JEONG** ; **G.W. CHO**. Resveratrol-induced SIRT1 activation promotes neuronal differentiation of human bone marrow mesenchymal stem cells. *Neurosci Lett*, 2015, vol. 584, 97-102
- **MANOCHANTR, S. et al.** The expression of neurogenic markers after neuronal induction of chorion-derived mesenchymal stromal cells.. *Neurol Res*, 2015, vol. 37 (6), 545-52
- **MU, M.W** ; **Z.Y. ZHAO** ; **C.G. LI**. Comparative study of neural differentiation of bone marrow mesenchymal stem cells by different induction methods. *Genet Mol Res*, 2015, vol. 14 (4), 14169-76
- **RAFIEEMEHR, H.** ; **M. KHEIRANDISH** ; **M. SOLEIMANI**. Improving the neuronal differentiation efficiency of umbilical cord blood-derived mesenchymal stem cells cultivated under appropriate conditions.. *Iran J Basic Med Sci*, 2015, vol. 18 (11), 1100-6
- **NAN, C. et al.** Tetramethylpyrazine induces differentiation of human umbilical cord-derived mesenchymal stem cells into neuron-like cells in vitro. *Int J Oncol*, 2016, vol. 48 (6), 2287-94
- **JAVANMARD, F.** ; **M. AZADBAKHT** ; **M. POURMORADI**. The effect of hydrostatic pressure on staurosporine-induced neural differentiation in mouse bone marrowderived mesenchymal stem cells. *Bratisl Lek Listy*, 2016, vol. 117 (5), 283-9
- **JOE, I.S.** ; **G.W. CHO**. PDE4 Inhibition by Rolipram Promotes Neuronal Differentiation in Human Bone Marrow Mesenchymal Stem Cells. *Cell Reprogram*, 2016, vol. 18 (4), 224-9
- **SHAHBAZI, A. et al.** Rapid Induction of Neural Differentiation in Human Umbilical Cord Matrix Mesenchymal Stem Cells by cAMP-elevating Agents. *Int J Mol Cell Med*, 2016, vol. 5 (3), 167-177
- **BONILLA-PORRAS, A.R** ; **C. VELEZ-PARDO** ; **M. JIMENEZ-DEL-RIO**. Fast transdifferentiation of human Wharton'sjelly mesenchymal stem cells into neurospheres and nerve-like cells.. *J Neurosci Methods*, 2017, vol. 282, 52-60

- **ZARRINPOUR, V.** ; **Z. HAJEBRAHIMI** ; **M. JAFAR-INIA**. Expression pattern of neurotrophins and their receptors during neuronal differentiation of adipose-derived stem cells in simulated microgravity condition. *Iran J Basic Med Sci*, 2017, vol. 20 (2), 178-186
- **GUO, L. et al.** Resveratrol Induces Differentiation of Human Umbilical Cord Mesenchymal Stem Cells into Neuron-Like Cells. *Stem Cells Int*, 2017, vol. 2017, 1651325
- **HUANG, Y. et al.** Histone deacetylase inhibitor significantly improved the cloning efficiency of porcine somatic cell nuclear transfer embryos. *Cell Reprogram*, 2011, vol. 13 (6), 513-20
- **MARQUEZ-CURTIS, L.A. et al.** Migration, proliferation, and differentiation of cord blood mesenchymal stromal cells treated with histone deacetylase inhibitor valproic Acid. *Stem Cells Int*, 2014, vol. 2014, 610495
- **YAMATO, K** ; **Z. EL-HAJJAOUI** ; **H.P. KOEFFLER**. Regulation of levels of IL-1 mRNA in human fibroblasts.. *J Cell Physiol*, 1989, vol. 139 (3), 610-6

- **ZUCALI, J.R** ; **C. MORSE** ; **C.A. DINARELLO**. The role of protein kinase C in interleukin 1 and tumor necrosis factor alpha induction of fibroblasts to produce and release granulocyte-macrophage colony-stimulating activity. *Exp Hematol*, 1990, vol. 18 (8), 888-92
- **HORI, T. et al.** Prostaglandins antagonize fibroblast proliferation stimulated by tumor necrosis factor.. *Biochem Biophys Res Commun*, 1991, vol. 174 (2), 758-66
- **ROSENBERG, G.A. et al.** Collagenase-induced intracerebral hemorrhage in rats. *Stroke*, 1990, vol. 21 (5), 801-7
- **MANAENKO, A. et al.** Arginine-vasopressin V1a receptor inhibition improves neurologic outcomes following an intracerebral hemorrhagic brain injury. *Neurochem Int*, 2011, vol. 58 (4), 542-8
- **MANAENKO, A. et al.** Effect of gap junction inhibition on intracerebral hemorrhage-induced brain injury in mice. *Neurol Res*, 2009, vol. 31 (2), 173-8
- **MANAENKO, A. et al.** Geldanamycin reduced brain injury in mouse model of intracerebral hemorrhage. *Acta Neurochir Suppl*, 2011, vol. 111, 161-5